# EUROPEAN PATENT SPECIFICATION

(11) **EP 3 403 655 B1**
(45) Date of publication and mention of the grant of the patent: **07.06.2023**
(21) Application number: 17738695.0
(22) Date of filing: 13.01.2017
(51) Int. Cl.: A61K 31/7028, A61K 8/60, A61K 8/14, C07H 3/04, C07H 13/02, A61K 9/127, A61K 9/51, A23L 33/10, A61Q 19/08, A61P 25/28, A61K 9/00, A61K 9/107, A61P 3/06, A61P 39/00, A61P 25/16, A61P 9/00, A61P 25/08

(54) **COMPOSITION FOR INCREASING EXPRESSION OF PGC-1 ALPHA**
ZUSAMMENSETZUNG ZUR ERHÖHUNG DER EXPRESSION VON PGC-1 ALPHA
COMPOSITION POUR AUGMENTER L'EXPRESSION DE PGC-1 ALPHA

(30) Priority: 13.01.2016 KR 20160004383
(43) Date of publication of application: 21.11.2018
(60) Divisional application: 21154787.2
(73) Proprietor: Benebiosis Co., Ltd., Seoul 03925 (KR)
(72) Inventor: KANG, Seung Woo, Seoul 03944 (KR)
(74) Representative: J A Kemp LLP
(86) International application number: PCT/KR2017/000505
(87) International publication number: WO 2017/123066

(56) References cited:
- WO-A1-2014/100126
- WO-A1-2014/100191
- WO-A1-2016/146789
- WO-A2-92/11017
- KR-A- 20100 014 098
- US-A1- 2012 122 814
- T. YONEKAWA ET AL: "Sialyllactose ameliorates myopathic phenotypes in symptomatic GNE myopathy model mice", BRAIN., vol. 137, no. 10, 24 July 2014 (2014-07-24), pages 2670-2679, XP055275736, GB ISSN: 0006-8950, DOI: 10.1093/brain/awu210
- TARR ANDREW J ET AL: "The prebiotics 3'Sialyllactose and 6'Sialyllactose diminish stressor-induced anxiety-like behavior and colonic microbiota alterations: Evidence for effects on the gut-brain axis", BRAIN, BEHAVIOR AND IMMUNITY, ACADEMIC PRESS, SAN DIEGO, CA, US, vol. 50, 2 July 2015 (2015-07-02), pages 166-177, XP029300005, ISSN: 0889-1591, DOI: 10.1016/J.BBI.2015.06.025
- SAKAI F ET AL: "Effects of feeding sialyllactose and galactosylated N-acetylneuraminic acid on swimming learning ability and brain lipid composition in adult rats", OYO TOSHITSU KAGAKU - JOURNAL OF APPLIED GLYCOSCIE, NIHON OYO TOSHITSU KAGAKUKAI, TSUKUBA, JP, vol. 53, no. 4, 1 January 2006 (2006-01-01), pages 249-254, XP009190182, ISSN: 1340-3494
- LIANG, H. et al.: "PGC-1alpha: a Key Regulator of Energy Metabolism", Advances in Physiology Education, vol. 30, 2006, pages 145-151, XP055214165,
- TARR, A.J. et al.: "The Prebiotics 3' Sialyllactose and 6' Sialyllactose Diminish Stressor-induced Anxiety-like Behavior and Colonic Microbiota Alterations: Evidence for Effects on the Gut-brain Axis", Brain, Behavior, and Immunity, vol. 50, 2015, pages 166-177, XP029300005,
- YONEKAWA, T. et al.: "Sialyllactose Ameliorates Myopathic Phenotypes in Symptomatic GNE Myopathy Model Mice", Brain, vol. 137, 2014, pages 2670-2679, XP055275736,
- ZHU, L. et al.: "PGC-1alpha is a Key Regulator of Glucose-induced Proliferation and Migration in Vascular Smooth Muscle Cells", PloS One, vol. 4, no. 1, 2009, pages 1-7, XP055535737,

## Description

### Technical Field

This application claims priority to and the benefit of Korean Patent Application No. 10-2016-0004383 filed in the Korean Intellectual Property Office on 13 January 2016.

The present invention relates to a composition for preventing or treating a disease or symptom associated with a decrease in peroxisome proliferator-activated receptor coactivator 1-alpha (PGC-1α) expression, wherein the composition is for use in reducing the volume of adipose tissue or in reducing the content of triglycerides in adipose tissue.

### Background Art

Cell fate is regulated by the balance and combination of factors to maintain cell survival and factors to induce cell death. The signaling of cell death stimulating factors begins cell death processes through various predetermined routes, and the representative process thereof is apoptosis. The cell death process of apoptosis, which is characterized by distinctive changes in forms of chromatin condensation and nuclear division, occurs through the activity of caspase enzymes due to the cell death stimulating factors in the mitochondrial inner membrane, and this process occurs with the transduction through the mitochondrial outer membrane (Galluzzi et al., 2007; Kroemer et al., 2009) [1] (Chipuk et al., 2010; Youle and Strasser, 2008) [2]. Such apoptosis through mitochondria begins by various stimulations, such as developmental programs, DNA damage, deficiency in growth factors and nutrients, viral infection, and oxidative stress (Youle and Strasser, 2008) [3]. However, abnormal induction of apoptosis is an obvious cause of diseases. The abnormal induction of apoptosis causes, for example, neurodegeneration, ischemia reperfusion injury, autoimmune diseases, and the like (Fadeel and Orrenius, 2005) [4]. Recent research results confirmed various diseases that may be caused by PGC-la functions and PGC-la dysregulation in the cell death and survival.

### 1. Neurodegenerative diseases

Neurodegenerative diseases, such as Alzheimer's disease (AD), Parkinson's disease (PD), Huntington's disease (HD), and amyotrophic lateral sclerosis (ALS), are caused by gradual dysfunctions and apoptosis of nerve cells (Jones et al., 2012) [5] . The overall signs of these diseases result from the loss of nerve cells in specific parts. It can be seen that PGC-la is directly associated with such neurodegenerative diseases, from the hyperactivity caused by neurodegeneration and the lesions apparent in the striatal region of the brain unlike lesions less abundant in the cerebral cortex, which can be observed in PGC-la knock-out mice (Lin et al., 2004) [6]. It can be seen, together with these findings, that PGC-la plays an important role in maintaining nerve cell functions by confirming vacuolar lesions shown in the central nervous system of PGC-la knock-out mice (Leone et al., 2005) [7].

The pathophysiological phenomena of AD, such as mitochondrial oxidative dysfunction, mitochondrial production degradation, and brain dysfunction in the brain of AD patients, are due to mitochondrial dysfunction (Chaturvedi & Flint, 2013) [8]. Especially, PGC-la expression decreases in the Alzheimer patient brain, which results in apoptosis of nerve cells due to reduced mitochondrial production and functions and increased oxidative stress (Katsouri et al., 2011; Qin et al., 2009) [9, 10]. The decreased PGC-la expression increases the expression of BACE1, which breaks and cleaves the amyloid precursor protein, causing AD, to produce β-amyloid, to thereby increase the amount of β- amyloid, causing mitochondrial dysfunction and apoptosis (Wang et al., 2013) [11].

Single nucleotide polymorphisms of PGC-la gene (PPARGC1A) is significantly associated with increased risks of PD and HD (Clark et al., 2011; Weydt et al., 2009) [12, 13]. The PGC-la gene expression is decreased in AD, PD, and HD patients as well as in mouse HD models (Cui et al., 2006; Qin et al., 2009; Ranganathan et al., 2009; Weydt et al., 2006; Xiang et al., 2011; Zheng et al., 2010) [14-19] . Thus, the artificial expression of PGC-la in HD model cell culture significantly reduces the apoptosis of nerve cells (Chaturvedi et al., 2009) [20]. In addition, PGC-la overexpression improves motor performance through motor neurons of ALS model mice (Zhao et al., 2011) [21]. Due to such PGC-la functions closely associated with neurodegenerative diseases, mechanisms of pharmacological activation of PGC-la are emerging as new methods for curing various neurodegenerative diseases.

### 2. Aging phenomenon by reactive oxygen species (ROS)

PGC-la plays a key role in initiating defense mechanisms in PGC1-oxidative stress situations (Chaturvedi & Flint, 2013) [22]. This interlocks with functions in neurodegenerative diseases, and the amounts of mitrochondrial respiration-related complexes and uncoupling protein (UCP) increase by PGC1-α overexpression (St-Pierre et al., 2003) [23]. These increases occur with the expression increases of protein groups that detoxify reactive oxygen species (ROS) in mitochondria and cytoplasm (Cowell et al., 2009)[24]. The first study, revealing the roles of PGC-la in ROS metabolisms, reported that the ectopic expression of PGC-la in muscular cells increases the expression of superoxide dismutase 2 (SOD2), scavenging superoxide, and glutathione peroxidase (GPX1), scavenging scavenging hydrogen peroxide (St-Pierre et al., 2003)[23]. Through advanced research since then, it was investigated that all types of ROX detoxification enzymes present in various intercellular organs, such as mitochondria, cytoplasm, and peroxisomes, are regulated by PGC-la (St-Pierre et al., 2006; Valle et al., 2005) [25, 26]. These studies also established the physiological importance in ROS metabolic programs regulated by PGC-la. It can be seen that PGC-la performs cytoprotective functions in oxidative stress situations since the suppression of PGC-la expression prevents the increase in ROS detoxification protein groups (St-Pierre et al., 2006)[25].

In addition, aging-related chronic diseases, for example, degenerative brain diseases, metabolic diseases, and cardiovascular diseases, precede cell aging or organism aging. These diseases increase stress by reactive oxygen species, sterile inflammation, mitochondrial dysfunction, DNA damage, and telomere dysfunction and shortening. The understanding of common mechanisms of these phenomena has been incomplete until now, but a paper recently has reported that the removal of PGC-la shortens telomeres and induces DNA damages, causing vascular aging and atherosclerosis (Xiong, S 2015) [27]. According to the paper, the removal of PGC-la reduced the activity and expression of telomere reverse transcriptase (TERT), which maintains the length of telomeres, and increased the activity and expression of p53. This study shows that PGC-la plays a key role in alleviating aging and aging-causing chronic diseases.

### 3. Regarding vascular diseases or myocardial diseases

Anti-oxidative functions of PGC-la are associated with the protection of vascular endothelial cells. Like in neural models, the increase of PGC- 1α expression in human vascular endothelial cells produces mitochondria and increases ROS detoxification enzyme groups (Valle et al., 2005)[26]. The application of ROS to bovine endothelial cells increases PGC-la and intercellular anti-oxidative functions (Borniquel et al., 2006)[28]. The application of artificial oxidative stress after the overexpression of PGC-la in human vascular endothelial cells reduces the intercellular ROS increase and obstructs the caspase 3 activity (Valle et al., 2005) [26]. The major cause of death is heart failure in mouse PGC-la and PGC-1β double knock-out models (Lai et al., 2008) [29], and the PGC-la decrease is associated with stress heart failure and myocardial cell death in congestive heart failure mouse models (Garnier et al., 2003) [30]. As such, PGC-la plays an important role in the metabolism and growth of myocardial cells.

### 4. Muscle loss and related diseases

Anti-oxidative functions of PGC-la is associated with muscle maintaining and strengthening functions. The reduced muscle mass and degraded muscular functions (senile muscle loss, sarcopenia, or muscular dysfunction) have a wide range of adverse effects on from hormone-related diseases to intracellular homeostasis maintenance. Several studies have revealed that increased PGC-la expression (motor or gene expression) in muscular cells can resolve mitochondrial dysfunction, which causes muscle loss, to maintain muscle [31-38].

### 5. Fat removal and body temperature maintenance

In PGC-la null mice, the expression of mitochondrial genes, which contain various genes acting as components of the electronic transfer system (ETC), was decreased to lower respiration (Lin et al., 2004; Leone et al., 2005) [6, 7]. These reduced mitrochondrial functions damaged physiological processes processes dependent on mitochondrial metabolic processes. Actually, the PGC-la deficient mice could not increase the expression of UCP1, of which the expression is increased by the exposure to cold, showed sensitivity to cold (Lin et al., 2004; Leone et al., 2005) [6, 7]. These mice had reduced motor ability compared with normal mice (Leone et al., 2005) [7]. The mitochondrial production was increased and the mitochondrial gene expression was increased in transgenic mice overexpressing PGC-la in the heart and muscle in contrast to mice lacking PGC-la (Lehman et al., 2000; Lin et al., 2002b; Wende et al., 2007). These studies show that the *in vivo* presence or absence of PGC-1α has an important effect on mitochondrial physiology. The increased PGC-la in beige or brite preadipocytes induces the differentiation of beige or brite adipocytes into brown adipocytes to oxidize fatty acids, increasing the ability to radiate heat from the body instead of producing ATP. In addition, PGC-la also removes fat [39-42].

As is well known, the subcutaneous fat is composed of granular layers surrounded by cellulite. This subcutaneous fat is not well decomposed by exercise. The reason is that the lipolytic enzymes in the body are blocked by the cellulite. Conventionally, a procedure of physically sucking adipocytes by inserting a catheter called a acupuncture needle is prevalent, and such a procedure causes pain on a patient undergoing the procedure, and thus general anesthesia usually precedes the procedure. However, it cannot be excluded that general anesthesia may act as a risk factor to a patient undergoing the procedure, and the insertion of the catheter inevitably causes internal bleeding and increases the tissue recovery time resulting therefrom.

In lipolysis using injection (injection lipolysis), a drug capable of lipolysis is directly subcutaneously injected into a fat site to dissolve and discharge fat, and representative products therefor comprise Lipostabil^{®}, Lipodissolve, Lipo-zap, and Flab-Jab, which are known as PPC injection. These PPC injections are composed of phosphatidylcholine and deoxycholic acid for maintaining phosphatidylcholine in a liquid state, and are special medicines that were first approved as an aid for treating hepatic coma for patients failing into a coma caused by cirrhosis. However, the PPC injections are misused as unauthorized fat removers as the PPC injections are known to degrade adipocytes to lose weight when injected into the fat area. Phosphatidylcholines are main materials constituting cellular membranes, and it has been thought that the phosphatidylcholines allow fatty components to be well dissolved in the blood, and dissolve adipocytes of fat tissues to discharge the fat in the adipocytes out of the adipocytes. Theoretically, the PPC injections can be used for remove a small amount of subcutaneous fat and cellulite that are topically limited, but the PPC injection *per* se does not remove fat. The mechanisms of action of PPC injections were not revealed. Most of the studies on PPC injections are dependent on subjective assessment of patients, and there are no study results of objectively showing changes in subcutaneous fat mass. The U.S. FDA does not approve the use of phosphatidylcholines for the reduction of subcutaneous fat because of the lack of data on efficacy and safety of PPC injections, and warns against the indiscriminate use thereof. In addition, there is literature reporting adverse effects on steatohepatitis, skin, and respiratory system.

Therefore, the present inventors endeavored to develop materials capable of safely degrading body fats in order to overcome side effects of medicines and problems of chronic diseases.

### 6. Regarding aging

Aging is a complex and heterogeneous state involving several changes occurring over time. Actually, in the cases of the failure to meet energetic demands, the dysfunction in some physiological processes and the increased stress contribute to a state of aging. Mitochondria have been at the center of aging for a long period of time since mitochondrial functions are generally reduced with aging (Quinlan et al., 2011) [43]. For example, mitochondrial functions, together with PGC-la and PGC1b, are reduced during telomere dysfunction, and this situation is associated with aging (Sahin et al., 2011)[44].

In aging studies, the most prominent hypothesis is "free radical theory of ageing", in which the ROS production increased by mitochondria and the resulting oxidative damage are factors to determine aging (Quinlan et al., 2011)[43]. Specifically, mitochondrial DNA (mtDNA) mutations are thought to play a core role in mitochondrial malfunction associated with aging. Mitochondrial polymerase c (POLG) mouse models (Kujoth et al., 2005; Trifunovic et al., 2004) [45, 46] have helped to highlight the importance of mitochondria in aging. POLG is DNA polymerase located in mitochondria, and involved in mitochondrial DNA replication and DNA repair. Mice with mutations in POLG have increased mitochondrial DNA mutations, show alopecia (hair loss), and have osteoporosis and cardiomyopathy, and these symptoms are associated with aging (Kujoth et al., 2005; Trifunovic et al., 2004) [45, 46]. In order to test whether mitochondrial increase through PGC-la expression could improve external phenotypes of POLG mice, these mice were crossed with MCK-PGC-la Tg mice (Lin et al., 2002b)[47]. Mice expressing mutant POLG and PGC-la increased mitochondrial activity in the heart and skeletal muscle, which resulted in improvements in these tissue functions compared with mice expressing only mutant GOLG (Dillon et al., 2012) [48]. These data highlighted that elevated mitochondrial functions have beneficial effects regardless of mitochondrial DNA mutations. Importantly, the elevated expression of PGC-la during the lifetime delays the onset of symptoms associated with aging, such as loss of muscle mass (sarcopenia) (Wenz et al., 2009) [49]. These improved functions are attributed to the lowering of oxidative damage accumulation by age and mitochondrial malfunctions (Wenz et al., 2009)[49]. Collectively, these studies show that PGC-la delays the onset of aging-related symptoms and mitigates the effects of oxidative damage that has occurred.

WO 2014/100126 A1 relates to human milk oligosaccharides to ameliorate symptoms of stress. Yonekawa et al. (Brain 137.10 (2014): 2670-2679) relates to sialyllactose ameliorating myopathic phenotypes in symptomatic GNE myopathy model mice. Tarr et al. (Brain, behavior, and immunity 50 (2015): 166-177) relates to the prebiotics 3'Sialyllactose and 6'Sialyllactose diminishing stressor-induced anxiety-like behavior and colonic microbiota alterations and includes evidence for effects on the gut-brain axis. US 2012/122814 A1 relates to a composition for preventon or treatment of hypertrophic scars or keloids. WO 2014/100191 A1 relates to nutritional compositions comprising neuroprotective dietary oligonucleotides. Sakai et al. (Journal of Applied Glycoscience 53.4 (2006): 249-254) relates to the effects of feeding sialyllactose and galactosylated N-acetylneuraminic acid on swimming learning ability and brain lipid composition in adult rats. WO 2016/146789 A1 relates to a composition comprising siallyllactose for use in enchancing learning skills and memory function. WO 92/11017 A2 relates to the use of sialyllactose for the treatment of arthritis.

### Summary of the Invention

The invention provides a composition for preventing or treating a disease or symptom associated with a decrease in peroxisome proliferator-activated receptor coactivator 1-alpha (PGC-1α) expression, the composition comprising, as an active ingredient, a compound represented by general formula I or a salt, hydrate, or solvate thereof: General formula I: S-(MS)p-(MS)q, wherein S is sialic acid; and (MS)p and (MS)q each are independently a monosaccharide residue, wherein the composition is for use in reducing the volume of adipose tissue or in reducing the content of triglycerides in adipose tissue.

### Detailed Description of the Invention

The invention is set out in the appended set of claims.

In a composition described herein, the active ingredient is a compound of general formula I, or a salt, hydrate, or solvate thereof. In general formula I, S represents sialic acid.

Sialic acid may be linked with (MS)ρ in various manners, but is linked to a monosaccharide compound (MS)p via α2,3 linkage or α2,6 linkage. Modified sialic acid, instead of sialic acid, may be positioned at S. For example, a modification in which H of -OH group on carbon no. 4 of sialic acid is substituted with another substituent or OH thereof is substituted with another substituent may be positioned at S. The substitution may be a substitution with (e.g., H is a C1-C4 alkyl group). The C1-C4 alkyl group may be methyl, ethyl, propyl, or butyl. Most preferably, unmodified sialic acid is positioned at S.

The monosaccharide compounds corresponding to (MS)p and (MS)q may also correspond to any monosaccharide compound, and examples thereof comprise tetroses (e.g., erythrose and threose), pentoses (e.g., ribose, arabinose, xylose, and lyxose), and hexoses (allose, altrose, glucose, mannose, gulose, idose, galactose, and talose). The monosaccharide compounds positioned at (MS)p and (MS)q are preferably pentoses or hexoses, more preferably hexoses, still more preferably glucose, mannose, or galactose, and most preferably glucose or galactose. The monosaccharide compound corresponding to (MS)p and (MS)q may be D- or L-stereoisomers, and most preferably D-stereoisomers.

The same or different monosaccharide compounds, preferably different monosaccharide compounds may be positioned at (MS)p and (MS)q.

According to an aspect described herein, (MS)p is galactose or glucose and (MS)q is glucose or galactose, and most preferably, (MS)p is galactose and (MS)q is glucose. When (MS)p is galactose and (MS)q is glucose, the disaccharide compound lactose is obtained.

The monosaccharide compounds corresponding to (MS)ₚ and (MS)_{q} may be modified or unmodified. For example, a modified monosaccharide compound may be one in which H of the -OH group may be substituted with an acetyl group and OH thereof may be substituteed with an N-acetyl group. Preferably, the monosaccharide compounds corresponding to (MS)ₚ and (MS)_{q} may be unmodified monosaccharide compounds.

According to an aspect described herein, the compound of general formula I used as an active ingredient is sialyllactose. Sialyllactose, which is used as an active ingredient, is a compound formed by sequentially linking galactose and glucose to sialic acid.

Sialic acid may be linked to galactose in various manners, for example, via α-2,3 or α-2,6 linkage. Sialic acid may be modified, and for example a modification in which H of -OH group on carbon no. 4 of sialic acid is substituted with another substituent or OH thereof is substituted with another substituent may be positioned at S. The substitution may be a substitution with (e.g., H is a C1-C4 alkyl group). The C1-C4 alkyl group may be methyl, ethyl, propyl, or butyl.

The galactose and glucose of sialyllactose may have D- or L-stereoisomers, and most preferably D-stereoisomers. The galactose and glucose of sialyllactose are modified or unmodified. For example, a modified monosaccharide compound may be one in which H of the -OH group may be substituted with an acetyl group and OH thereof may be substituted with an N-acetyl group. Preferably, the galactose and glucose of sialyllactose are unmodified monosaccharide compounds.

According to an aspect described herein, the sialyllactose used as an active ingredient is α-NeuNAc-(2→3)-β-D-Gal-(1→4)-D-Glc or α-NeuNAc-(2→6)-β-D-Gal-(1→4)-D-Glc [NeuNAc: N-Acetylneuraminyl, Gal:Galactose, Glc: Glucose]. α-NeuNAc-(2→3)-β-D-Gal-(1→4)-D-Glc is a substance found in GM3 ganglioside, and α-NeuNAc-(2→6)-β-D-Gal-(1→4)-D-Glc is an isomer of the substance.

More preferably, the sialyllactose used as an active ingredient is α-NeuNAc-(2→6)-β-D-Gal-(1→4)-D-Glc. As validated in the examples below, α-NeuNAc-(2→6)-β-D-Gal-(1→4)-D-Glc has superior efficacy to α-NeuNAc-(2→3)-β-D-Gal-(1→4)-D-Glc.

One that is used as an active ingredient in the composition described herein may be the compound per se as well as a pharmaceutically acceptable salt, hydrate, or solvate thereof.

The term "pharmaceutically acceptable salt" refers to a salt of the compound that produces desired pharmacological effects, that is, increasing PGC-la expression and mitochondrial functions. Examples of this salt are formed by using inorganic acids, such as hydrochloride, hydrobromide, and hydroiodide, and organic acids, such as acetate, adipate, alginate, aspartate, benzoate, benzenesulfonate, p-toluenesulfonate, bisulfate, sulfamate, sulfate, naphthylate, butyrate, citrate, camphorate, camphorsulfonate, cyclopentane propionate, digluconate, dodecylsulfate, ethane sulfonate, fumarate, glucoheptanoate, glycerophosphate, hemisulfate, heptanoate, hexanoate, 2-hydroxyethane sulfate, lactate, maleate, methane sulfonate, 2-naphthalene sulfonate, nicotinate, oxalate, tosylate, and undecanoate.

The term "pharmaceutically acceptable hydrate" refers to a hydrate of the compound that has desired pharmacological effects. The term "pharmaceutically acceptable solvate" refers to a solvate of the compound that has desired pharmacological effects. The hydrate and solvate may be also prepared by using the acids.

The composition described herein comprises, as an active ingredient, the foregoing compound of general formula 1, or a salt (e.g. a pharmaceutically acceptable salt), hydrate, or solvate thereof.

As used herein, the term "neurodegenerative disease" refers to a generic term of diseases in which certain brain cell groups of the brain and spinal cord gradually lose functions thereof and the number of brain cells decreases. The nerve cells of the brain and spinal cord have a wide variety of functions depending on their location, and thus show a great variety of clinical aspects depending on which location the nerve cells are first damaged and lose functioning and depending on what form such dysfunction progresses.

Neurodegenerative disease includes Alzheimer's disease (AD), amyotrophic lateral sclerosis (ALS, Lou Gehrig's disease), Duchenne muscular dystrophy, Parkinson's disease (PD), Huntington's disease (HD), Pick's disease, Kuf's disease, Mohr-Tranebjerg syndrome, Wilson's disease, sporadic Alzheimer's disease, sporadic amyotrophic lateral sclerosis, sporadic Parkinson's disease, autonomic function change, sleep disorder, neuropsychiatric disorder, depression, schizophrenia, schizoaffective disorder, Korsakov's psychosis, mania, anxiety disorder, phobic disorder, learning or memory impairment, amnesia or age-related memory loss, attention deficit disorder, mood depressive disorder, major depressive disorder, anankastic personality disorder, psychoactive substance use disorder, panic disorder, bipolar affective disorder, migraine, hyperactivity disorder, and dyskinesia.

More specifically, neurodegenerative diseases may comprise acute, subacute, or chronic neurodegenerative diseases.

Acute neurodegenerative diseases may comprise stroke, cerebral infarction, cerebral hemorrhage, head injury, or spinal cord injury, and the subacute neurodegenerative diseases comprise demyelinating diseases, neurologic paraneoplastic syndrome, subacute combined degeneration, subacute necrotizing encephalitis, or subacute sclerosing encephalitis. Chronic neurodegenerative diseases may comprise memory loss (including senile dementia, vascular dementia, diffusive white matter disease (Binswanger's disease), dementia of endocrine or metabolic origin, dementia of head trauma and diffuse brain damage, dementia pugilistica, and frontal lobe dementia), Alzheimer's disease, Pick's disease, diffuse Lewy Body disease, progressive supranuclear palsy (Steel-Richardson syndrome), multiple system atrophy, chronic epileptic conditions associated with neurodegeneration, motor neuron diseases (including amyotrophic lateral sclerosis, degenerative ataxias, cortical basal degeneration, ALS-Parkinson's-Dementia complex of Guam, subacute sclerosing panencephalitis, Huntington's disease, Parkinson's disease, synucleinopathies, primary progressive aphasia, striatonigral degeneration, Machado-Joseph disease/spinocerebellar ataxia, and olivopontocerebellar degenerations), Gilles De La Tourette's disease, bulbar and pseudobulbar palsy, spinal and spinobulbar muscular atrophy (Kennedy's disease), multiple sclerosis, primary lateral sclerosis, familial spastic paraplegia, Werdnig-Hoffmann disease, Kugelberg-Welander disease, Tay-Sach's disease, Sandhoff disease, familial spastic disease, Wohlfart-Kugelberg-Welander disease, spastic paraparesis, progressive multi-focal leukoencephalopathy, familial dysautonomia (Riley-Day syndrome), and prion diseases (including Creutzfeldt-Jakob, Gerstmann-Sträussler-Scheinker disease, Kuru, and fatal familial insomnia).

Metabolic diseases, lipid metabolism-related diseases, aging and diseases caused by aging, and muscle loss (sarcopenia, cachexia) and diseases caused by muscle loss include gluconeogenesis, cellulitis, gynecomastia, pseudogynecomastia, lipodystrophy, aging, photoaging, cutaneous traumas, reepithelialization of injuries, dehydration of the skin, xerosis, keratinization disorders, calluses, hard skin, lichen planus, skin lesions associated with lupus, seborrheic dermatitis, senile dermatitis, dandruff, cradle cap, seborrhea, hyperseborrhea of acne, solar dermatitis, seborrheic keratosis, senile keratosis, actinic keratosis, photoinduced keratosis, follicular keratosis, acne, nevus, change in the function of fibroblasts, nodular fasciitis, scleroderma, Dupuytren's contracture, Sebaceous gland disorder, acne rosacea, polymorphic acne, comedones, polymorphous, rosacea, nodulocystic acne, conglobate acne, senile acne, ichthyosis, Darier's disease, keratoderma palmoplantaris, leukoplakia, mucosal lichen, cutaneous lichen, eczema, common warts, flat warts, epidermodysplasia verruciformis, oral papillomatosis, lupus erythematosus, bullous diseases, bullous pemphigoid, scleroderma, pigmentation disorders, vitiligo, alopecia areata, Lewy Body disease, neurofibrillary tangles, Rosenthal fibers, Mallory's hyaline, myasthenia gravis, Gilles de la Tourette syndrome, multiple sclerosis, amyotrophic lateral sclerosis, progressive supranuclear palsy, epilepsy, Creutzfeldt-Jakob disease, deafness-dystonia syndrome, Leigh's disease, Leber's hereditary optic neuropathy, dystonia, motor neuron disease, neuropathy syndrome, ataxia and retinitis pigmentosa, maternally inherited Leigh's disease, Friedreich's ataxia, and hereditary spastic paraplegia.

The composition described herein may be selected from the group consisting of suspensions, syrups, emulsions, liposomes, extracts, powders, granules, tablets, sustained-release preparations, and capsules.

The composition described herein may be a composition for oral administration, and is in a dosage form of a drug delivery system comprising liposomes or a sustained-release preparation.

When the composition described herein is a composition for parenteral administration, the composition may be in a dosage form of a drug delivery system comprising liposomes and an ultrasound contrast agent or a sustained-release preparation.

The composition described herein may be prepared as a pharmaceutical composition, a cosmetic composition, a nutraceutical composition, or a food composition.

The composition described herein may comprise: (a) a pharmaceutically effective amount of the foregoing compound of general formula I; and (b) a pharmaceutically acceptable carrier.

As used herein, the term "pharmaceutically effective amount" refers to an amount sufficient to attain efficacy or activity of the foregoing compound of general formula I.

The composition described herein may be prepared into a pharmaceutical composition, wherein the pharmaceutical composition described herein comprises a pharmaceutically acceptable carrier. The pharmaceutically acceptable carrier contained in the pharmaceutical composition described herein is ordinarily used at the time of formulation, and examples thereof may comprise, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. The pharmaceutical composition described herein may further comprise a lubricant, a wetting agent, a sweetening agent, a flavoring agent, an emulsifier, a suspending agent, a preservative, and the like, in addition to the above ingredients. Suitable pharmaceutically acceptable carriers and agents are described in detail in Remington's Pharmaceutical Sciences Sciences (19th ed., 1995).

The pharmaceutical composition described herein may be administered orally or parenterally. Examples of parenteral administration may comprise intravenous injection, subcutaneous injection, intramuscular injection, intraperitoneal injection, transdermal injection, mucosal administration, administration of eye drops, and the like.

A proper dose of the pharmaceutical composition described herein may vary depending on various factors, such as the method for formulation, the manner of administration, patient's age, body weight, gender, morbidity, and diet, the time of administration, the excretion rate, and the response sensitivity. Preferably, the dose of the pharmaceutical composition described herein is 0.0001-1000 mg/kg (body weight), for example, 0.001-800 mg/kg (body weight), or 0.001-600 mg/kg (body weight) per day in adults. In addition, the pharmaceutical composition may be administered once or several times a day at a predetermined time interval according to the judgment of a doctor or pharmacist.

The pharmaceutical composition described herein may be formulated into a unit dosage form or may be prepared in a multi-dose container by using a pharmaceutically acceptable carrier and/or excipient according to the method easily conducted by a person having an ordinary skill in the art to which the present invention pertains.

The dosage form of the composition described herein may be solutions, suspensions, syrups, emulsions, liposomes, extracts, powders, granules, tablets, sustained-release preparations, and capsules, and the composition described herein may further comprise a dispersant or a stabilizer.

Specifically, according to the route of administration, the solid dosage form for oral administration comprises capsules, tablets, pills, powders, and granules. In these solid dosage forms, active compounds may be mixed with at least one inert, pharmaceutically acceptable excipient or carrier (e.g., sodium citrate or dicalcium phosphate) and/or a) fillers or extenders (e.g., starches, lactose, sucrose, glucose, mannitol, and silicic acid), b) binders (e.g., carboxymethylcellulose, alginate, gelatin, polyvinylpyrrolidinone, sucrose, and Arabia gum, c) moisturizing agents (e.g., glycerol), d) disintegrating agents (e.g., agar-agar, calcium carbonate, potato or tapioca starch, alginic acid, predetermined silicate, and sodium carbonate), e) solution retarders (e.g., paraffin), f) absorption accelerators (e.g., quaternary ammonium compounds), g) wetting agents (e.g., cetyl alcohol and glycerol monostearate), h) absorbents (e.g., kaolin and bentonite clay), and i) lubricants (e.g., talc, calcium stearate, magnesium stearate, solid polyethylene glycols, sodium lauryl sulfate, and mixtures thereof). In the case of capsules, tablets and pills, the dosage form thereof may also comprise buffers.

In addition, the active compounds may be used as excipients, such as lactose or milk sugar, as well as fillers in soft and hard gelatin capsules using high-molecular weight polyethylene glycols and the like.

The solid administration forms of tablets, sugar coated tablets, capsules, pills, and granules can be prepared with coatings and shells, such as enteric coatings and other coatings well known in the pharmaceutical field. These may optionally comprise an opacifier, and they may be formulated such that only the active ingredient is released at a particular site in the gastrointestinal tract in a sustained manner or preferentially. Also, if necessary, the active compounds may be prepared into microcapsules together with at least one of the excipients.

Liquid dosage forms for oral administration comprise pharmaceutically acceptable emulsions, solvents, suspensions, syrups, and elixirs. In addition to addition to the active compounds, the liquid dosage forms forms may comprise inert diluents commonly used in the art, such as, water or other solvents, solubilizers, and emulsifiers (e.g., ethyl alcohol, isopropyl alcohol, ethyl carbonate, ethyl acetate, benzyl alcohol, benzyl benzoate, propylene glycol, 1,3-butylene glycol, dimethyl formamide), oils (in particular, cottonseed oil, groundnut, corn oil, germ oil, olive oil, castor oil, or sesame oil), glycerol, tetrahydrofuryl alcohol, polyethylene glycol, and fatty acid ester of sorbitan, and mixtures thereof. Besides the inert diluents, the oral composition may also comprise adjuvants, such as a wetting agent, an emulsifier, a suspending agent, a sweetening agent, a flavoring agent, and an aroma.

A dosage form for rectal or vaginal administration is preferably a suppository which can be prepared by mixing the compound described herein with a suitable non-irritating adjuvant or carrier (e.g., cocoa butter, polyethylene glycol, or suppository wax), which is a solid at room temperature but a liquid at the body temperature and therefore melts in the rectum or vaginal cavity to releases the active compound.

A suitable dosage form for parenteral injection may comprise a physiologically acceptable sterile aqueous or non-aqueous solution, a dispersion, a suspension, or an emulsion, and a sterile powder which can be reconfigured into a sterile injectable solution or dispersion. Suitable examples of the aqueous or non-aqueous carrier, diluent, solvent, or vehicle comprise water, ethanol, polyols(propylene glycol, polyethylene glycol, glycerol, etc.), vegetable oils (olive oil), injectable organic esters (e.g., ethyl oleate), and adequate mixtures thereof.

In addition, the composition described herein may comprise an adjuvant, such as a preservative, a wetting agent, an emulsifier, and a dispersant. The actions of microorganisms may be suppressed by various anti-bacterial and anti-fungal agents (e.g., paraben, chlorobutanol, phenol, sorbic acid, etc.). In addition, the composition described herein may preferably comprise osmoregulators, such as sugar and sodium chloride. The prolonged absorption of the injectable formulation may be attained by using absorption delaying agents (e.g., aluminum monostearate and gelatin).

A suspension, in addition to the active compounds, may comprise a suspending agent (e.g., ethoxylated isostearyl alcohol, polyoxyethylene sorbitol and sorbitan ester, microcrystalline cellulose, aluminum metahydroxide, bentonite, agar-agar, tragacanth, or mixtures thereof).

In some cases, in order to prolong drug effects, it is desirable to slow the absorption of drugs from subcutaneous or intramuscular injection. This may be accomplished by using a liquid suspension of crystalline or amorphous material having poor water solubility. Here, the rate of drug absorption depends on the rate of dissolution, which may depend on the crystal size and crystalline form. Meanwhile, the delayed absorption of a parenterally administered drug form is accomplished by dissolving or suspending a drug in an oil vehicle.

An injectable depot form is made by forming drug microencapsulated matrices into biodegradable polymers, such as polylactide-polyglycolide. The rate of drug release may be controlled depending on the ratio of drug to polymer and the properties of the particular polymer used.

Other examples of the biodegradable polymer comprise poly(orthoester) and poly(anhydride). In addition, a depot injectable dosage form is prepared by encapsulating a drug in liposomes or microemulsions that are compatible with the body tissue.

An injectable dosage form may be sterilized by, for example, filtration through a bacteria-retaining filter, or by incorporating a sterilizing agent in the form of a sterile solid composition that can be dissolved or dispersed in sterile water or some other sterile injectable medium immediately before use.

The composition described herein may be a composition for oral administration, which is liposomes or a sustained-release preparation.

The composition described herein may be a composition for parenteral administration, and is in a dosage form of liposomes or a sustained-release preparation.

When the pharmaceutical composition described herein is prepared in a dosage form for oral administration as well as a dosage form for parenteral administration (preferably, intravenous administration), the pharmaceutical composition is in a dosage form of liposomes or a sustained-release preparation.

The pharmaceutical composition described herein may be encapsulated in a liposome to provide the stability of a dosage form for drug delivery. The liposomes used may be prepared by mixtures comprising polyols, surfactants, phospholipids, fatty acids, and water (Prescott, Ed., Methods in Cell Biology, (XIV), p.33et seq.(1976)).

The polyols used in the liposomes are not particularly limited, and preferable examples thereof comprise propylene glycol, dipropylene glycol, 1,3-butylene glycol, glycerin, methylpropanediol, isoprene glycol, pentylene glycol, erythritol, xylitol, and sorbitol, most preferably propylene glycol.

The surfactant used in the preparation of liposomes may be any surfactant known in the art, and examples thereof comprise an anionic surfactant, a cationic surfactant, an amphoteric surfactant, and a non-ionic surfactant, preferably an anionic surfactant and a non-ionic surfactant. Specific examples of the anionic surfactant comprise alkylacyl glutamates, alkyl phosphates, alkyl lactates, dialkyl phosphates, and trialkyl phosphates. Specific examples of the non-ionic surfactant comprise alkoxylated alkyl ethers, alkoxylated alkyl esters, alkyl polyglycosides, polyglyceryl esters, and sugar esters. Most preferably, polysorbates belonging to the non-ionic surfactants are used.

A phospholipid, which is another component used in the preparation of liposomes, is an amphoteric lipid, and examples thereof comprise natural phospholipids (e.g., yolk lecithin, soybean lecithin, or sphingomyelin) and synthetic phospholipids (e.g., dipalmitoylphosphatidylcholine or hydrogenated lecithin), and a preferable example thereof is lecithin. More preferably, the lecithin is naturally occurring unsaturated lecithin or saturated lecithin extracted from soybeans or egg yolks. In general, naturally occurring lecithin comprises 23-95% of phosphatidylcholines and 20% or more of phosphatidyl ethanolamines.

The fatty acids used in the preparation of liposomes are higher fatty acids, and preferable examples thereof comprise saturated or unsaturated fatty acids of C12-22 alkyl chains, for example, lauric acid, myristic acid, palmitic acid, stearic acid, oleic acid, and linoleic acid. In general, the water used in the preparation of liposomes is deionized distilled water.

The liposomes may be prepared by various methods known in the art, but most preferably, may be prepared by applying a mixture comprising the ingredients to a high-pressure homogenizer.

The thus prepared liposome systems have an advantage that the systems can maximize drug delivery by dissolving various poorly soluble materials and stabilizing unstable materials.

The pharmaceutical composition described herein may be prepared into a sustained-release preparation to continuously maintain an effective blood level of the effective ingredient, thereby reducing the number of times of taking medications to improve the drug compliance.

The sustained-release preparations are formulated to comprise a sustained-release carrier and other adjuvants in addition to the active ingredient described herein. The sustained-release carrier may comprise various sustained-release carriers known in the art, and preferably polyethylene oxide.

In addition, the pharmaceutical composition described herein may comprise, as the other adjuvant, a diluent carrier that is commonly in the pharmaceutical field. Examples of the diluent carrier used for this purpose comprise lactose, dextrin, starch, microcrystalline cellulose, calcium hydrogen phosphate, calcium carbonate, sugar, and silicon dioxide. In addition, the pharmaceutical composition described herein may comprise a glidant for improving flowability, such as zinc stearate or magnesium stearate, and the other adjuvant that may be used in the pharmaceutical field.

The pharmaceutical composition described herein can be used alone, but may further comprise typical active ingredients that are used for neurological diseases, vascular disease, aging, and muscle loss, which have been mentioned in the present technique, and in these cases, the pharmaceutical composition may be used as a more effective composition by synergistic effects.

The composition described herein may be prepared in the form of a cosmetic composition. The cosmetic composition described herein may be formulated into any dosage form that is conventionally prepared, and examples thereof may comprise solutions, suspensions, emulsions, pastes, gels, creams, lotions, powders, soaps, surfactant-containing cleansers, oils, powder foundations, emulsion foundations, wax foundations, and sprays, but are not limited thereto. More specifically, the cosmetic composition may be prepared in the dosage form of emollient lotion, nourishing lotion, nourishing cream, massage cream, essence, eye cream, cleansing cream, cleansing foam, cleansing water, pack, spray, or powder.

Animal oil, plant oil, wax, paraffin, starch, tracant, a cellulose derivative, polyethylene glycol, silicone, bentonite, silica, talc, or zinc oxide may be used as a carrier ingredient in cases where the dosage form is a paste, cream, lotion, or gel.

Lactose, talc, silica, aluminum hydroxide, calcium silicate, or a polyamide powder may be used as a carrier ingredient in cases where the dosage form described herein is a powder or spray. Especially, in cases where the dosage form is a spray, the spray may further comprise a propellant, such as chlorofluorohydrocarbon, propane/butane, or dimethyl ether.

A solvent, a solubilizer, or an emulsifier may be used as a carrier component in cases where the dosage form described herein is a solution or emulsion, and examples thereof comprise water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan.

A liquid diluent (such as water, ethanol, or propylene glycol), a suspending agent (such as ethoxylated isostearyl alcohol, polyoxyethylene sorbitol ester, or polyoxyethylene sorbitan ester), microcrystalline cellulose, aluminum metahydroxide, bentonite, agar, or tragacanth may be used as a carrier ingredient in cases where the dosage form described herein is a suspension.

Aliphatic alcohol sulfate, aliphatic alcohol ether sulfate, sulfosuccinate monoester, isethionate, imidazolium derivatives, methyl taurate, sarcosinate, fatty acid amide ether sulfate, alkyl amido betaine, aliphatic alcohol, fatty acid glyceride, fatty acid diethanolamide, plant oil, lanoline derivatives, or ethoxylated glycerol fatty acid ester may be used as a carrier ingredient in cases where the dosage form described herein is a surfactant-containing cleanser.

The ingredients contained in the cosmetic composition described herein comprise ingredients that are usually used in the cosmetic composition, in addition to the active ingredient and the carrier ingredients, and for example, may comprise common adjuvants, such as anti-oxidants, stabilizers, solubilizers, vitamins, pigments, and flavoring agents.

In cases where the composition described herein is prepared into a food composition (or nutraceutical composition), the food composition comprises the compound of general formula I as an active ingredient and ingredients that are ordinarily added at the time of food manufacturing, and examples thereof may comprise proteins, carbohydrates, fats, nutrients, seasonings, and flavoring agents. Examples of the carbohydrate comprise monosaccharides, e.g., glucose, fructose, etc.; disaccharides, e.g., maltose, sucrose, oligosaccharide, etc.; and polysaccharides, such as sugars (e.g., dextrin, cyclodextrin, etc.) and sugar alcohols (e.g., xylitol, sorbitol, erythritol, etc.). Examples of the flavoring agent may comprise natural flavoring agents (thaumatin, and stevia extract (e.g., rebaudioside A, glycyrrhizin, etc.)) and synthetic flavoring agents (saccharin, aspartame, etc.).

For example, when the food composition described herein is prepared into a drink, the food composition may further comprise, in addition to the compound of general formula I, citric acid, liquefied fructose, sugar, glucose, acetic acid, malic acid, fruit juice, an *Eucommia ulmoides* extract, a jujube extract, and a licorice extract.

The composition described herein may be incorporated in a sitological, cosmetical, or pharmaceutical delivery system or sustained-release system selected from the group consisting of liposomes, mixed liposomes, oleosomes, niosomes, ethosomes, millicapsules, microcapsules, nanocapsules, nanostructured lipid media, sponges, cyclodextrins, vesicles, micelles, mixed micelles of surfactants, surfactant-phospholipid mixed micelles, millispheres, microspheres, nanospheres, lipospheres, microemulsions, nanoemulsions, miniparticles, milliparticles, microparticles, nanoparticles, and solid lipid nanoparticles.

The nanocapsules described herein may comprise microemulsions.

The composition described herein may be for use by topical, oral, or parenteral application. Specifically, for example, the topical administration comprises dermal administration.

The topical application, specifically, for example, the dermal administration may be performed by iontophoresis, ultrasonophoresis, electroporation, mechanical pressure, osmotic pressure gradient, occlusive cure, microinjection, needleless injection by pressure, use of micro-electro-patches, use of face masks, or any combination thereof, but is not limited thereto.

The composition described herein may increase the expression of PGC-la.

According to the present invention, the composition of the present invention is for use in reducing the volume of adipose tissue or in reducing the content of triglycerides in the adipose tissue.

More specifically, for example, the adipose tissue is subcutaneous adipose tissue.

In an embodiment of the present invention, the subcutaneous adipose tissue is the subcutaneous adipose tissue of the femoral region, chest, a lower part of the neck, neckline, buttocks, face, lips, cheeks, eyelids and/or hands.

Still another embodiment of the present invention, the adipose tissue is any adipose tissue that may be formed in the body, including adipose tissue formed by fat embolism.

A sitological, cosmetical, or pharmaceutical composition may comprise a sitologically, cosmetically, or pharmaceutically effective amount of at least one general formula I or acceptable salt according to other aspects described herein, and at least one sitologically, cosmetically, or pharmaceutically acceptable excipient or adjuvant.

General formula I, a mixture thereof, and/or a sitologically, cosmetically, or pharmaceutically acceptable salt thereof may be confirmed in a state of being adsorbed on a sitologically, cosmetically, or pharmaceutically acceptable solid organic polymer or solid mineral support, which is formed by talc, bentonite, silica, starch, and maltodextrin.

The composition described herein may be provided in a dosage form selected from the group consisting of creams, multiple emulsions, anhydrous compositions, aqueous dispersions, oils, milks, balsams, foams, lotions, gels, cream gels, hydroalcoholic solutions, hydroglycolic solutions, liniments, sera, soaps, shampoos, conditioners, serums, ointments, mousses, pomades, powders, bars, pencils, sprays, aerosols, capsules, gelatin capsules, soft capsules, hard capsules, tablets, sugar coated tablets, granules, chewing gum, solutions, suspensions, emulsions, syrups, elixirs, polysaccharide films, jellies, and gelatins.

The composition described herein may be confirmed in a state of being incorporated into a product selected from the group consisting of under-eye concealers, makeup foundations, make-up removal lotions, make-up removal milks, eye shadows, lipsticks, lip glosses, lip protectors, and powders.

General formula I, a mixture thereof, and/or a sitologically, cosmetically, or pharmaceutically acceptable salt thereof may be incorporated into fabrics, nonwoven fabrics, or medical apparatuses.

The fabrics, nonwoven fabrics, or medical apparatuses described herein may be selected from the group consisting of bandages, gauzes, t-shirts, tights, socks, underwear, girdles, gloves, diapers, sanitary napkins, dressings, bedspreads, wipes, adhesive patches, non-adhesive patches, occlusive patches, micro-electric patches, and face masks.

The composition described herein may further comprise a sitologically, cosmetically, or pharmaceutically effective amount of at least one adjuvant selected from the group consisting of other PGC-la regulators, other PPARγ regulators, preparations for reducing adipocyte triglycerides, preparations for delaying adipocyte differentiation, lipolytic agents or lipolysis stimulators, anti-cellulite agents, adipogenetic agents, acetylcholine-receptor clustering inhibitors, muscle contraction inhibitors, anticholinergic agents, elastase inhibitors, matrix metalloproteinase inhibitors, melanin synthesis stimulators or inhibitors or depigmenting agents, propigmenting agents, self- tanning agents, anti-aging agents, NO-synthase inhibitors, 5α-reductase-inhibitors, inhibitors, lysyl-hydroxylase and/or prolyl-hydroxylase inhibitors, anti-oxidant agents, free radical scavengers and/or anti-atmospheric pollution agents, reactive carbonyl species scavengers, anti-glycation agents, anti-histaminic agents, anti-viral agents, anti-parasitic agents, emulsifiers, emollients, organic solvents, liquid propellants, skin conditioners, wetting agents, moisture retaining substances, α- and β-hydroxy acids, moisturizing agents, dermal hydrolases, vitamins, amino acids, proteins, pigments or colorants, dyes, biopolymers, gelling polymers, viscosity increasing agents, surfactants, softening agents, binders, preservatives, anti-wrinkling agents, agents capable of reducing or treating bags under eyes, exfoliating agents, desquamating agents, keratolytic agents, anti-bacterial agents, anti-fungal agents, fungistatic agents, bactericidal agents, bacteriostatic agents, dermal or stimulators, elastin synthesis stimulators, decorin synthesis stimulators, laminin synthesis stimulators, defensin stimulators, chaperone stimulators, cAMP synthesis stimulators, thermal-shock proteins, HSP70 synthesis stimulators, thermal-shock protein synthesis stimulators, aquaporin synthesis stimulators, hyaluronic acid synthesis stimulators, fibronectin synthesis stimulators, sirtuin synthesis stimulators, agents stimulating the synthesis of stratum corneum components and lipids, ceramides, fatty acids, collagen degradation inhibitors, elastin degradation inhibitors, serine protease inhibitors, fibroblast proliferation stimulators, keratinocyte proliferation stimulators, melanocyte proliferation stimulators, keratinocyte differentiation stimulators, acetylcholinesterase inhibitors, skin relaxants, glycosaminoglycan synthesis stimulators, hyperkeratosis inhibitors, comedolytic agents, DNA repairing agents, DNA protecting agents, stabilizers, anti-pruritic agents, agents for the treatment and/or care of sensitive skin, firming agents, redensifying agents, restructuring agents, anti-stretch mark agents, sebum production regulators, anti-sudorific agents, healing stimulators, coadjuvant healing agents, re-epithelialization stimulators, coadjuvant re-epithelialization agents, cytokine growth factors, sedative agents, antiinflammatory agents, anesthetic agents, agents acting on capillary circulation and/or microcirculation, vascular permeability inhibitors, venotonic agents, agents acting on cellular metabolisms, agents for improving dermal-epidermal junction, hair growth inducers or retarders, flavoring agents, chelating agents, plant extracts, essential oils, marine extracts, agents obtained from biological fermentation processes, mineral salts, cell extracts, sunscreens, and organic or mineral photoprotective agents having activity against UV A and/or B, and mixtures thereof.

The adjuvant described herein may be derived from synthesis origin, plant extracts, biological fermentation processes, or a combination of synthesis or biotechnology processes.

The adjuvant described herein may be selected from the group consisting of agents for increasing or decreasing the content of triglycerides in adipose tissue, agents for for increasing or delaying adipocyte differentiation, lipolytic agents and/or venotonic agents.

The agents for increasing or decreasing the content of triglycerides in adipose tissue, agents for delaying adipocyte differentiation, anti-cellulite agents, lipolytic agents and/or venotonic agents described herein may be selected from the group consisting of forskolin, caffeine, escin, carnitine, coenzyme A, lipase, glaucine, esculin, visnadine, sarsasapogenin, extracts of *Coffea Arabica,* extracts of *Coleus forskohlii,* extracts of *Anemarrhena apshodeloides,* and a mixture of water, glycerin, lecithin, caffeine, extracts of Butcher's broom (*Ruscus Aculeatus*), maltodextrin, silica, triethanolamine hydroiodide, propylene glycol, extracts of ivy (*Hedera helix*), carnitine, escin, tripepide-1, xanthan gum, carrageenan (Chondrus crispus), and disodium EDTA.

The adjuvant described herein may be selected from the group consisting of firming agents, redensifying agents, and restructuring agents.

The firming agents, redensifying agents, and restructuring agents described herein may be selected from the group consisting of *Pseudoalteromonas* fermented extracts, tripeptide-10 citrulline, acetylarginyl-tryptophyl diphenylglicine, hexapeptide-10, and a mixture of *Pseudoalteromonas* fermentation extracts, hydrolyzed wheat proteins, hydrolyzed soy proteins, tripeptide-10 citrulline, and tripeptide-1.

The adjuvant described herein may be selected from anti-stretch mark agents. More specifically, for example, the anti-stretch mark agents described herein are selected from the group consisting of extracts of *Centella Asiatica,* extracts of *Rosa canina,* extracts of *Rosa moschata,* extracts of *Rosa rubiginosa,* and a mixture of water, caprylyl/capryl glucoside, lecithin, glycerin, *Pseudoalteromonas* ferment extract, acetyl tripeptide-30 citrulline, pentapeptide-18, xanthan gum, and caprylyl glycol.

The adjuvant described herein may be selected from anti-wrinkling agents or anti-aging agents.

The anti-wrinkling agents or anti-aging agents described herein may be selected from the group consisting of: acetyl heptapeptide-8; acetyl heptapeptide-4; acetyl octapeptide-3; pentapeptide-18; acetylhexapeptide-30; a mixture of hydrolyzed wheat proteins, hydrolyzed soy proteins, and tripeptide-1; a mixture of diaminopropionyl tripeptide-33, tripeptide-10 citrulline, Pseudoalteromonas fermentation extract, hydrolyzed wheat proteins, hydrolyzed soy proteins, and tripeptide-10 citrulline, and tripeptide-1; a mixture of acetyl tetrapeptide-5, acetyltripeptide-30 citrulline, acetylarginyltriphenyldiphenylglycine, acetyltetrappeptide-22, dimethylmethoxychromanol, dimethylmethoxychromanyl palmitate, *Pseudoalteromonas* fermentation extract, lysine HCl, lecithin, and tripeptide-9 citrulline; and a mixture of lysine HCl, lecithin and tripeptide 10 citrulline.

### Brief Description of the Drawings

FIGS. 1a, 1b, and 1c show the gene expression changes in organs (FIG. 1a), skeletal muscles (FIG. 1b), and abdominal fat (FIG. 1c) by the treatment with 6'-sialyllactoase (SL) in normal mouse models. FIGS. 1d, 1e, and 1f show the gene expression changes in organs (FIG. 1d), skeletal muscles (FIG. 1e), and abdominal fat (FIG. 1f) by the treatment with 3'-SL in normal mouse models.
FIGS. 2a and 2b show the numerical values (FIG. 2a) and western blots (FIG. 2b) of PGC-la protein expression changes in the brain and hippocampus.
FIG. 3 shows the gene expression changes by the treatment with SL (3'-SL & 6'-SL) compositions in nerve cells.
FIG. 4 shows the gene expression changes by the treatment with SL (3'-SL & 6'-SL) compositions in C2C12 immature muscle cells.
FIGS. 5a and 5b show the numerical values of (gene expression level of the (Alzheimer's disease model) group)/gene expression level of the control group), (gene expression level of the (Alzheimer's disease model + 3'-SL) group)/gene expression level of the control group), and (gene expression level of the (Alzheimer's disease model + 6'-SL) group)/gene expression level of the control group) in respective organs with respect to relative gene expression changes in the brain and hippocampus compared with the control group.
FIG. 6 shows the numerical value of escape time in the cognitive ability test when a control group, an (Alzheimer's disease model) group, an (Alzheimer's disease model + 3'-SL) group, and an (Alzheimer's disease model + 6'-SL) group were subjected to the cognitive ability test (Morris water maze) for 7 days.
FIGS. 7a and 7b show the gene expression changes by the treatment with SL (3'-SL & 6'-SL) compositions in Parkinson's disease models.
FIG. 8 shows the behavior test results in Parkinson's disease animal models.
FIGS. 9a and 9b show the gene expression changes by the treatment with SL compositions in epilepsy/convulsion brain disease models.
FIGS. 10a and 10b show the gene expression changes by the treatment with SL (3'-SL & 6'-SL) compositions in Huntington's disease models.
FIG. 11 shows the rotarod travel test results in Huntington's disease models.
FIG. 12 shows the observation results of ischemic volume in ischemic models.
FIG. 13 shows the MLPT test score changes by SL (3'-SL & 6'-SL) treatment after ICH induction
FIG. 14 shows the gene expression changes by the treatment with SL compositions in mouse models.
FIG. 15 shows subcutaneous fat reduction effects when SL (3'-SL & 6'-SL) compositions were topically administered in mouse models.
FIGS. 16a and 16b show the gene expression changes by the treatment with SL (3'-SL & 6'-SL) compositions in aging stimulating models.
FIGS. 17a and 17b show the measurement results of intercellular H₂O₂ (ROS values) (FIG. 17a) and the measurement results of mitochondrial superoxide production (FIG. 17b).
FIGS. 18a and 18b show the measurement results of telomerase activity by the administration of SL (3'-SL & 6'-SL) compositions.
FIGS. 19a and 19b show the measurement results of intercellular H₂O₂ (ROS values) (FIG. 19a) and the measurement results of mitochondrial superoxide production (FIG. 19b).
FIGS. 20a and 20b show the analysis results of telomerase activity by the treatment of arteriosclerosis models (ApoE^{-/-}; FIG. 20a) and MASMs (FIG. 20b) with SL (3'-SL & 6'-SL).
FIGS. 21a and 21b show the gene expression changes by the treatment with SL (3'-SL & 6'-SL) compositions in skin test models.
FIGS. 22a and 22b show the intercellular fat changes when 6'-sialyllactose (FIG. 22a) and 3'-sialyllactose (FIG. 22b) were administered into differentiated adipocytes.
FIGS. 23a and 23b show the optical microscopic images of cells, showing the intercellular fat changes when 6'-sialyllactose (FIG. 23a) and 3'-sialyllactose (FIG. 23b) were administered into differentiated adipocytes.
FIG. 24 shows the effect of 6'-sialyllactose on cell viability in differentiated adipocytes.
FIG. 25 shows the glycerol secretion changes by sialyllactose in differentiated adipocytes.
FIGS. 26a and 26b show the skin changes of high-fat diet mice by subcontenous injection of sialyllactose.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### Example 1: Evaluation of gene expression stimulation by treatment with SL (3'-SL & 6'-SL) compositions in normal mouse models

In order to investigate relative gene expression changes by organs compared with before SL (3'-SL & 6'-SL) aministration, 4-week-old C57BL/6 mice were purchased from Dooyeul Biotech (Korea). Water was freely accessible, and a commercially available pellet feed (Dooyeul Biotech, Korea) was given for two weeks. At 6 weeks of age, the mice (initial body weight, average 21.4±1.1 g) were randomly divided into three groups (composed of eight mice for each group) below, and the diets were maintained for 10 weeks (a total of 24 animals):
- Control group: Normal diet mouse group
- 3'-SL administration group: Separate administration with 3'-sialyllactose (3'-SL, Sigma) (oral administration of 0.1 mg per kg of mouse weight per day) in addition to the normal diet group
- 6'-SL administration group: Separate administration with 6'-sialyllactose (6'-SL, Sigma) (oral administration of 0.1 mg per kg of mouse weight per day) in addition to the normal diet group

Sialyllactose or deionized water was orally administered daily. The mice were kept in animal rooms for 10 weeks, fasted for 12 hours, and sacrificed. The dietary intake and body weight change were measured every 5 days. 3'-Sialyllactose (3'-N-acetylneuraminyl-D-lactose, 3'-sialyl-D-lactose, or α-NeuNAc-(2,3)-β-D-Gal-(1,4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

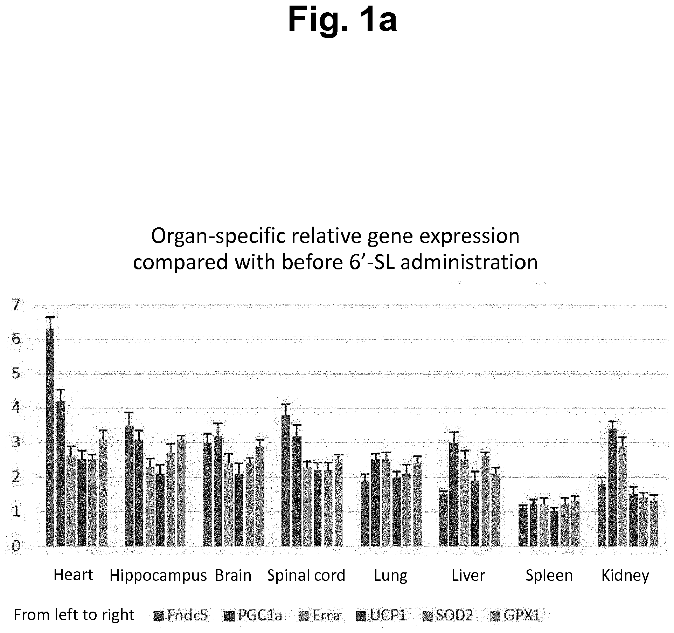

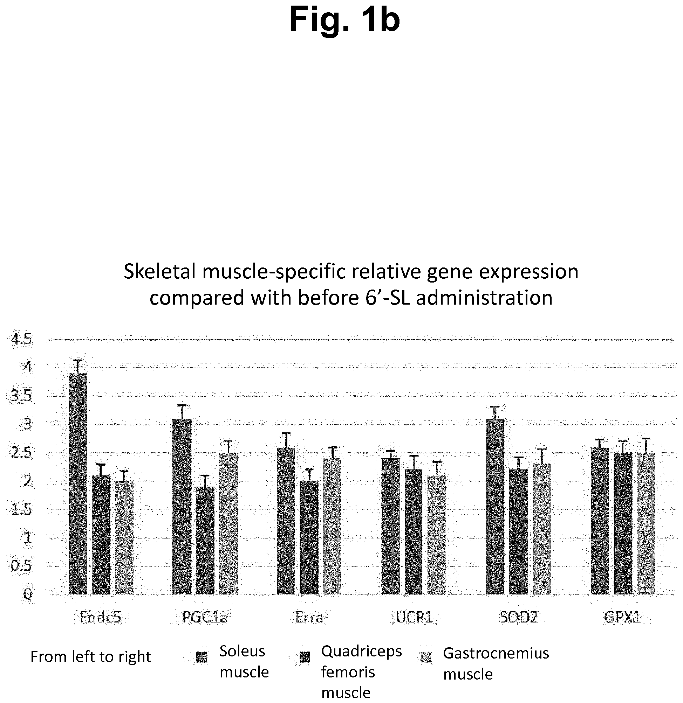

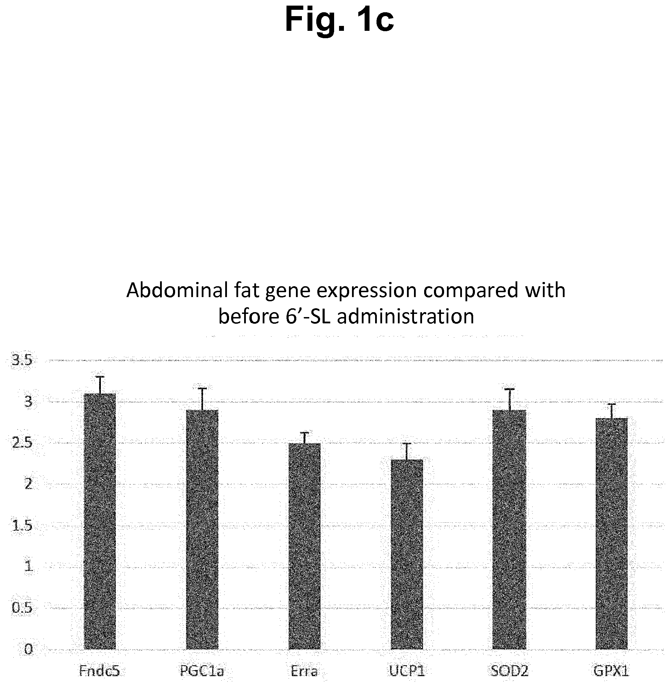

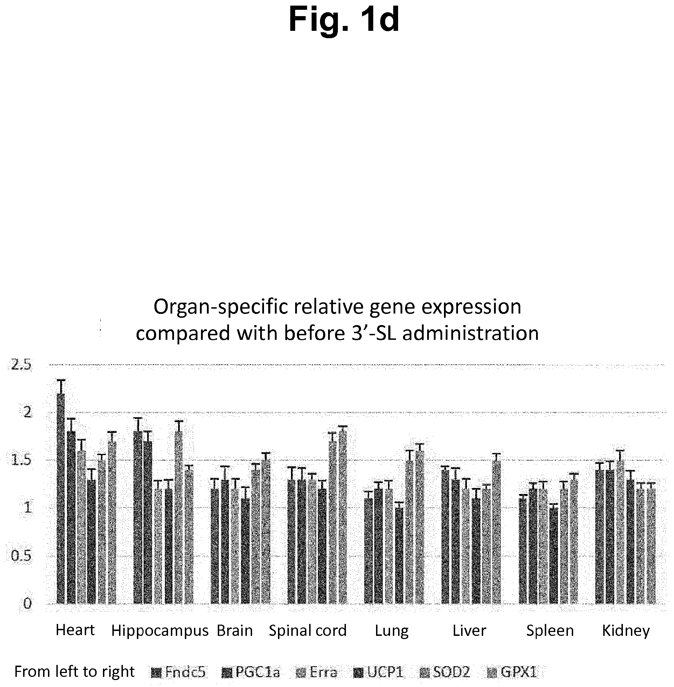

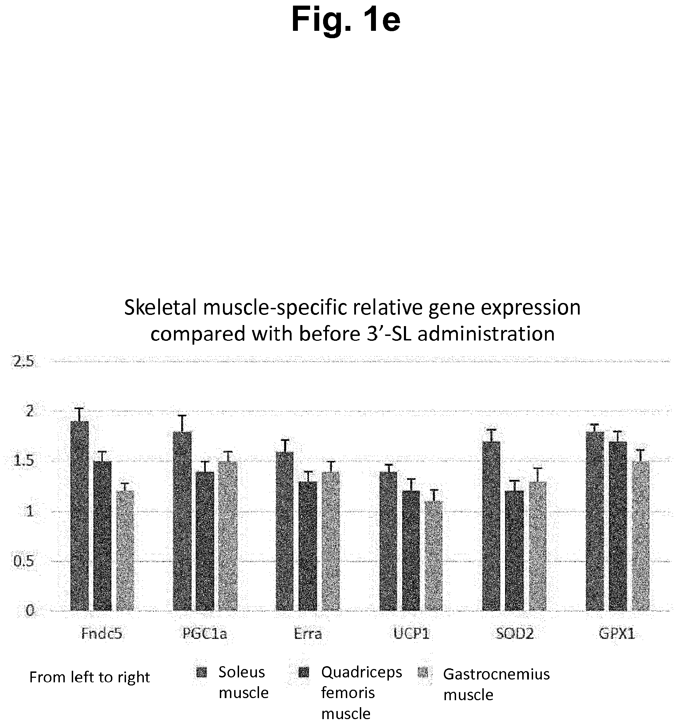

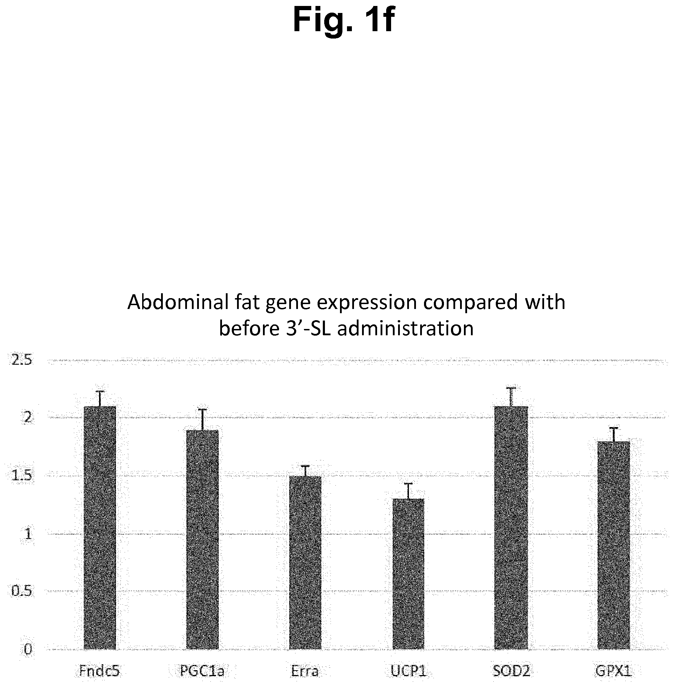

The gene expression changes by administration of the SL (3'-SL & 6'-SL) compositions were quantitatively compared for eight main organs (heart, hippocampus, brain, spinal cord, lung, liver, spleen, and kidney), three skeletal muscles (soleus muscle, quadriceps femoris muscle, and gastrocnemius muscle), and abdominal fat. RNA was extracted by TRIzol agent (Invitrogen). cDNA was synthesized by using RNA, which has been extracted as above and quanti-fied, and a reverse transcription system (Promega, USA). The expression patterns of PGC-la and related genes were measured by using pre-designed primers and probes (Applied Biosystems; PGC-1α, Mm00447181_m1, GAPDH, and Mm99999915_q1) for the synthesized cDNA and analysis targets (Fndc5, PGC-1α, Erra, UCP1, SOD2, and GPX1). The Rotor-Gene 3000 system (Corbett Research, Sydney, Austrailia) was used for PCR reaction and analysis, and the the results are shown in FIG. 1.

In FIG. 1, the relative gene expression changes by organs compared with before SL (3'-SL & 6'-SL) aministration were expressed by values of (gene expression levels of SL-administration groups)/(gene expression level of the control group). As for 6'-SL, as can be seen from most of the organs (FIG. 1a), skeletal muscles (FIG. 1b), and abodominal fat (FIG. 1c), the 6'-SL administration groups were higher than the negative control group in view of the expression levels of several analysis targets (Fndc5, PGC-1α, Erra, and UCP1) including PGC-1α in several body parts. That is, it could be confirmed that 6'-SL stimulated the expression of PGC-1α and related genes in several organs, mucles, and fat of normal mice. As for 3'-SL, as can be seen from most of the organs (FIG. 1d), skeletal muscles (FIG. 1e), and abodominal fat (FIG. 1f), the 3'-SL administration groups were higher than the negative control group in view of the expression levels of several analysis targets in several body parts, but were less effective than the 6'-SL administration groups.

FIGS. 1a, 1b, and 1c show the gene expression changes in organs (FIG. 1a), skeletal muscles (FIG. 1b), and abdominal fat (FIG. 1c) by the treatment with 6'-SL in normal mouse models. FIGS. 1d, 1e, and 1f show the gene expression changes in organs (FIG. 1d), skeletal muscles (FIG. 1e), and abdominal fat (FIG. 1f) by the treatment with 3'-SL in normal mouse models. In FIGS. 1a to 1f, the vertical axes represent the relative gene expression ratio compared with the control group.

### Example 2: Evaluation of PGC-la protein expression stimulation by the treatment with SL (3'-SL & 6'-SL) compositions in the brain and hippocampus of old mouse models

In order to investigate the relative protein expression in the brain compared with before SL (3'-SL & 6'-SL) aministration, 4-week-old ICR mice were purchased from Central Lab Animal (Korea). Water was freely accessible, and a commercially available pellet feed (Dooyeul Biotech, Korea) was given for two weeks. At 6 weeks of age, the mice (initial body weight, average 20.3±1.5 g) were randomly divided into three groups (composed of eight mice for each group) below, and the diets were maintained for 42 weeks (a total of 24 animals).
- Control group: Normal diet mouse group
- 3'-SL administration group: Treatment with 3'-sialyllactose (3'-SL, Sigma) (oral administration of 0.1 mg per kg of mouse weight per day) in addition to the normal diet group
- 6'-SL administration group: Treatment with 6'-sialyllactose (6'-SL, Sigma) (oral administration of 0.1 mg per kg of mouse weight per day) in addition to the normal diet group

Sialyllactose or DW was orally administered daily. The mice were kept in animal rooms for 42 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2→3)-β-D-Gal-(1→4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

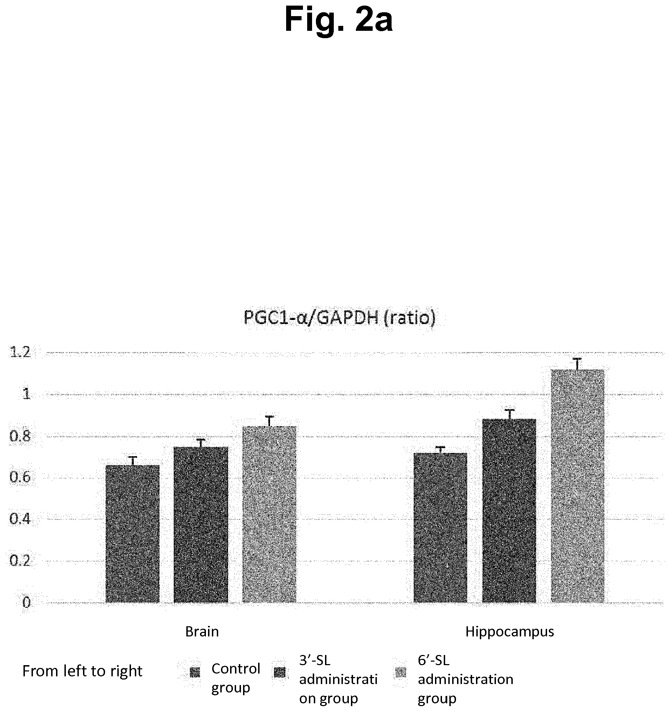

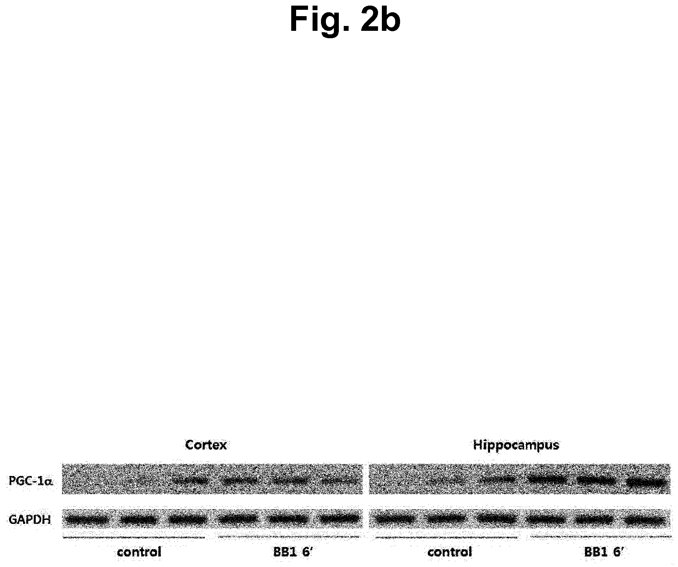

FIG. 2 shows numerical values (FIG. 2a) and western blots (FIG. 2b) of PGC-la protein expression changes in the brain and hippocampus. In FIG. 2b, BB1 6' represents 6'-SL. The "PGC-1α protein expression level" and "GAPDH protein expression level" in each site were quanti-fied, and the relative ratio of the values, (PGC-1α protein expression level)/(GAPDH protein expression level) was numerically expressed for the the control group, 3'-SL administration group, and 6'-SL SL administration group. The PGC-la expression level was much higher in the 6'-SL administration group rather than the negative control group in both of the brain and hippocampus. That is, it could be confirmed that 6'-SL stimulated the expression of PGC-la protein in several sites of the brain of normal mice.

FIG. 2 shows the PGC-la protein expression changes in the brain and hippocampus by the administration of SL compositions in aged mouse models. 6-Week-old male ICR mice were grouped into the control group and the 6'-SL administration group (8 animals per group), which were then subjected to a dietary control test for 42 weeks. The mice were sacrificed and then the brain and hippocampus were extracted. The cerebral cortex was extracted from the brain. The PGC-la protein expression changes in the extracted brain and hippocampus were expressed by numerical values (FIG. 2a) and western blots (FIG. 2b).

### Example 3: Evaluation of gene expression stimulation by treatment with SL (3'-SL & 6'-SL) compositions in nerve cell test

In order to investigate whether SL (3'-SL & 6'-SL) also has an effect of stimulating the expression of PGC-1α gene and related genes in nerve cells, the following test was carried out.

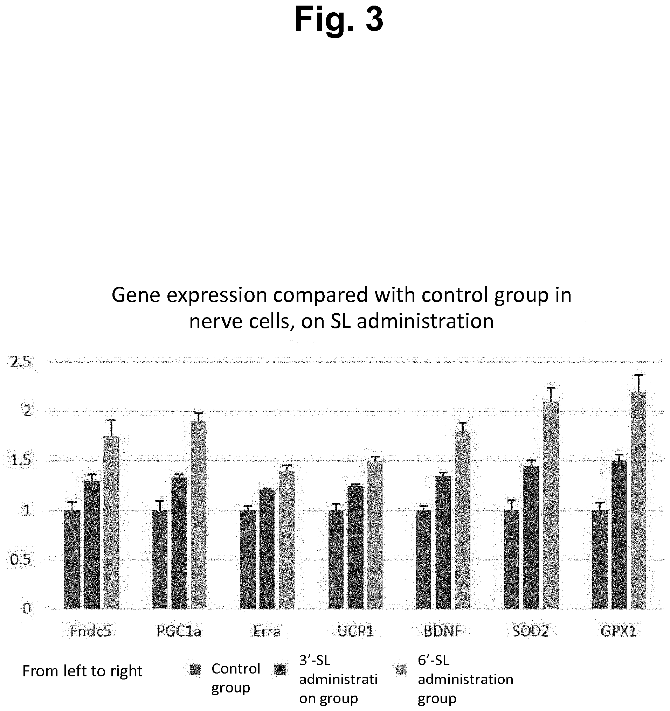

Neuroblasts (Neuro-2a, American Type Culture Collection, USA) were cultured in DMEM supplemented with 10% fetal bovine serum, 100U penicillin, and 0.1 mg/mL streptomycin in each well of 6-well plates at 37°C in 5% CO₂/95% atmospheric conditions. Neuro-2a cells correspond to the fast-growing mouse neuroblastoma cell line. After Neuro-2a cells were seeded at a density of 6000 cells per well, SL was added at a concentration of 0.1 mg/ml into the wells showing a confluency of about 5×10⁶ cells/ml, followed by incubation for 24 hours under the same conditions. SL (3'-SL or 6'-SL) materials were the same as those described in Example 1. Unless otherwise stated, SL (3'-SL or 6'-SL) and the use concentrations thereof are understood to be the same as those described in Example 1. The negative control group was treated with physiological saline with 1/1000 of the volume of the medium. The cells treated with each sample were incubated at 37°C for 24 hours, and then washed twice with cool saline solution, and RNA was extracted using TRIzol agent (Invitrogen). The expression patterns of PGC-la and related genes were measured by using pre-designed primers and probes (Applied Biosystems; PGC-1α, Mm00447181_m1, GAPDH, and Mm99999915_q1) for the synthesized cDNA and analysis targets (Fndc5, PGC-1α, Erra, UCP1, BDNF, SOD2, and GPX1). The Rotor-Gene 3000 system (Corbett Research, Sydney, Australia) was used for PCR reaction and analysis, and the results are shown in FIG. 3.

FIG. 3 shows the gene expression changes by the treatment with SL (3'-SL & 6'-SL) composition in nerve cells. As shown in FIG. 3, as a result of comparing the administration groups, in which Neuro-2a cells were treated with SL (3'-SL & 6'-SL) for 24 hours, and the control group without the treatment, the SL (3'-SL & 6'-SL) administration groups showed significant increases in expression levels of several analysis targets (Fndc5, PGC-1α, Erra, UCP1, BDNF, SOD2, and GPX1) including PGC-1α, compared with the negative control group. It could be confirmed that 6'-SL stimulated the expression of PGC-la and related genes in nerve cells, and the stimulation effect of 3'-SL was lower than that of 6'-SL.

### Example 4: Evaluation of gene expression stimulation by treatment with SL (3'-SL & 6'-SL) compositions in muscle cell test

In order to investigate whether SL actually has an effect of stimulating the expression of PGC-1α gene in mucle cells, the following test was carried out.

C2C12 immanture muscle cells were prepared from the purchase from American Tissue Culture Collection (ATCC, USA). The cells were cultured in Dulbecco's modified Eagle's medium (DMEM, Gibco, USA) supplemented with 10% fetal bovine serum (FBS, USA) in a 5% CO₂ incubator at 37°C until the cells grew to confluency of 70%, while the medium was exchanged every two days. The differentiation into muscle cells was induced by culturing in a medium containing 2% horse serum (HS, Gibco, USA). The mucle cells cultured in the medium containing 2% HS for 4 days were treated with SL with various concentrations. The negative control group was treated with physiological saline with 1/1000 of the volume of the medium. The cells treated with each sample were incubated at 37°C for 24 hours, and then washed twice with cool saline solution, and RNA was extracted using TRIzol agent (Invitrogen). cDNA was synthesized by using 1*µ*g/*µ*ℓ of RNA, which has been extracted as above and quantified, and a reverse transcription system (Promega, USA).

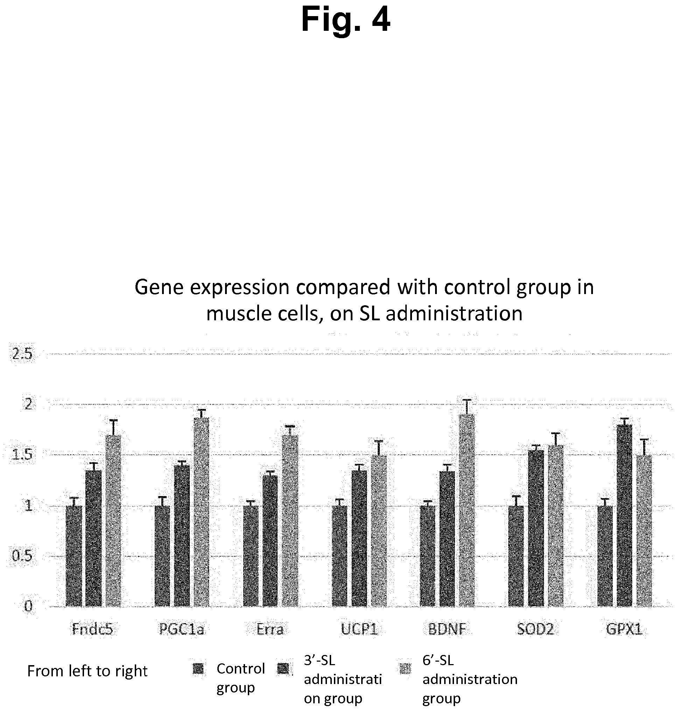

The expression patterns of PGC-la and related genes were measured by using pre-designed primers and probes (Applied Biosystems; PGC-la, Mm00447181_m1, GAPDH, and Mm99999915_q1) for the synthesized cDNA and analysis targets (Fndc5, PGC-1α, Erra, UCP1, SOD2, and GPX1). The Rotor-Gene 3000 system (Corbett Research, Sydney, Australia) was used for PCR reaction and analysis, and the results are shown in FIG. 4.

FIG. 4 shows the gene expression changes by the treatment with SL (3'-SL & 6'-SL) compositions in C2C12 immature musclue cells. As shown in FIG. 4, as a result of comparing the administration groups, in which C2C12 immature mucle cells were treated with SL (3'-SL & 6'-SL) for 24 hours, and the control group without the treatment, the SL (3'-SL & 6'-SL) administration groups showed significant increases in expression levels of several analysis targets (Fndc5, PGC-1α, Erra, UCP1, BDNF, SOD2, and GPX1) including PGC-1α, compared with the negative control group. It could be confirmed that 6'-SL stimulated the expression of PGC-1α and related genes in C2C12 immature mucle cells, and the stimulation effect of 3'-SL was lower than that of 6'-SL.

### Example 5: Evaluations of gene expression stimulation and cognitive ability improvement by treatment with SL (3'-SL & 6'-SL) compositions in Alzheimer's brain disease models

In order to investigate the changes in gene expression and cognitive ability by the treatment with SL (3'-SL & 6'-SL) compositions in Alzheimer's brain disease models, 6-week-old mice were purchased from Central Lab Animal (Korea). Water was freely accessible, and a commercially available pellet feed (Dooyeul Biotech, Korea) was given for two weeks. At 8 weeks of age, the mice were divided into three groups (composed of eight mice for each group): Eight 8-week-old male c57/BL6 mice (normal mice; initial body weight, average 35.6 ± 3.3g) treated with a normal diet were used for a control group. The 8-week-old male Alzheimer's disease model mice (3xTg; initial body weight, average 33.9±2.8g) were radndomly divided into three different dietary treatment groups (eight mice per group) below, and these diets were maintained for 10 weeks (total 32 animals):
- Control group: Normal mice fed with a normal diet without SL administration (8 animals)
- (Alzheimer's disease model) group: Alzheimer disease models fed with a normal diet without SL administration (8 animals)
- (Alzheimer's disease model + 3'-SL) group: Alzheimer disease models separately administered with 3'-sialyllactose (3'-SL, Sigma) (oral administration of 0.1 mg per kg of mouse weight per day) in addition to the normal dietary group (8 animals)
- (Alzheimer's disease model + 6'-SL) group: Alzheimer disease models separately administered with 6'-sialyllactose (6'-SL, Sigma) (oral administration of 0.1 mg per kg of mouse weight per day) in addition to the normal dietary group (8 animals)

Sialyllactose or DW was orally administered daily. The mice were kept in animal rooms for 10 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2→3)-β-D-Gal-(1→4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

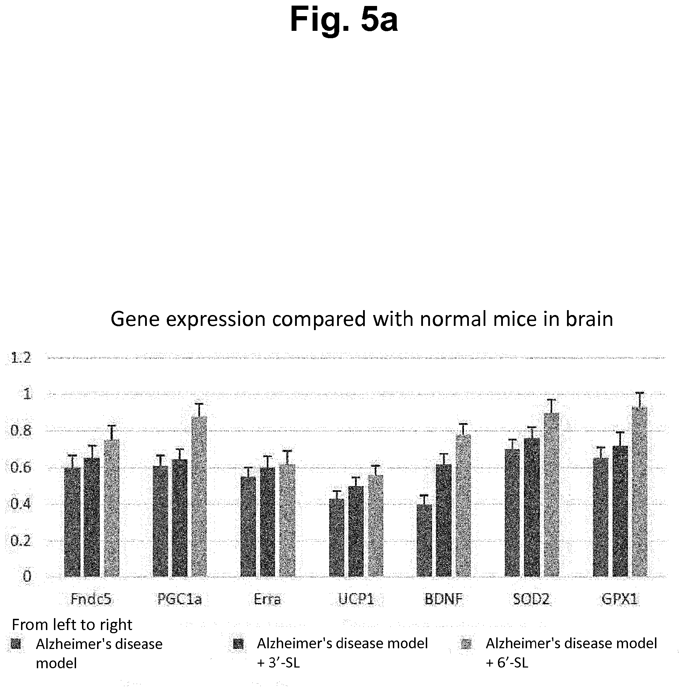

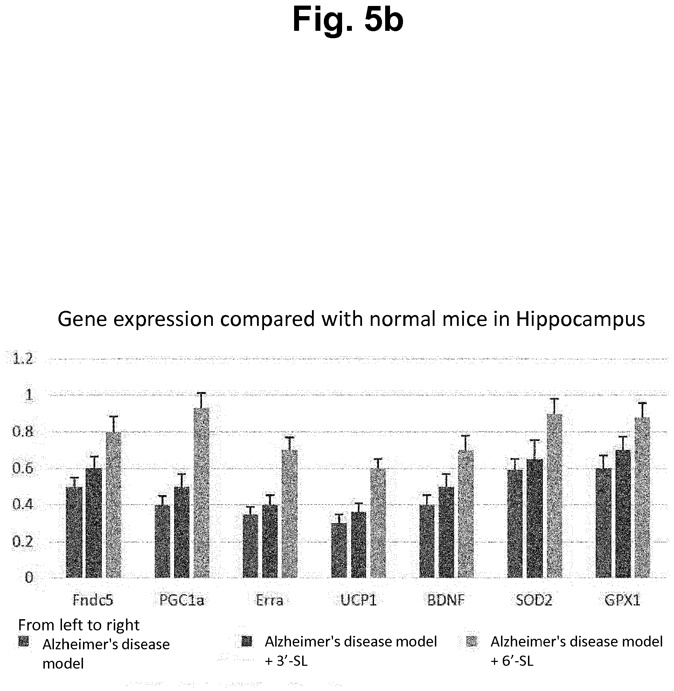

The gene expression changes by the treatment with SL (3'-SL & 6'-SL) compositions were quantitatively compared in two main organs (brain and hippocampus) associated with bran diseases. The cerebral cortex was used for the brain. RNA was extracted by TRIzol agent (Invitrogen). cDNA was synthesized by using RNA, which has been extracted as above and quantified, and a reverse transcription system (Promega, USA). The expression patterns of PGC-la and related genes were measured by using pre-designed primers and probes (Applied Biosystems; PGC-1α, Mm00447181_m1, GAPDH, and Mm99999915_q1) for the synthesized cDNA and analysis targets (Fndc5, PGC-1α, Erra, UCP1, BDNF, SOD2, and GPX1). The Rotor-Gene 3000 system (Corbett Research, Sydney, Australia) was used for PCR reaction and analysis, and the results are shown in FIG. 5.

FIG. 5 shows the gene expression changes by the treatment with SL (3'-SL & 6'-SL) compositions in Alzheimer disease models. FIG. 5 shows the numerical values of (gene expression level of the (Alzheimer's disease model) group)/gene expression level of the control group), (gene expression level of the (Alzheimer's disease model + 3'-SL) group)/gene expression level of the control group), and (gene expression level of the (Alzheimer's disease model + 6'-SL) group)/gene expression level of the control group) in respective organs with respect to relative gene expression changes in the brain and hippocampus compared with the control group. The 8-week-old male Alzheimer's disease model (3xTg) mice were grouped into SL administration groups and an SL non-administration group (8 animals per group), and then subjected to a dietary control test for 10 weeks, and the gene expression levels in the test groups were compared with that in the normal mice (c57/BL6) with a normal diet. As shown in FIG. 5, on the basis of the normal mouse control group with a normal diet, the expression levels of several analysis targets (Fndc5, Erra, UCP1, BDNF, SOD2, and GPX1) including PGC-1α in the brain (FIG. 5a) and hippocampus (FIG. 5b) were somewhat lower in the 6'-SL administration (Alzheimer's disease model + 6'-SL) group compared with the normal control group, but were very high in the 6'-SL administration (Alzheimer's disease model + 6'-SL) group compared with the SL-non-administration (Alzheimer's disease model) group. It could be seen that 3'-SL was relatively low compared with 6'-SL in view of related gene expression stimulation effects. That is, 6'-SL showed significant increases in expression levels of PGC-la and related genes (Fndc5, PGC-1α, Erra, UCP1, BDNF, SOD2, and GPX1) in in the brain and hippocampus of Alzhhimer's deisease model model mice.

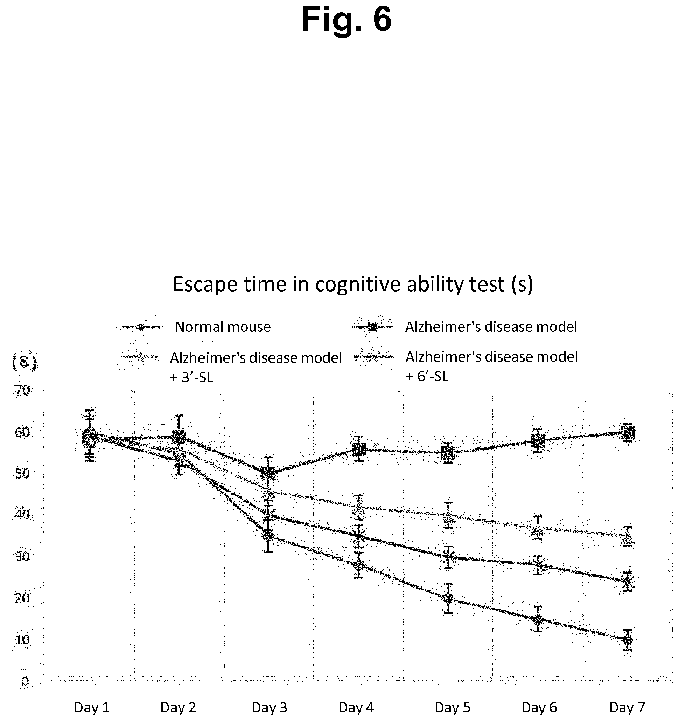

FIG. 6 shows the numerical values of escape time in the cognitive ability test when a control group, an (Alzheimer's disease model) group, an (Alzheimer's disease model + 3'-SL) group, and an (Alzheimer's disease model + 6'-SL) group were subjected to the cognitive ability test (Morris water maze) for 7 days. It could be seen that the escape tim in the cognitive ability test was almost constant without improvement even after the lapse of the test time in the SL non-administration (Alzheimer's disease model) group, but the escape time in the test in the 6'-SL administration (Alzheimer's disease model + 6'-SL) group was definitely fast, but somewhat decreased compared with the normal mouse control group with a normal diet. It could be seen that 3'-SL was relatively low compared with 6'-SL in view of the cognitive ability improvement effects. That is, it could be confirmed that 6'-SL improved the cognitive ability best in Alzheimer's disease model mice.

### Example 6: Evaluations of gene expression stimulation and behavior improvement by treatment with SL (3'-SL & 6'-SL) compositions in Parkinson's brain disease models

In order to investigate the changes in gene expression and behavior improvement by the treatment with SL (3'-SL & 6'-SL) compositions in Parkinson's brain disease models, 13-week-old normal SD rats and Parkinson's brain disease models were purchased from Central Lab Animal (Korea). Water was freely accessible, and a commercially available pellet feed (Dooyeul Biotech, Korea) was given for one week. At 14 weeks of age, the SD rats were divided into four groups (composed of 8 animals per group): Eight 14-week-old male SD rats (normal rats; initial body weight, average 355.6 ± 32.3g) treated with a normal diet were used for a control group. The 6-OHDA induced SD rat Parkinson's disease models, which were administered with 6-hydroxydopamine (6-OHDA) at 8 weeks of age and supplied at 13 weeks of age, were randomly divided into three different dietary treatment groups below (8 animals per group), and these diets were maintained from 14 weeks of age for 10 weeks (total 32 animals):
- Control group: Normal rats fed with a normal diet without SL administration (8 animals)
- (Parkinson's disease model) group: Parkinson's disease models fed with a normal diet without SL administration (8 animals)
- (Parkinson's disease model +3'-SL) group: Parkinson's disease models treated with 3'-sialyllactose (3'-SL, Sigma) (oral administration of 0.1 mg per kg of rat weight per day) in addition to the normal diet group (8 animals)
- (Parkinson's disease model +6'-SL) group: Parkinson's disease models treated with 6'-sialyllactose (6'-SL, Sigma) (oral administration of 0.1 mg per kg of rat weight per day) in addition to the normal diet group (8 animals)

Sialyllactose or DW was orally administered daily. The rats were kept in animal rooms for 10 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2,3)-β-D-Gal-(1,4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

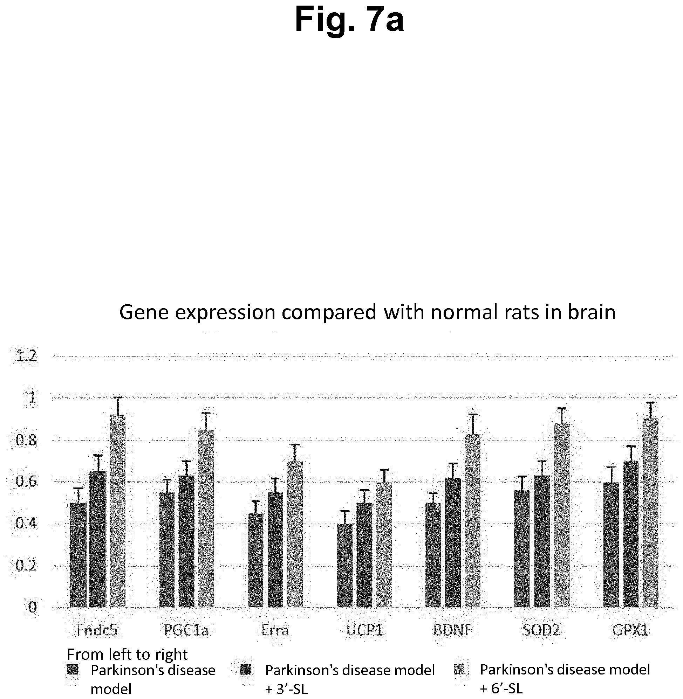

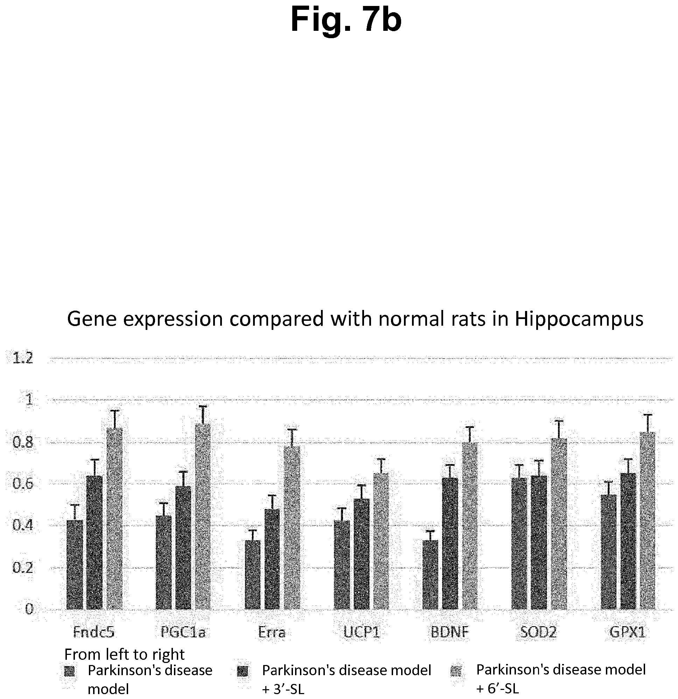

The gene expression changes by the treatment with SL (3'-SL & 6'-SL) compositions were quantitatively compared in two main organs (brain and hippocampus) associated with bran diseases. RNA was extracted by TRIzol TRIzol agent (Invitrogen). cDNA was synthesized by using using RNA, which has been extracted as above and quantified, and a reverse transcription system (Promega, USA). The expression patterns of PGC-la and related genes were measured by using pre-designed primers and probes (Applied Biosystems; PGC-1α, Mm00447181_m1, GAPDH, and Mm99999915_q1) for the synthesized cDNA and analysis targets (Fndc5, PGC-1α, Erra, UCP1, BDNF, SOD2, and GPX1). The Rotor-Gene 3000 system (Corbett Research, Sydney, Australia) was used for PCR reaction and analysis, and the results are shown in FIGS. 7a and 7b.

FIGS. 7a and 7b show the gene expression changes by the treatment with SL (3'-SL & 6'-SL) compositions in Parkinson's disease models. The 6-OHDA induced SD rat Parkinson's disease models, which were administered with 6-OHDA at 8 weeks of age and supplied at 13 weeks of age, were grouped into SL (3'-SL & 6'-SL) administration groups and an SL non-administration group (8 animals per group), and then subjected to a dietary control test for 8 weeks, and the gene expression levels of the test groups were compared with that in the control group in which 13-week-old male SD rats (normal) were fed with a normal diet. FIGS. 7a and 7b show the numerical values of (gene expression level of the (Parkinson's disease model) group)/gene expression level of the control group), (gene expression level of the (Parkinson's disease model + 3'-SL) group)/gene expression level of the control group), and (gene expression level of the (Parkinson's disease model + 6'-SL) group)/gene expression level of the control group) in respective organs with respect to the gene expression changes in the brain and hippocampus compared with the normal rats. As a result, as shown in FIGS. 7a and 7b, on the basis of the normal rat control group with a normal diet, the expression levels of several analysis targets (Fndc5, Erra, UCP1, BDNF, SOD2, and GPX1) including PGC-la in the brain (FIG. 7a) and hippocampus (FIG. 7b) were somewhat lower in the 6'-SL administration (Parkinson's disease model + 6'-SL) compared with the normal control group, but were very high in the 6'-SL administration group compared with the SL non-administration (Parkinson's disease model) group. It could be seen that 3'-SL was relatively low compared with 6'-SL in view of the gene expression stimulation effects. That is, 6'-SL showed significant increases in expression levels of PGC-1a and related genes in the brain and hippocampus of Parkinson's disease model rats.

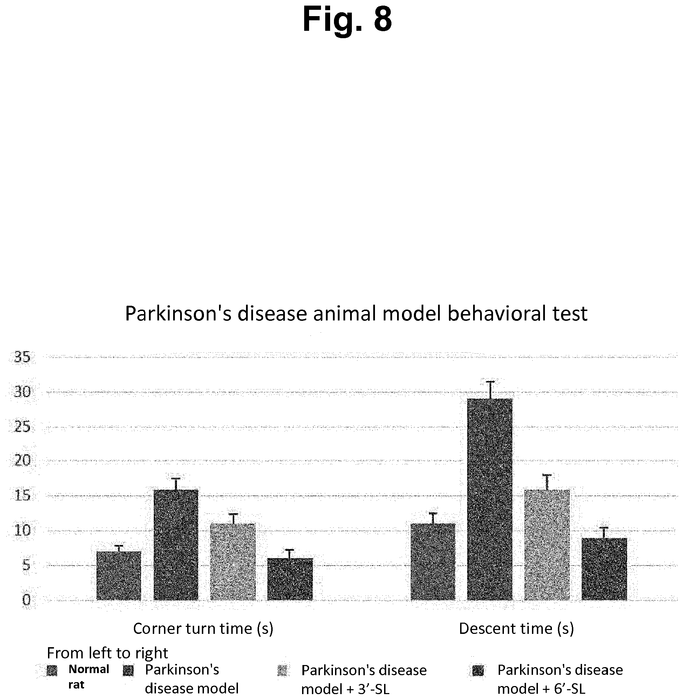

FIG. 8 shows the behavior test results on Parkinson's disease models. In FIG. 8, the vertical axis represents the corner turn time and the descent time as a result of carrying out behavioral ability tests (corner turn and descent) using a vertical grating device on a control group, (Parkinson's disease model) group, (Parkinson's disease model + 3'-SL) group, and (Parkinson's disease model + 6'-SL) group. The vertical grating device was in the form of an opened box of 5cm × 55cm × 8cm, and the frant face was formed of a wiring of 0.8cm × 0.8cm and the other surfaces were formed of black plexiglass. The bottom was made 5 cm longer for safety. In the test, the mice were placed in an upward direction at a distance of 3 cm from the top of the device, and were allowed to acclimate to the device three times for two days before testing. The above procedure was repeated if the rat could not come down within 60 seconds. The corner turn time is the time it takes for a rat facing upwards to turn downwards, and the descent time is the time obtained by subtracting the corner turn time from the overall test time for which the rat turns at the corner and descends to the bottom.

As shown in FIG. 8, the corner turn time and descent time in the SL non-administration (Parkinson's disease model) group were two or three times longer than those in the normal rat control group with a normal diet, and the corner turn time and descent time in the 6'-SL administration (Parkinson's disease model + 6' SL) group were almost similar to those in the control group. That is, it could be confirmed that 6'-SL improved behavioral ability (corner turn or descent) in Parkinson's disease model rats. It could be seen that 3'-SL was relatively low compared with 6'-SL in view of the behavioral ability improvement effects.

### Example 7: Evaluation of gene expression stimulation by treatment with SL (3'-SL & 6'-SL) compositions in epilepsy/convulsion brain disease models

In order to investigate the gene expression changes by the treatment with SL (3'-SL & 6'-SL) compositions in epilepsy/convulsion brain disease models, 4-week-old normal SD rats and epilepsy/convulsion brain disease models (Noda epileptic rat, NER) were purchased from Central Lab Animal (Korea). Water was freely accessible, and a commercially available pellet feed (Dooyeul Biotech, Korea) was given for two weeks. At 6 weeks of age, the SD rats were divided into four groups (composed of 8 animals per group): Eight 6-week-old male SD rats (normal rats; initial body weight, average 176.3 ± 13.3g) treated with a normal diet were used for a control group. The 6-week-old male epilepsy/convulsion brain disease models (initial body weight, average 181.8±11.3g) were randomly divided into three different dietary treatment groups (8 animals per group) below, and these diets were maintained from 6 weeks of age for 10 weeks (total 32 animals):
- Control group: Normal rats fed with a normal diet without SL administration (8 animals)
- (Epilepsy/convulsion brain disease model) group: Epilepsy/convulsion brain disease models fed with a normal diet without SL administration (8 animals)
- (Epilepsy/convulsion brain disease model + 3'-SL) group: Epilepsy/convulsion brain disease models treated with 3'-sialyllactose (3'-SL, Sigma) (oral administration of 0.1 mg per kg of rat weight per day) in addition to the normal dietary group (8 animals)
- (Epilepsy/convulsion brain disease model + 6'-SL) group: Epilepsy/convulsion brain disease models treated with 6'-sialyllactose (6'-SL, Sigma) (oral administration of 0.1 mg per kg of rat weight per day) in addition to the normal dietary group (8 animals)

Sialyllactose or DW was orally administered daily. The rats were kept in animal rooms for 10 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2→3)-β-D-Gal-(1→4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

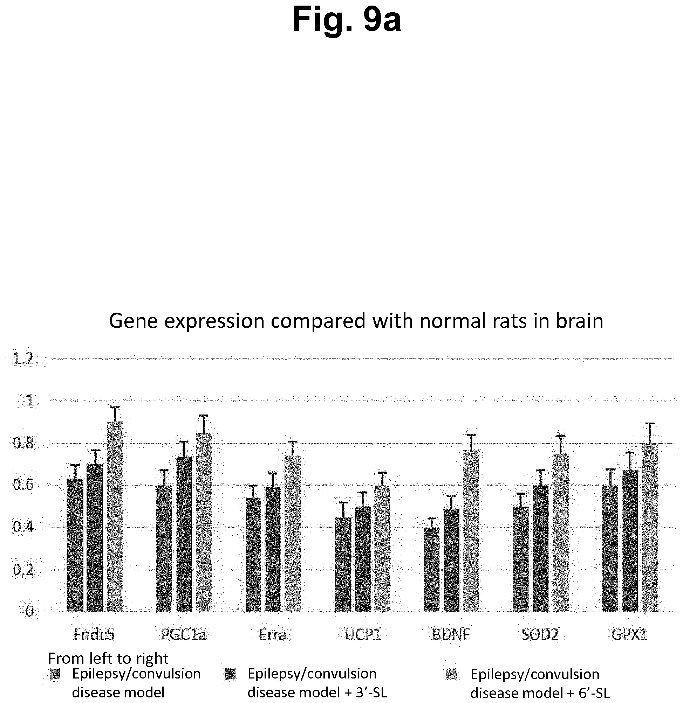

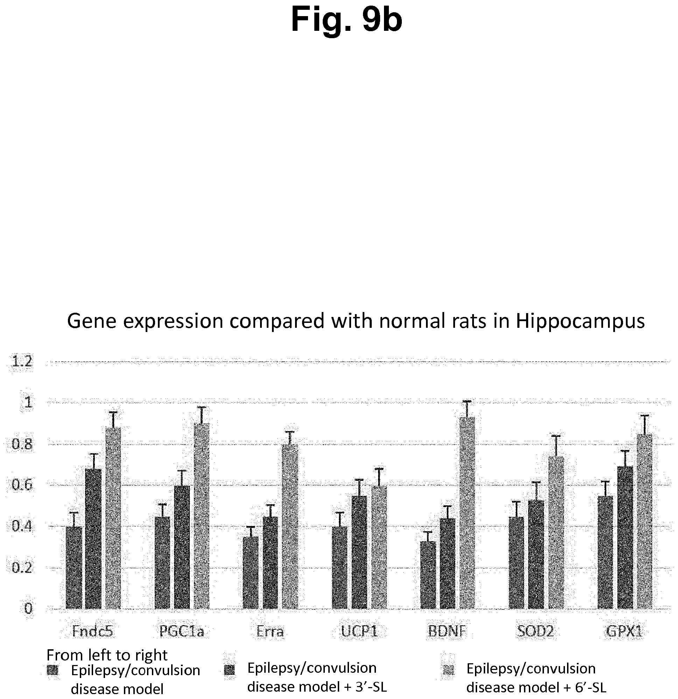

The gene expression changes by the treatment with SL (3'-SL & 6'-SL) compositions were quantitatively compared in two main organs (brain and hippocampus) associated with bran diseases. RNA was extracted by TRIzol agent (Invitrogen). cDNA was synthesized by using RNA, which has been extracted as above and quantified, and a reverse transcription system (Promega, USA). The expression patterns of PGC-la and related genes were measured by using pre-designed primers and probes (Applied Biosystems; PGC-la, Mm00447181_m1, 999915_q1) for the synthesized cDNA and analysis targets (Fndc5, PGC-1α, Erra, UCP1, BDNF, SOD2, and GPX1). The Rotor-Gene 3000 system (Corbett Research, Sydney, Australia) was used for PCR reaction and analysis, and the results are shown in FIG. 9.

FIGS. 9a and 9b show the gene expression changes by the by the treatment with SL compositions in epilepsy/convulsion brain disease models. In FIGS. 9a and 9b, the vertical axes represent the numerical values of (gene expression level of the (epilepsy/convulsion brain disease model) group)/gene expression level of the control group), (gene expression level of the (epilepsy/convulsion brain disease model + 3'-SL) group)/gene expression level of the control group), and (gene expression level of the (epilepsy/convulsion brain disease model + 6'-SL) group)/gene expression level of the control group) in respective organs with respect to relative gene expression changes in the brain or hippocampus compared with the control group. The 6-week-old epilepsy/convulsion brain disease models were grouped into SL administration groups and an SL non-administration group (8 animals per group), and then subjected to a dietary control test for 10 weeks, and the gene expression levels in the test groups were compared with that in the control group in which 6-week-old male SD rats (normal) were fed with a normal diet. As a result, as shown in FIGS. 9a and 9b, on the basis of the normal rat control group with a normal diet, the expression levels of several analysis targets (Fndc5, Erra, UCP1, BDNF, SOD2, and GPX1) including PGC-1a in the brain (FIG. 9a) and hippocampus (FIG. 9b) were somewhat lower in the SL administration (epilepsy/convulsion brain disease model + SL) groups compared with the normal control group, but were very high in the SL administration groups compared with the SL non-administration (epilepsy/convulsion brain disease model) group. That is, 6'-SL showed significant increases in expression levels of PGC-la and related genes in the brain and hippocampus of epilepsy/convulsion brain disease models rats. It could be seen that 3'-SL was relatively low compared with 6'-SL in view of the gene expression stimulation effects.

### Example 8: Evaluations of gene expression stimulation and rotarod travel time improvement by treatment with SL (3'-SL & 6'-SL) compositions in Huntington's brain disease models

In order to investigate the changes in gene expression and cognitive ability by the treatment with SL (3'-SL & 6'-SL) compositions in Huntington's brain disease model, 4-week-old mice were purchased from Central Lab Animal (Korea). Water was freely accessible, and a commercially available pellet feed (Dooyeul Biotech, Korea) was given for one week. At 5 weeks of age, the mice were divided into three groups (composed of eight mice for each group) : Eight 5-week-old male c57/BL6 mice (normal mice; initial body weight, average 25.3 ± 4.3g) treated with a normal diet were used for a control group. The 5-week-old male Huntington's disease model mice (R6/2 series (B6CBATg (HDexon1) 62Gpb/ 3J, 111 CAGs) transgenic HD mice; initial body weight, average 26.9±4.8 g) were randomly divided into three different dietary treatment groups (eight mice per group), and these diets were maintained for 10 weeks (total 32 animals):
- Control group: Normal mice fed with a normal diet without SL administration (8 animals)
- (Huntington's disease model) group: Huntington's disease models fed with a normal diet without SL administration (8 animals)
- (Huntington's disease model +3'-SL) group: Huntington's disease models treated with 3'-sialyllactose (3'-SL, Sigma) (oral administration of 0.1 mg per kg of mouse weight per day) in addition to the normal diet group (8 animals)
- (Huntington's disease model +6'-SL) group: Huntington's disease models treated with 6'-sialyllactose (6'-SL, Sigma) (oral administration of 0.1 mg per kg of mouse weight per day) in addition to the normal diet group (8 animals)

Sialyllactose or DW was orally administered daily. The mice were kept in animal rooms for 10 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2→3)-[3-D-Gal-(1→4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

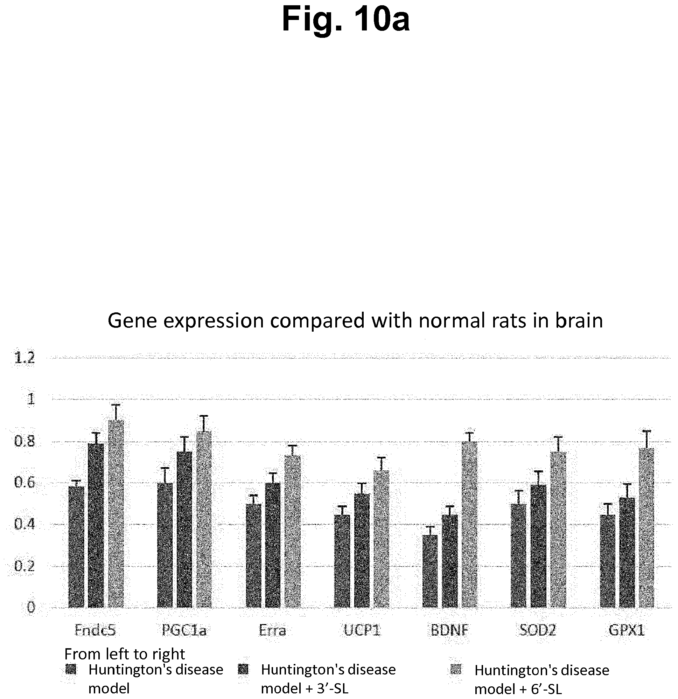

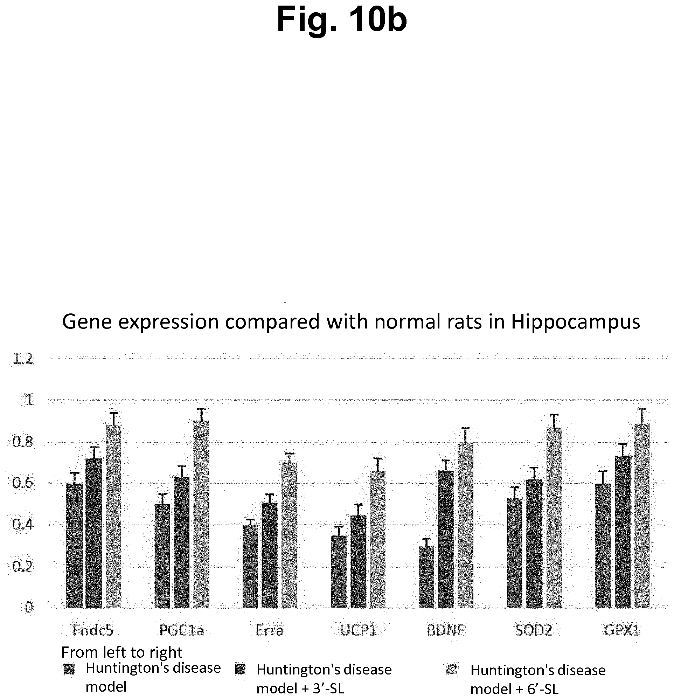

The gene expression changes by the treatment with SL (3'-SL & 6'-SL) compositions were quantitatively compared in two main organs (brain and hippocampus) associated with bran diseases. RNA was extracted by TRIzol agent (Invitrogen). cDNA was synthesized by using RNA, which has been extracted as above and quantified, and a reverse transcription system (Promega, USA). The expression patterns of PGC-la and related genes were measured by using pre-designed primers and probes (Applied Biosystems; PGC-la, Mm00447181_m1, GAPDH, and Mm99999915_q1) for the synthesized cDNA and analysis targets (Fndc5, PGC-1α, Erra, UCP1, BDNF, SOD2, and GPX1). The Rotor-Gene 3000 system (Corbett Research, Sydney, Australia) was used for PCR reaction and analysis, and the results are shown in FIG. 10.

FIGS. 10a and 10b show the gene expression changes by the treatment with SL (3'-SL & 6'-SL) compositions in Huntington's disease models. FIGS. 10a and 10b show the numerical values of (gene expression level of the (Huntington's disease model) group)/gene expression level of the control group), (gene expression level of the (Huntington's disease model + 3'-SL) group)/gene expression level of the control group), and (gene expression level of the (Huntington's disease model + 6'-SL) group)/gene expression level of the control group) in respective organs with respect to the gene expression changes in the brain and hippocampus compared with the normal mice control group. The 5-week-old male Huntington's disease model mice were grouped into SL (3'-SL & 6'-SL) administration groups and an SL non-administration group (8 animals per group), and then subjected to a dietary control test for 10 weeks, and the gene expression levels in the test groups were compared with that in the normal mice (c57/BL6) with a normal diet. As a result, as shown in FIGS. 10a and 10b, on the basis of the normal mouse control group with a normal diet, the expression levels of several analysis targets (Fndc5, Erra, UCP1, BDNF, SOD2, and GPX1) including PGC-la in the brain (FIG. 10a) and hippocampus (FIG. 10b) were somewhat lower in the 6'-SL administration (Huntington's disease model + 6'-SL) group compared with the normal control group, but were very excellent in the 6'-SL administration group compared with the SL non-administration (Huntington's disease model) group. That is, 6'-SL showed significant increases in expression levels of PGC-la and related genes in the brain and hippocampus of Huntington's disease model mice. It could be seen that 3'-SL was relatively low compared with 6'-SL in view of the gene expression stimulation effects.

In addition, the behavior improvement was evaluated through the rotarod travel time changes of the (Huntington's disease model) group, (Huntington's disease model + 3'-SL) group, and (Huntington's disease model + 6'-SL) group, compared with the normal mouse with a normal diet. The rotarod travel test is to investigate the changes of the numerical values of (rotarod travel time of (Huntington's disease model) group)/rotarod travel time of the control group), (rotarod travel time of the (Huntington's disease model + 3'-SL) group)/rotarod travel time of the control group), and (rotarod travel time of the (Huntington's disease model + 6'-SL) group)/rotarod travel time of the control group). The rotarod travel time was measured by using a rotarod device (rod accelerating at a revolution speed of 4-40 rpm through 3 minutes, Jungdo Instruments, Korea). After 4-week-old mice were practiced on the rotarod test, the rotarod test was performed from 5 weeks of age, and the mean time to fall was measured.

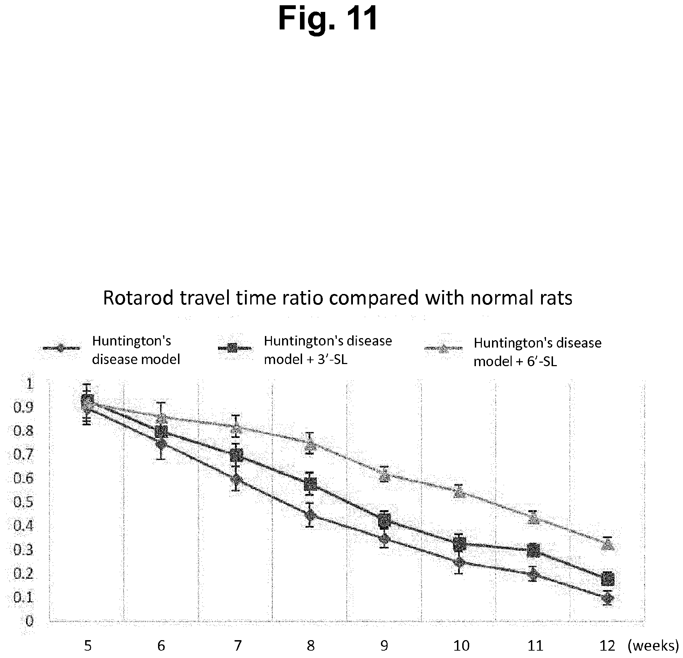

FIG. 11 shows the rotarod travel test results in Huntington's disease models. In FIG. 11, the vertical axis represents the numerical values of (rotarod travel time of (Huntington's disease model) group)/rotarod travel time of the control group), (rotarod travel time of the (Huntington's disease model + 3'-SL) group)/rotarod travel time of the control group), and (rotarod travel time of the (Huntington's disease model + 6'-SL) group)/rotarod travel time of the control group). As a result, as shown in FIG. 11, when the rotarod travel tests for the Huntington's disease model) group, (Huntington's disease model + 3'-SL) group, and (Huntington's disease model + 6'-SL) group were campared with the normal mouse control group with a normal diet, the rotarod travel time was increased in the 6'-SL administration (Huntington's disease model + 6'-SL) group compared with the SL non-administration group (Huntington's disease model) group, and the travel time increasing effect of 3'-SL was less than that of 6'-SL. That is, it could be confirmed that 6'-SL stimulated the behavior improvement best in Huntington's disease models.

### Example 9: Changes in ischemic volume and MLPT score by treatment with SL (3'-SL & 6'-SL) compositions in stroke models

In order to investigate the changes in ischemic volume and modified limb placing test (MLPT) score by treatment with SL (3'-SL & 6'-SL) compositions in stroke models, 4-week-old mice were purchased from Central Lab Animal (Korea). Water was freely accessible, and a commercially available pellet feed (Dooyeul Biotech, Korea) was given for one week. Stroke models involving temporary and permanent middle cerebral artery (MCA) occlusion and intracerebral hemorrhage (ICH) were fabricated using 6-week-old male Sprague-Dawley rats (weight, average 185.3 ± 15.8 g) and male BALB/c mice weight, average 24.6±3.8 g). For the comparision of SL aministration effects, the mice, one hour after stroke induction, were randomly grouped into three different intraperitoneal administration groups (8 animals per group, 24 animals in total) below, and then intraperitoneally administered:
- Control group: Lysis buffer control group medium intraperitoneal administration group (8 animals)
- 3'-SL treatment: 3'-SL lysis buffer medium intraperitoneal administration group (8 animals)
- 6'-SL treatment: 6'-SL lysis buffer medium intraperitoneal administration group (8 animals)

Local cerebral infarction mouse models were used as additional cerebral infarction models, and in these models, SL (3'-SL & 6'-SL) was intraperitoneally administered 3 hours after cerebral infarction introduction:
- Control group: Lysis buffer control group medium intraperitoneal administration group (8 animals)
- 3'-SL treatment: 3'-SL lysis buffer medium intraperitoneal administration group (8 animals)
- 6'-SL treatment: 6'-SL lysis buffer medium intraperitoneal administration group (8 animals)

The ischemic volume measurement was conducted by measuring the volume of the ischemic area (infarct and boundary region thereof) using 2,3,7-triphenyl tetrazolium chloride (TTC), 24 hours after ischemic induction, as follows. After brain extraction, the extraction, the frontal tip was cut into 1 mm thickness, and thickness, and immersed in 2% TCC solution. The stained slices were then fixed with PBS-4% paraformaldehyde, and the ischemic site and hemispherical region of each slice were measured using an image analysis system. The values due to brain edema were corrected as follows: corrected ischemic volume value: measured ischemic area × 1-{[(ipsilateral hemisphere area-contralateral hemisphere area)/contralateral hemisphere]}. The ischemic volume was expressed as a percentage of the total hemispherical volume.

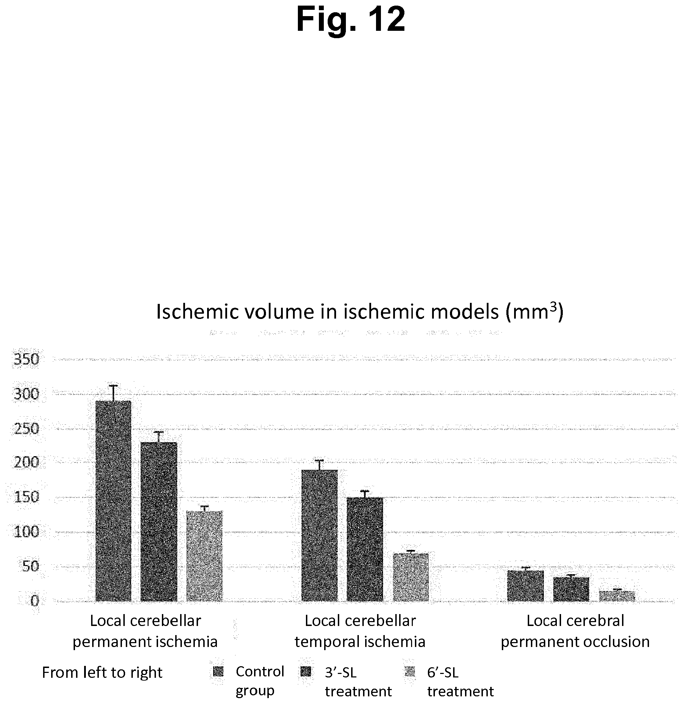

FIG. 12 shows the observation resulst of the ischemic volume in ischemic models. FIG. 12 shows the observation results of the *in vivo* ischemic neuronal damage protective effects of SL when mouse models with temporary and permanent middle cerebral artery (MCA) occlusion were treated with 3'- or 6'-SL. As shown in FIG. 12, the reduction of ischemic volume by 6'-SL treatment were about 50% in all of local cerebellar permanent ischemia models (50%), local cerebellar temporal ischemia models (60%), and local cerebral permanent occlusion models (40%). It could be seen that 3'-SL were relatively low compared with 6'-SL in view of the ischemic neuronal damage protective effects.

In addition, in order to investigate the neurodegeneration inhibition by SL (3'-SL & 6'-SL) in ICH models, the MLPT test was conducted. The MLPT test was conducted one day before, one day after, and three days after ICH induction. The model was suspended at 10 cm above a table, and the stretch of the forelimbs toward the table was scored (0 points for normal stretch and 1 point for abnormal fexion). Next, the forelimbs of the model were allowed to move through the edge, and each forelimb was pulled down gently, and the retrieval and placement were checked (forelimb, second task; hind limb, third task). Finally, the rat was placed toward the table edge to check for lateral placement of the forelimbs. The evaluation results for three tasks were scored in the following manner: 0 points for normal performance, 1 point for performance with a delay (at least 2 seconds) or incomplete performance, and 2 points for no performance. A total of seven points indicates maximal neurological deficit, and a score of 0 points indicates normal performance.

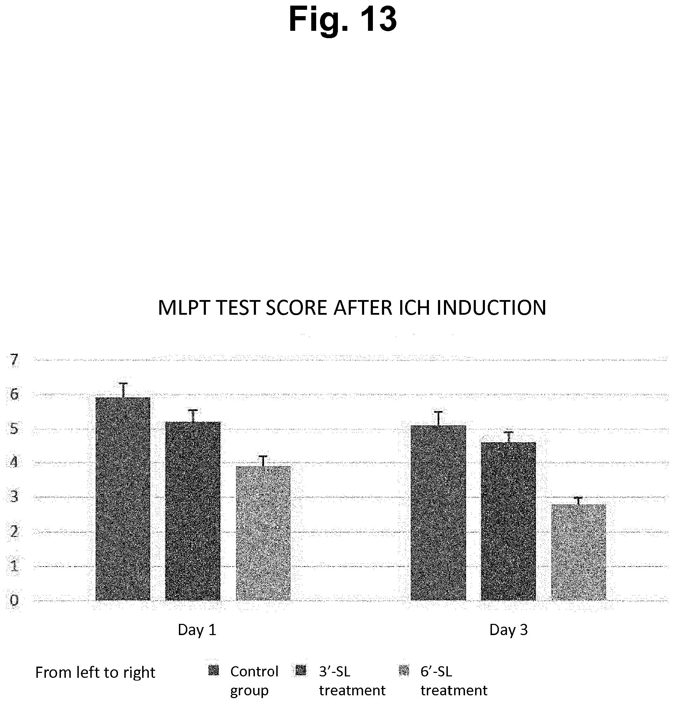

FIG. 13 shows the MLPT test score changes by SL (3'-SL & 6'-SL) treatment after ICH induction As shown in FIG. 13, as can be seen from the results on the first day and the third day after IHC model induction, the MLPT test score was lower when the ICH models were treated with 6'-SL compared with the control group. That is, the neurodegeneration inhibitory effects by 6'-SL treatment were observed. It could be seen that 3'-SL were relatively low compared with 6'-SL in view of the neurodegeneration inhibitory effects.

### Example 10: Abdominal fat gene expression changes and topical fat removal evaluation by treatment with SL (3'-SL & 6'-SL) compostions

In order to investigate gene expression changes and topical fat removal effects by treatment with SL (3'-SL & 6'-SL) compostion in mouse models, 4-week-old male ob mouse (C57BL/6J-ob/ob) models were purchased from Central Lab Animal (Korea). Water was freely accessible, and a high-fat diet (Rodent Diet with 60 kcal% fat) was supplied for two weeks. 6-Week-old male ob mouse (C57BL/6J-ob/ob) models (initial body weight, average 34.2 ± 3.7 g) were randomly grouped into three different dietary treatment groups (8 animals per group) below, and these diets were maintained for 10 weeks (a total of 24 animals):
- Control group: Models fed with a high-fat diet (Rodent Diet with 60 kcal% fat) without SL treatment (8 animals)
- 3'-SL administration group: Models treated with 3'-sialyllactose (3'-SL, Sigma) (oral administration of 0.1 mg per kg of mouse weight per day) in addition to the high-fat diet group (8 animals)
- 6'-SL administration group: Models treated with 6'-sialyllactose (6'-SL, Sigma) (oral administration of 0.1 mg per kg of mouse weight per day) in addition to the high-fat diet group (8 animals)

Sialyllactose or DW was orally administered daily. The mice were kept in animal rooms for 10 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2→3)-β-D-Gal-(1→4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

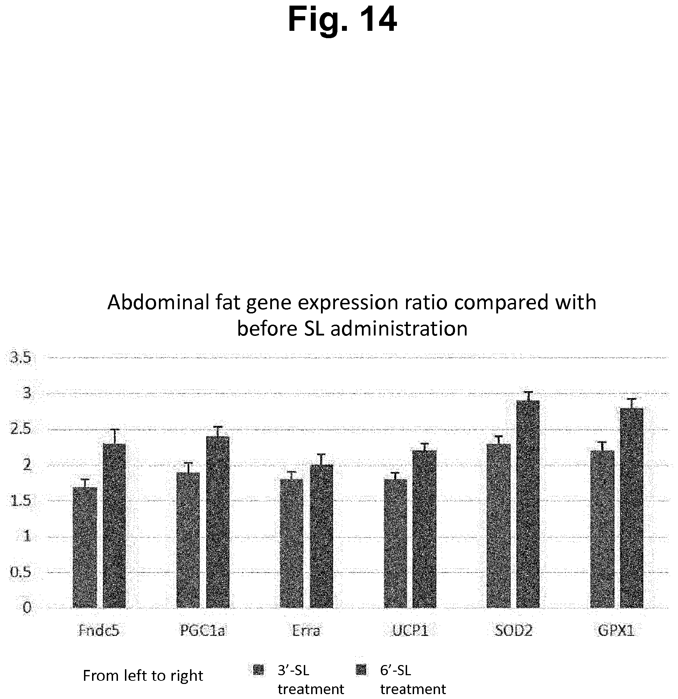

Gene expression changes by the treatment with SL (3'-SL & 6'-SL) compositions were quantitatively compared in the abdominal part. RNA was extracted by TRIzol agent (Invitrogen). cDNA was synthesized by using RNA, which has been extracted as above and quantified, and a reverse transcription system (Promega, USA). The expression patterns of PGC-la and related genes were measured by using pre-designed primers and probes (Applied Biosystems; PGC-la, Mm00447181_m1, GAPDH, and Mm99999915_q1) for the synthesized cDNA and analysis targets (Fndc5, PGC-1α, Erra, UCP1, SOD2, and GPX1). The Rotor-Gene 3000 system (Corbett Research, Sydney, Australia) was used for PCR reaction and analysis, and the results are shown in FIG. 14.

FIG. 14 shows gene expression changes by the treatment with SL compositions in mouse models. In FIG. 14, the vertical axis represents the ratio of the abdominal fat gene expression in the models fed with a high-fat diet compared with SL administration, which show the numerical values of (the gene expression level of the 3'-SL administration group/the gene expression level of the control group) and (the gene expression level of the 6'-SL administration group/the gene expression level of the control group) in the abdominal fat. 6-Week-old male mice were divided into the control group and the SL administration groups (8 animals per group), which were subjected to a high-fat dietary control test for 10 weeks. As a result, as shown in FIG. 14, the 6'-SL administration group showed about two-time significant increases in expression levels of several analysis targets (Fndc5, PGC-la, Erra, UCP1, SOD2, and GPX1) compared with the negative control group. It could be seen that 3'-SL was relatively low compared with 6'-SL in view of the expression stimulation effects of PGC-1α and related genes.

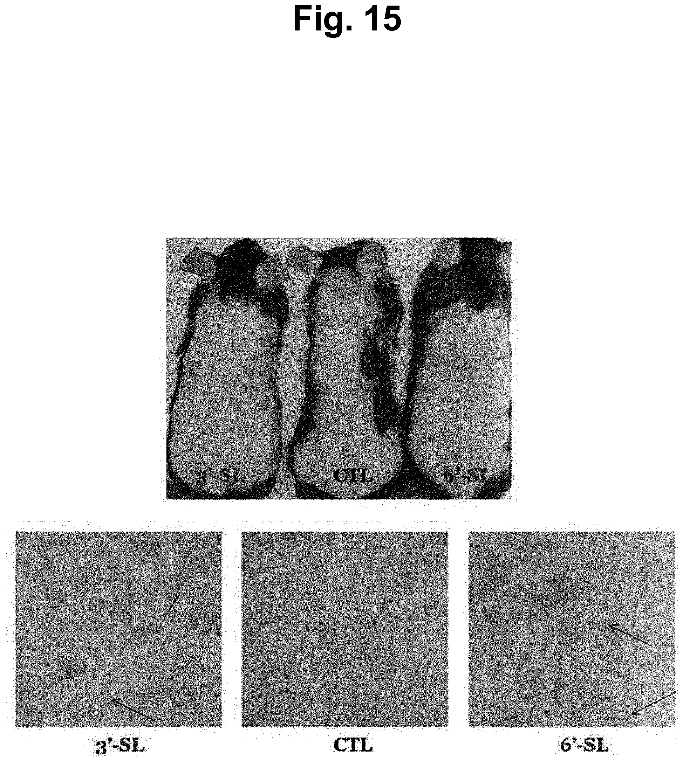

In addition, in the ob mouse (C57BL/6J-ob/ob) models fed with a high-fat diet without SL treatment, the topical fat removal effect by SL (3'-SL & 6'-SL) administration using a meso roller (0.5 mm, INTO MR, Intomedi Inc.) was investigated. Specifically, the hairs on the dorsal side of the rat were shaved, and 0.1 M SL in the saline buffer was applied to the skin, and was absorbed into the skin by rubbing with the meso roller. The control group was tested by the same method except that the buffer was used without SL. The results are shown in FIG. 15. FIG. 15 shows gene expression changes when SL (3'-SL & 6'-SL) compositions were topically administered in the mouse models. In FIG. 15, 3'-SL, 6'-SL, and CTL represent the 3'-SL administration group, the 6'-SL administration group, and and the control group, respectively. After feeding with a with a high-fat diet for 10 weeks, SL (3'-SL & 6'-SL) compositions were topically administered on day 0 and day 4 using the meso roller, and then dermoscopy observation was conducted on day 7. The observation results are shown.

As a result, as shown in FIG. 15, it could be confirmed that the topical fat was removed in the portion administered with 6'-SL using the meso roller compared with the negative control portion. It could be seen that 3'-SL was relatively low compared with 6'-SL in view of the topical fat removing effects.

### Example 11: Gene expression changes by body parts and aging-related chronic disease preventing effect through telomere functions and ROS control by the treatment with SL (3'-SL & 6'-SL) compositions in aging stimulation models

In order to investigate the gene expression changes by body parts by the treatment with SL (3'-SL & 6'-SL) compositions in aging stimulation models, 4-week-old male aging stimulation model mice (SAM P1/Sku Slc) were purchased from Central Lab Animal (Korea). Water was freely accessible, and a commercially available pellet feed (Dooyeul Biotech, Korea) was given for one week. 6-Week-old male aging stimulation model mice (initial body weight, average 28.8 ± 2.3g) were randomly grouped into three different dietary treatment groups (8 animals per group) below, and these diets were maintained for 12 weeks (a total of 24 animals):
- Control group: Normal mice fed with a normal diet without SL administration (8 animals)
- 3'-SL administration group: Aging stimulation models treated with 3'-sialyllactose (3'-SL, Sigma) (oral administration of 0.1 mg per kg of mouse weight per day) in addition to the normal diet group (8 animals)
- 6'-SL administration group: Aging stimulation models treated with 6'-sialyllactose (6'-SL, Sigma) (oral administration of 0.1 mg per kg of mouse weight per day) in addition to the normal diet group (8 animals)

Sialyllactose or DW was orally administered daily. The mice were kept in animal rooms for 14 weeks, fasted for 12 hours, and sacrificed. The dietary intake and body weight change were measured every 5 days. 3'-Sialyllactose (3'-N-Acetylneuraminyl-D-lactose, 3'-Sialyl-D-lactose, or α-NeuNAc-(2,3)-β-D-Gal-(1,4)-DGlc) or 6'-sialyllactose (6'-N-acetylneuraminyl-lactose, 6'-sialyl-D-lactose, or α-NeuNAc-(2→6)-β-D-Gal-(1→4)-D-Glc) was purchased from Sigma-Aldrich.

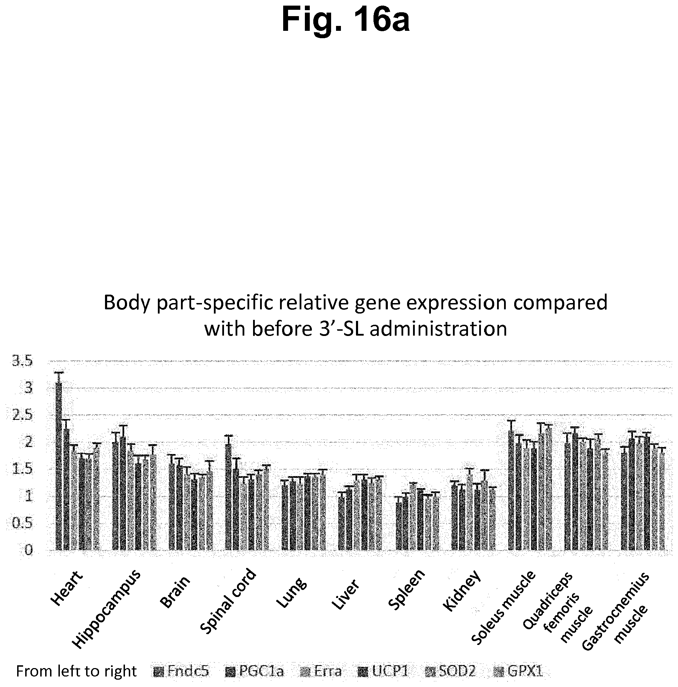

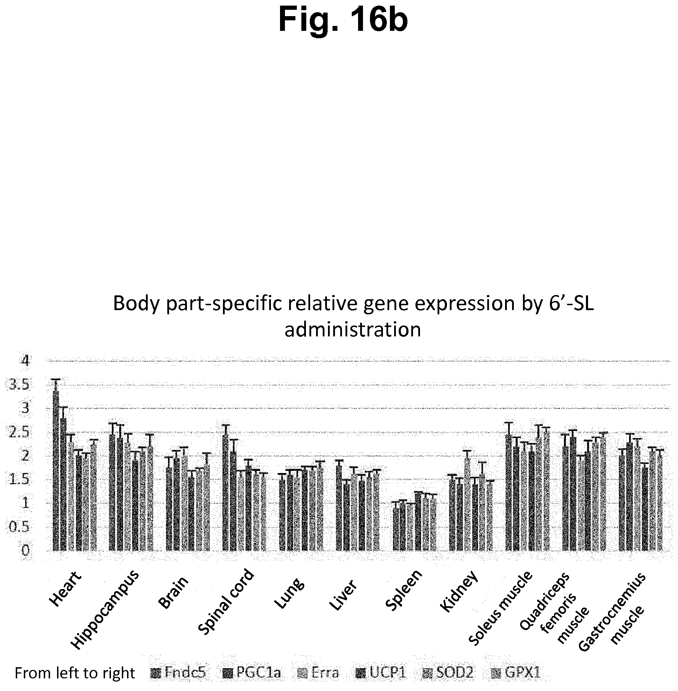

The gene expression changes by administration of the SL (3'-SL & 6'-SL) compositions were quantitatively compared for eight main organs (heart, hippocampus, brain, spinal cord, lung, liver, spleen, and kidney), three skeletal muscles (soleus muscle, quadriceps femoris muscle, and gastrocnemius muscle), and the like. RNA was extracted by TRIzol agent (Invitrogen). cDNA was synthesized by using RNA, which has been extracted as above and quantified, and a reverse transcription system (Promega, USA). The expression patterns of PGC-la and related genes were measured by using pre-designed primers and probes (Applied Biosystems; PGC-la, Mm00447181_m1, GAPDH, and Mm99999915_q1) for the synthesized cDNA and analysis targets (Fndc5, PGC-la, Erra, UCP1, SOD2, and GPX1). The Rotor-Gene 3000 system (Corbett Research, Sydney, Australia) was used for PCR reaction and analysis, and the results are shown in FIG. 16.

FIGS. 16a and 16b show the gene expression changes by the treatment with SL (3'-SL & 6'-SL) compositions in aging stimulating models. The 6-week-old male aging stimulation model mice (SAM P1/Sku Slc) were grouped into the control group, 3'-SL administration administration group, and 6'-SL administration group (8 animals per group), and then subjected to a dietary control test for 12 weeks. In FIGS. 16a and 16b, the vertical axes represent the relative gene expression changes by body parts compared with before SL (3'-SL & 6'-SL) administration, which show the numerical values of (the gene expression level of the 3'-SL administration group/the gene expression level of the control group) and (the gene expression level of the 6'-SL administration group/the gene expression level of the control group) in the respective body parts. As a result, as shown in FIGS. 16a and 16b, both of 3'-SL (FIG. 16a) and 6'-SL (FIG. 16b) showed significant increases in expression levels of several analysis targest (Fndc5, PGC-1α, Erra, UCP1, SOD2, and GPX1) in most of the organs and skeletal muscles, and 3'-SL was relatively low compared with 6'-SL in view of the gene expression stimulation effects of analysis targets.

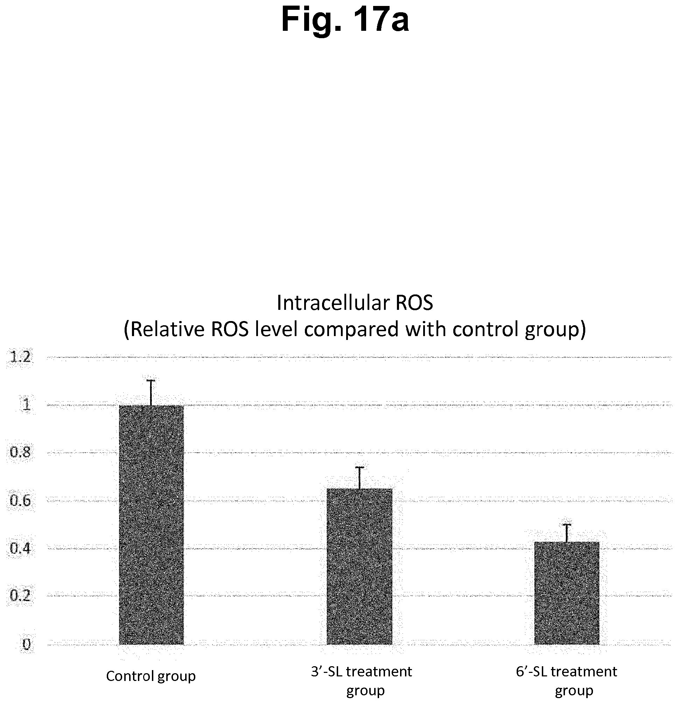

In addition, for cell-level tests, mouse aortic smooth muscle cells (MASMs) were isolated from the control group fed with a high-fat diet (Rodent Diet with 60 kcal% fat) without SL administration (Griendling et al., 1991), and the SL (3'-SL & 6'-SL) compositions were added to the culture liquid, followed by incubation. For intracellular H₂O₂ measurement (ROS values), the cells were cultured in 12-well culture plates immersed in 0.1% bovin calf serum. The intracellular ROS values were measured using H2DCFDA. For assay, the cells were cultured together with H2DCFDA in HBSS buffer for 30 minutes. The cells were trypsinized, washed, and lysed in HBSS. The fluorescence values were measured immediately by a CytoFluor plate reader (FIG. 17a).

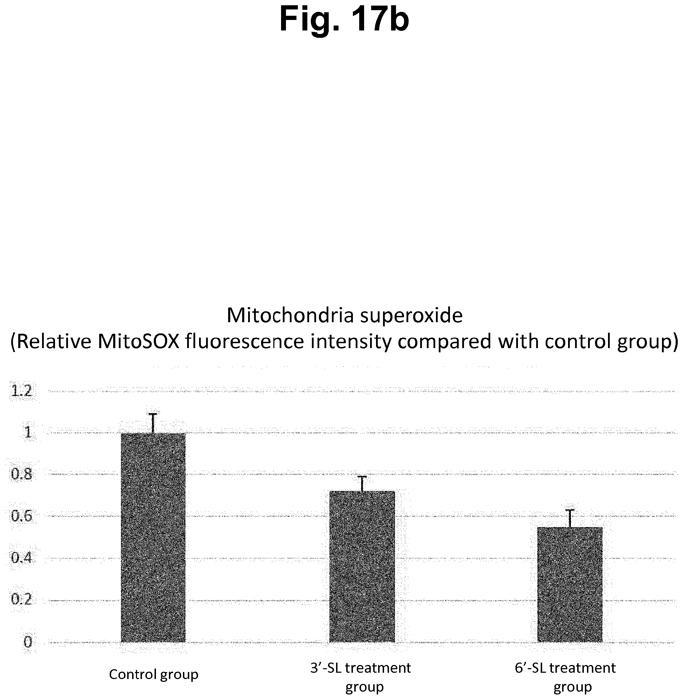

For the measurement of mitochondrial superoxide production, mitochondrial ROS was measured using MitoSOX Red (mitochondrial superoxide fluorescent marker). MASMs were extracted, and incubated with MitoSOX (4 µM) in the dark room at 37°C for 20 minutes. The MitoSOX fluorescence was quanti-fied by the cell fluorescence intensity read using a fluorescent plate reader (480 nm excitation / 580 nm emission) (Figure 17b) .

In FIGS. 17a and 17b, MASMs were extracted from the aging stimulatin model mouse control group, and the SL (3'-SL & 6'-SL) compositions were added to the culture liquid to conduct comparision. For intracellular H2O2 (ROS values) measurement, the cells were cultured together H2DCFDA (FIG. 17a), and for mitochondrial ROS measurement, the cells were cultured together with MitoSOX Red (mitochondrial superoxide fluorescent marker (FIG. 17a). The intracellular H₂O₂ and mitochondrial superoxide production were quantified by a fluorescence plate reader. It could be seen that the compositions dropped the ROS values and inhibited the mitochondrial superoxide production.

FIGS. 17a and 17b show the measurement results of intercellular H₂O₂ (ROS values) (FIG. 17a) and the measurement results of mitochondrial superoxide production (FIG. 17b) . MASMs were extracted from the aging stimulation model mice (SAM P1/Sku Slc), and the SL (3'-SL & 6'-SL) compositions were added to the culture liquid to conduct comparision For intracellular H2O2 measurement, the cells were cultured together H2DCFDA (FIG. 17a), and for mitochondrial ROS measurement, the cells were cultured together with MitoSOX Red (mitochondrial superoxide fluorescent marker (FIG. 17a). The intracellular H₂O₂ and mitochondrial superoxide production were quanti-fied by a fluorescence plate reader. It could be seen that the SL (3'-SL & 6'-SL) compositions dropped the ROS values and inhibited the mitochondrial superoxide production.

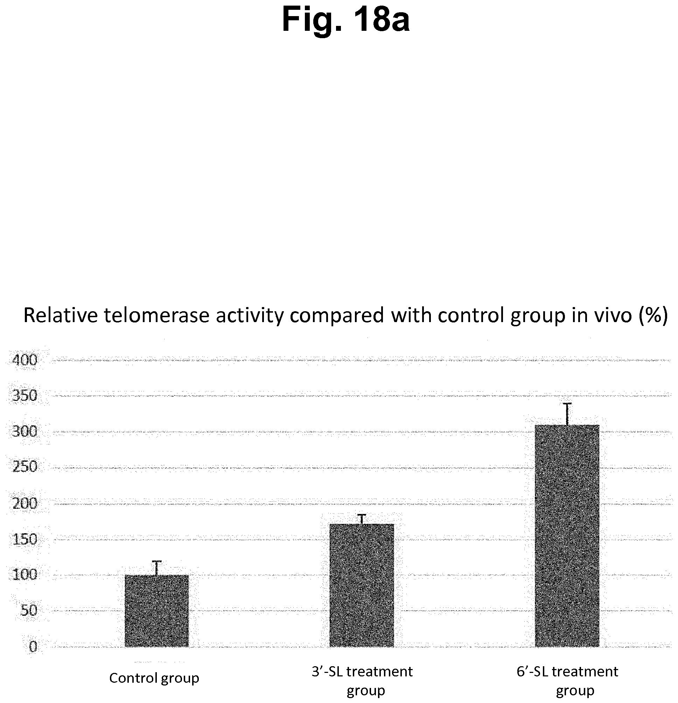

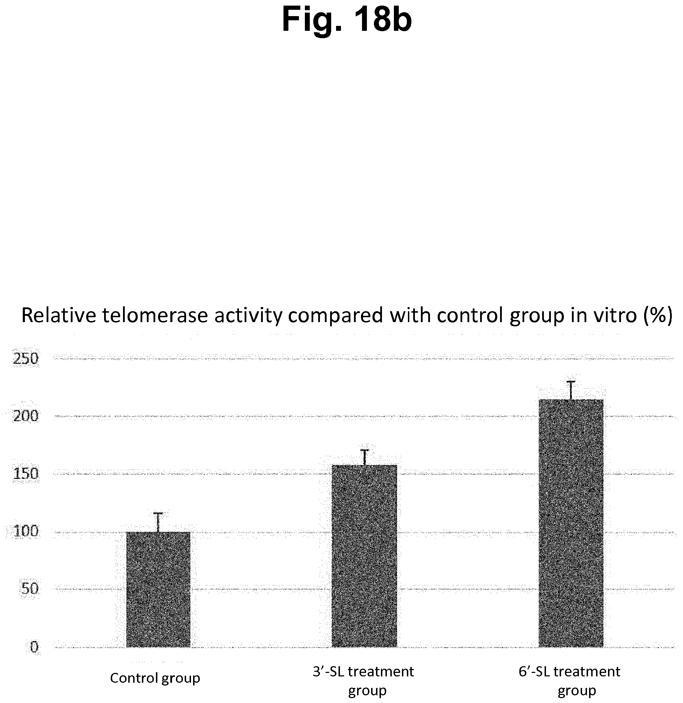

In FIGS. 18a and 18b, the telomerase activity was measured using TRAP protocol (Wright et al., 1995). In summary, cell pellets were lysed in CHAPS lysis solvent (containing ribnuclease inhibitor), followed by incubation at 4°C for 30 minutes. Cell extracts (1 mg) were TRAPeze reaction mix (telomerase substrate (TS) primer, fluorescent-labeled RP (reverse) primer, control standard template, and sulforhodamine-labeled standard K2 primer). The TS primer was elongated at 30°C for 30 minutes, and then PCR was performed. The fluorescence of the thus obtained TRAP product was measured using a fluorescent plate reader to quanti-fy telomerase activity. Relative telomerase activity was normalized by the ratio of native fluorouracil to sulforhodamine (internal control standard), expressed as a percentage. Fluorescein (M0250) was purchased from Marker Gene Technologies, Inc. H2-dichlorofluorescin diacetate (DCFDA) was purchased from Molecular Probes. MitoSOXTM Red, MitoTracker Green FM, and MitoTracker^{®} Orange CMTMRos (MTO) were purchased from Invitrogen. 10,000x SYBR^{®} Gold dye was purchased from Molecular Probes, Inc.. Nuclear/Cytosol fractionation kit (K266-100) was purchased from BioVision. TRAPeze^{®} XL telomerase detection kit (S7707) was purchased from MILLIPORE. Telomere PNA FISH Kit/Cy3 (K5326) was purchased from Dako.

FIGS. 18a and 18b show the measurement results of telomerase activity by the administration of SL (3'-SL & 6'-SL) compositions. Through these values, TERT and antioxidant/electrophile-responsive element (ARE/ERE) nerve pathway activation could be deduced (Xiong et al., 2015, Cell Reports 12, 1391-1399). The aging stimulation models (FIG. 18a) and MASMs (FIG. 18b) were treated with SL (3'-SL & 6'-SL) to analyze telomerase activity. FIG. 18a shows the data of in vivo analysis of telomerase activity in the aorta sample extracted from the control group, 3'-SL administration group, and 6'-SL administration group. FIG. 18b shows the data of in vitro analysis of temomerase activity when the smooth muscle cells of the control group were isolated (Griendling et al., 1991) and MASMs were cultured for 1 day in 12-well culture plates treated with SL (3'-SL & 6'-SL). The test results showed that the administration of the SL (3'-SL & 6'-SL) compositions increased telomerase activity. These results suggest that the SL (3'-SL & 6'-SL) compositions may be associated with TERT dysfunction and DNA damage recovery (increased telomerase activity -TERT expression) through increased PGC-la.

### Example 12: Aging-related chronic disease preventing effect through telomere functions and ROS control by the treatment with SL (3'-SL & 6'-SL) compositions in arteriosclerosis models

It has been recently reported that the removal of PGC-la gene results in vascular aging, atherosclerosis, telomere dysfunction and length reduction, DNA damage, decreased expression and activity of telomerase reverse transcriptase (TERT), and increased p53 (Xiong et al., 2015, Cell Reports 12, 1391-1399).

In general, ApoE^{-/-} mice increase the sensitivity to oxidative stress and inflammation and rapidly develop atherosclerosis wounds observed in persons, and thus the mice were used as representative models of arteriosclerosis (Weiss et al., 2001). Therefore, ApoE^{-/-} mice (C57BL/6 based) were purchased from Jackson Laboratory. The genotype was identified by PCR using tail DNA.

In order to investigate the aging-related chronic disease preventing effect through the control of telomerase functions and DNA damage by the treatment with SL (3'-SL & 6'-SL) compositions in arteriosclerosis models, two types of 24-week-old male model mice (PGC-1α^{+/+}ApoE^{-/-}) were randomly grouped into three different dietary treatment groups (8 animals per group) below, and the diets were maintained for 6 weeks (a total of 24 animals):
- Control group: Arteriosclerosis models fed with a high-fat diet (Rodent Diet with 60 kcal% fat) (8 animals)
- 3'-SL administration group: Arteriosclerosis models treated with 3'-sialyllactose (3'-SL, Sigma) (oral administration of 0.1 mg per kg of mouse weight per day) in addition to the high-fat diet group (8 animals)
- 6'-SL administration group: Arteriosclerosis models treated with 6'-sialyllactose (6'-SL, Sigma) (oral administration of 0.1 mg per kg of mouse weight per day) in addition to the high-fat diet group (8 animals)

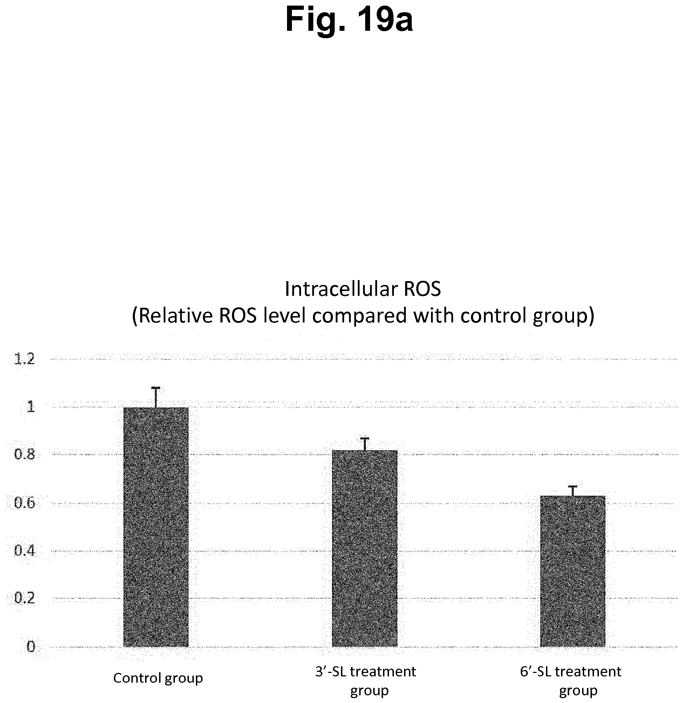

For cell-level tests, mouse aortic smooth muscle cells (MASMs) were isolated from the control group fed with a high-fat diet (Rodent Diet with 60 kcal% fat) without SL administration (Griendling et al., 1991), and the SL (3'-SL & 6'-SL) compositions were added to the culture liquid, followed by comparison. For intracellular H₂O₂ measurement (ROS values), the cells were cultured in 12-well culture plates immersed in 0.1% bovine calf serum. The intracellular ROS values were measured using H2DCFDA. For assay, the cells were cultured together with H2DCFDA in HBSS buffer for 30 minutes. The cells were trypsinized, washed, and lysed in HBSS. The fluorescence values were measured immediately by a CytoFluor plate reader (FIG. 19a).

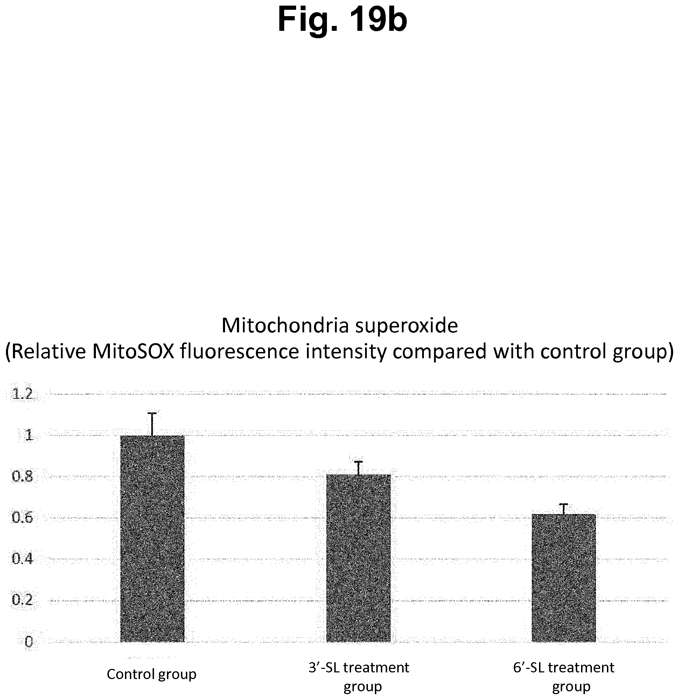

For the measurement of mitochondrial superoxide production, mitochondrial ROS was measured using MitoSOX Red (mitochondrial superoxide fluorescent marker). MASMs were extracted, and incubated with MitoSOX (4 µM) in the dark room at 37°C for 20 minutes. The MitoSOX fluorescence was quantified by the cell fluorescence intensity read using a fluorescent plate reader (480 nm excitation / 580 nm emission) (Figure 19b).

In FIGS. 19a and 19b, MASMs were extracted from the model mouse (PGC-1α^{+/+}ApoE^{-/-}) control group, and the SL (3'-SL & 6'-SL) compositions were added to the culture liquid to conduct comparison. For intracellular H2O2 (ROS values) measurement, the cells were cultured together H2DCFDA (FIG. 19a), and for mitochondrial ROS measurement, the cells were cultured together with MitoSOX Red (mitochondrial superoxide fluorescent marker (FIG. 19a). The intracellular H₂O₂ and mitochondrial superoxide production were quantified by a fluorescence plate reader. It could be seen that the compositions dropped the ROS values and inhibited the mitochondrial superoxide production.

FIGS. 19a and 19b show the measurement results of intercellular H₂O₂ (ROS values) (FIG. 19a) and the measurement results of mitochondrial superoxide production (FIG. 19b). MASMs were extracted from the model mice (PGC-1α^{+/+}ApoE^{-/-}), and the SL (3'-SL & 6'-SL) compositions were added to the culture liquid to conduct comparison. For intracellular H2O2 measurement, the cells were cultured together H2DCFDA (FIG. 19a), and for mitochondrial ROS measurement, the cells were cultured together with MitoSOX Red (mitochondrial superoxide fluorescent marker (FIG. 19a). The intracellular H₂O₂ and mitochondrial superoxide production were quantified by a fluorescence plate reader. It could be seen that the SL (3'-SL & 6'-SL) compositions dropped the ROS values and inhibited the mitochondrial superoxide production.

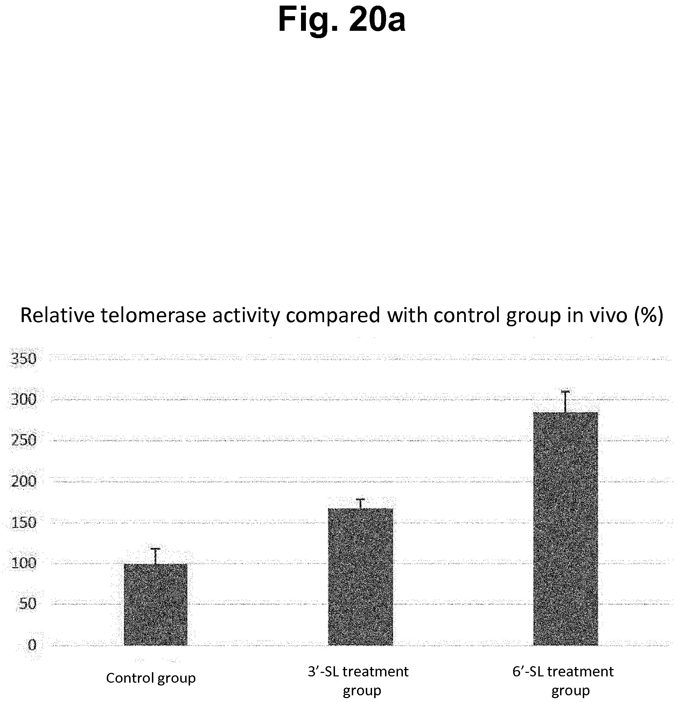

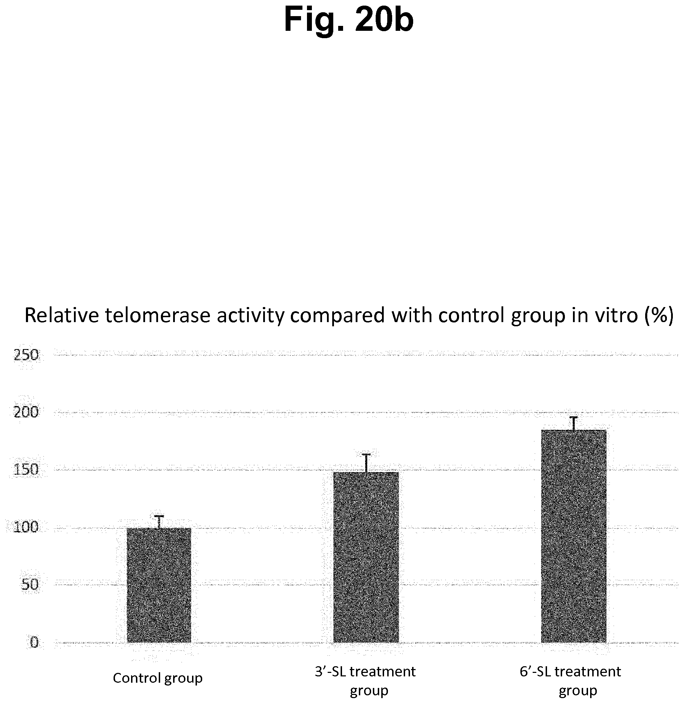

In FIG. 20, the telomerase activity was measured using TRAP protocol (Wright et al., 1995). In summary, cell pellets were lysed in CHAPS lysis solvent (containing ribonuclease inhibitor), followed by incubation at 4°C for 30 minutes. Cell extracts (1 mg) were TRAPeze reaction mix (telomerase substrate (TS) primer, fluorescent-labeled RP (reverse) primer, control standard template, and sulforhodamine-labeled standard K2 primer). The TS primer was elongated at 30°C for 30 minutes, and then PCR was performed. The fluorescence of the thus obtained TRAP product was measured using a fluorescent plate reader to quantify telomerase activity. Relative telomerase activity was normalized by the ratio of native fluorouracil to sulforhodamine (internal control standard), expressed as a percentage. Fluorescein (M0250) was purchased from Marker Gene Technologies, Inc. H2-dichlorofluorescin diacetate (DCFDA) was purchased from Molecular Probes. MitoSOXTM Red, MitoTracker Green FM, and MitoTracker^{®} Orange CMTMRos (MTO) were purchased from Invitrogen. 10,000x SYBR^{®} Gold dye was purchased from Molecular Probes, Inc. Nuclear/Cytosol fractionation kit (K266-100) was purchased from BioVision. TRAPeze^{®} XL telomerase detection kit (S7707) was purchased from MILLIPORE. Telomere PNA FISH Kit/Cy3 (K5326) was purchased from Dako.

FIGS. 20a and 20b show the measurement results of telomerase activity by the administration of SL (3'-SL & 6'-SL) compositions. Through these values, TERT and antioxidant/electrophile-responsive element (ARE/ERE) nerve pathway activation could be deduced (Xiong et al., 2015, Cell Reports 12, 1391-1399).

FIGS. 20a and 20b show the analysis results of telomerase activity by the treatment of arteriosclerosis models (ApoE^{-/-}; FIG. 20a) and MASMs (FIG. 20b) with SL (3'-SL & 6'-SL). FIG. 20a shows the data of in vivo analysis of telomerase activity in the aorta sample extracted from the control group, 3'-SL administration group, and 6'-SL administration group. FIG. 20b shows the data of in vitro analysis of telomerase activity when the smooth muscle cells of the control group were isolated (Griendling et al., 1991) and MASMs were cultured for 1 day in 12-well culture plates treated with SL (3'-SL & 6'-SL). The test results showed that the administration of the SL (3'-SL & 6'-SL) compositions increased telomerase activity. These results suggest that the SL (3'-SL & 6'-SL) compositions may be associated with TERT dysfunction and DNA damage recovery (increased telomerase activity -TERT expression) through increased PGC-1α.

### Example 13: Gene expression changes by body parts by the treatment with SL (3'-SL & 6'-SL) compositions in skin test

In order to investigate the gene expression chages by body parts by the treatment with the SL (3'-SL & 6'-SL) compositions in skin test models, 6-week-old male skin test model (HRM2) mice (hairless appearance containing melanin) were randomly grouped into three different dietary treatment groups (8 animals per group, a total of 24 animals) below, and the diets (AIN-76A, Dyets, USA) were maintained for 10 weeks. The UV irradiation (black spot, wrinkle test), skin sensitivity test, skin irritation test, subcutaneous absorption test, or the like were conducted using a portable color-difference meter (CR-10, Minolta, Japan):
- Control group: Group fed with a normal diet (8 animals)
   -- 3'-SL administration group: Models treated with 3'-sialyllactose (3'-SL, Sigma) (oral administration of 0.1 mg per kg of mouse weight per day) in addition to the normal diet group (8 animals)
   -- 6'-SL administration group: Models treated with 6'-sialyllactose (6'-SL, Sigma) (oral administration of 0.1 mg per kg of mouse weight per day) in addition to the normal diet group (8 animals)

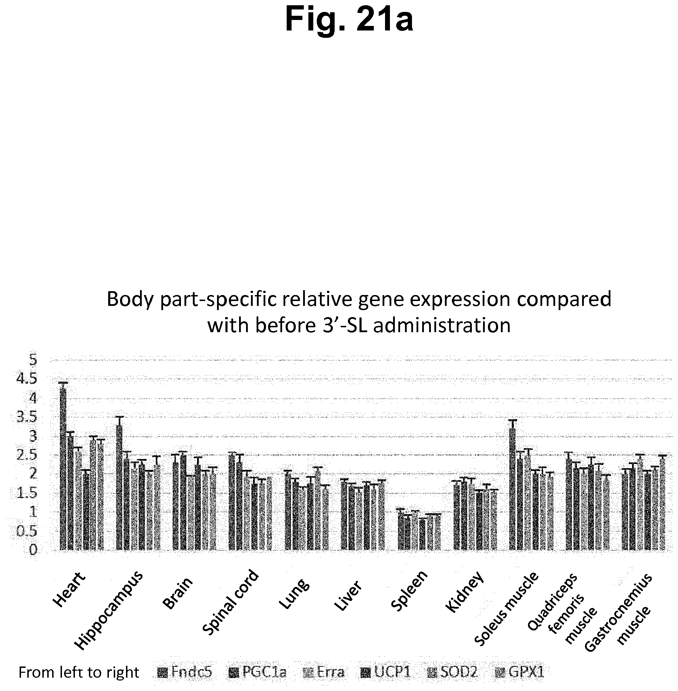

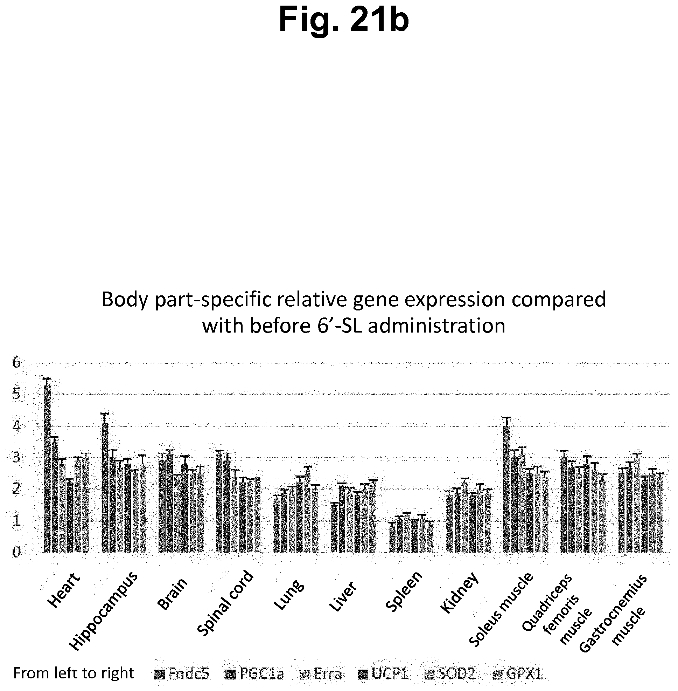

It could be seen that the SL (3'-SL & 6'-SL) administration groups showed much improved skin compared with the control group in view of the UV irradiation (black spot, wrinkle test), skin sensitivity test, skin irritation test, subcutaneous absorption test, or the like. The gene expression changes by administration of the SL (3'-SL & 6'-SL) compositions were quantitatively compared for eight main organs (heart, hippocampus, brain, spinal cord, lung, liver, spleen, and kidney), three skeletal muscles (soleus muscle, quadriceps femoris muscle, and gastrocnemius muscle), and the like. RNA was extracted by TRIzol agent (Invitrogen). cDNA was synthesized by using RNA, which has been extracted as above and quantified, and a reverse transcription system (Promega, USA). The expression patterns of PGC-1α and related genes were measured by using pre-designed primers and probes (Applied Biosystems; PGC-1α, Mm00447181_m1, GAPDH, and Mm99999915_q1) for the synthesized cDNA and analysis targets (Fndc5, PGC-1α, Erra, UCP1, SOD2, and GPX1). The Rotor-Gene 3000 system (Corbett Research, Sydney, Australia) was used for PCR reaction and analysis, and the results are shown in FIGS. 21a and 21b.

In FIGS. 21a and 21b, the relative gene expression changes by body parts compared with before SL (3'-SL & 6'-SL) administration were numerically expressed by (the gene expression level of the 3'-SL administration group/the gene expression level of the control group) and (the gene expression level of the 6'-SL administration group/the gene expression level of the control group) in the respective body parts. The expression levels of several analysis targets (Fndc5, PGC-1α, Erra, UCP1, SOD2, and GPX1) including PGC-1α in several body parts were very in the 6'-SL administration group compared with the control group. That is, it could be confirmed that 6'-SL stimulated the expression of PGC-1α and related genes in several organs and mucles of normal mice. It could be seen that 3'-SL was relatively low compared with 6'-SL in view of the gene expression stimulation effects of the analysis targets.

FIGS. 21a and 21b show the gene expression changes by the treatment with SL (3'-SL & 6'-SL) compositions in skin test models. 6-Week-old male skin test model (HRM2) mice were randomly grouped into the control group and the SL (3'-SL & 6'-SL) administration groups (8 animals per group), and subjected to a dieatery control test for 10 weeks. The relative gene expression changes compared with before SL (3'-SL & 6'-SL) administration were numerically expressed by (the gene expression level of the 3'-SL administration group/the gene expression level of the control group) and (the gene expression level of the 6'-SL administration group/the gene expression level of the control group), which are relative ratios of the values obtained by quanti-fying "the gene expression level of the SL administration group" and "the gene expression level of the control group gene" in each body part. The significant increases in expression levels of severa analysis targets (Fndc5, PGC-1α, Erra, UCP1, SOD2, and GPX1) were observed in most of organs and skeletal muscles. It could be seen that 3'-SL (FIG. 21a) was relatively low compared with 6'-SL (FIG. 21b) in view of the gene expression stimulation effects of analysis targets.

### Example 14: Effects of siallylactose (3'-SL & 6'-SL) composition on differentiated adipocytes

### (1) 3T3-L1 cell culture and differentiation

3T3-L1 adipocytes were purchased from Korean Cell Line Bank. For the culture and maintenance of 3T3-L1 adipocytes, the cells were subcultured in Dulbecco's modified Eagle's medium (DMEM, Welgene, Korea) supplemented with 10% bovin calf serum (FCS, Welgene, Korea) in a 5% CO₂ incubator at 37°C 3T3-L1 adipocytes were divided into six groups as follows: NT; Normal differentiated cell group (control group), sialyllactose test group; 1, 10, 100, 1000, and 10000 µM test groups treated with sialyllactose (SL, Sigma-Aldrich, USA). For cell differentiation, the cells were dispensed in 6-well plates at a density of 2 × 10⁵ cells per well, and grown to 100% confluency. After 2 days, the test groups were treated with DMEM medium containing 10% fetal bovine serum (FBS, Welgene, Korea), MDI solution (0.5 mM isobutylmethylxanthine (IBMX, Sigma-Aldrich, USA), 1 µM dexamethasone (Sigma-Aldrich, USA), and 1 ug/mL insulin (Sigma-Aldrich, USA)), and again treated with DMEM containing 10% FBS and 1 ug/mL insulin. Thereafter, the cells were differentiated into adipocytes while the medium was exchanged with DMEM supplemented with 10% FBS every two days. At the end of differentiation, DMEM medium supplemented with 10% FBS was treated with 3'-sialyllactose or 6'-sialyllactose at 0, 0.01, 0.1, 1, and 10mM for 10 days.

### (2) Oil-Red O staining

After differentiation in 6-well plates, 3T3-L1 adipocytes treated with '-sialyllactose or 6'-sialyllactose were washed two times with PBS, and then 2 ml of 10% formalin (Sigma-Aldrich, USA) was added thereto to fix the adipocytes at room temperature for 10 minutes. After the fixed cells were dried, the cells were treated with 1 ml of Oil Red O stain reagent (Sigma-Aldrich, USA) for 20 minutes, and then sufficiently washed four times with distilled water to remove the Oil Red O stain reagent. Thereafter, 1 ml of 100% isopropanol (Sigma- Aldrich, USA) was added to effuse stained adipocytes, and then the amount of accumulated fat was measured using absorbance at 500 nm.

### (3) Free glycerol measurement

The medium, obtained by treating 3T3-L1 adipocytes differentiated in 6-well plates with 6'-siallylactose at concentrations of 0, 0.01, 0.1, 1, and 10 mM, and culturing the adipocytes for 10 days, was sampled in an eppendorf tube, followed by free glycerol analysis using a glycerol cell-based assay kit (cayman, 10011725, USA). 100 uL of free glycerol reagent was added to 25ul of the medium, followed by incubation at room temperature for 15 minutes, and then the absorbance was measured at 540nm.

### (4) Cell viability measurement

After differentiation, 3T3-L1 adipocytes treated with 6'-siallylactose were measured for cell viability using a cell counting kit-8 (Dojindo Molecular Technologies, Inc. USA). After drug treatment, 10 uL of CCK-8 reagent was added, followed by incubation for 2 hours, and the absorbance was measured at 450 nm.

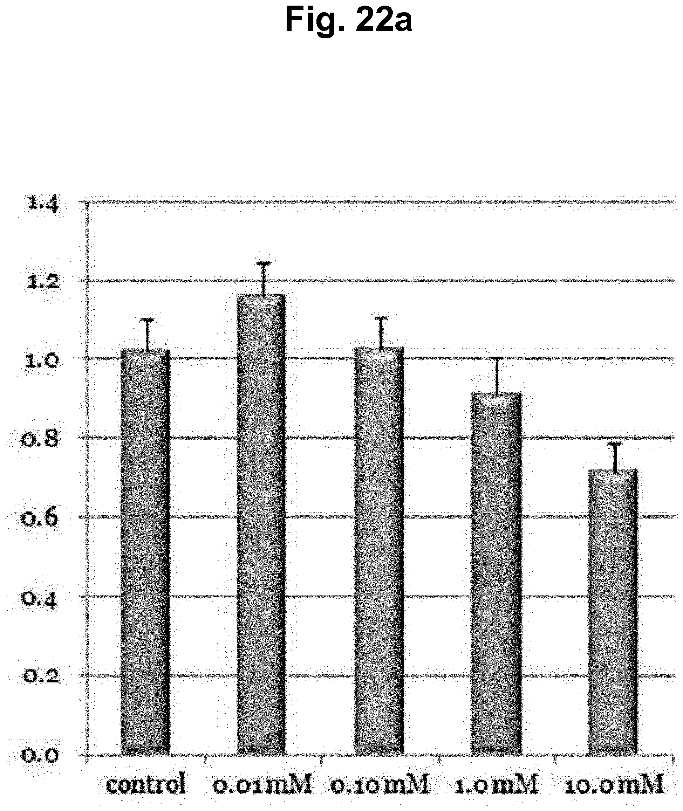

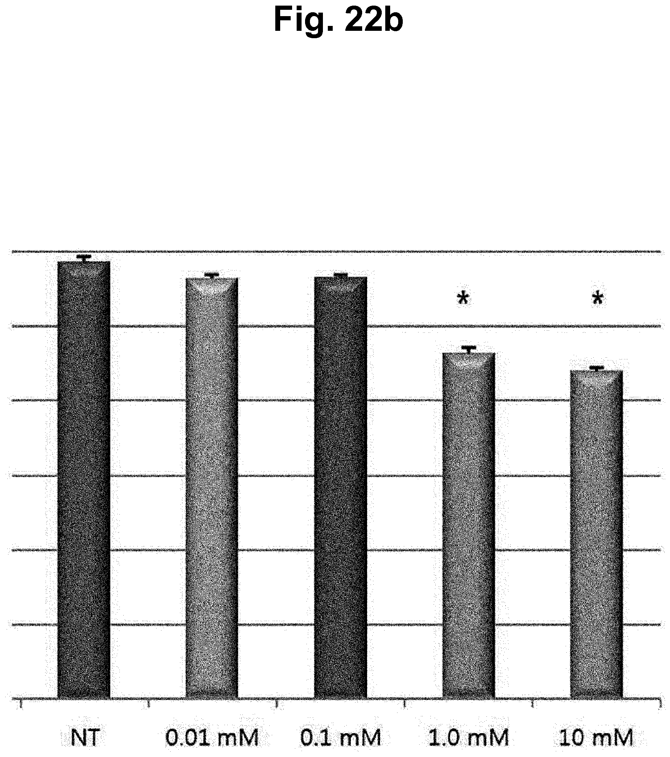

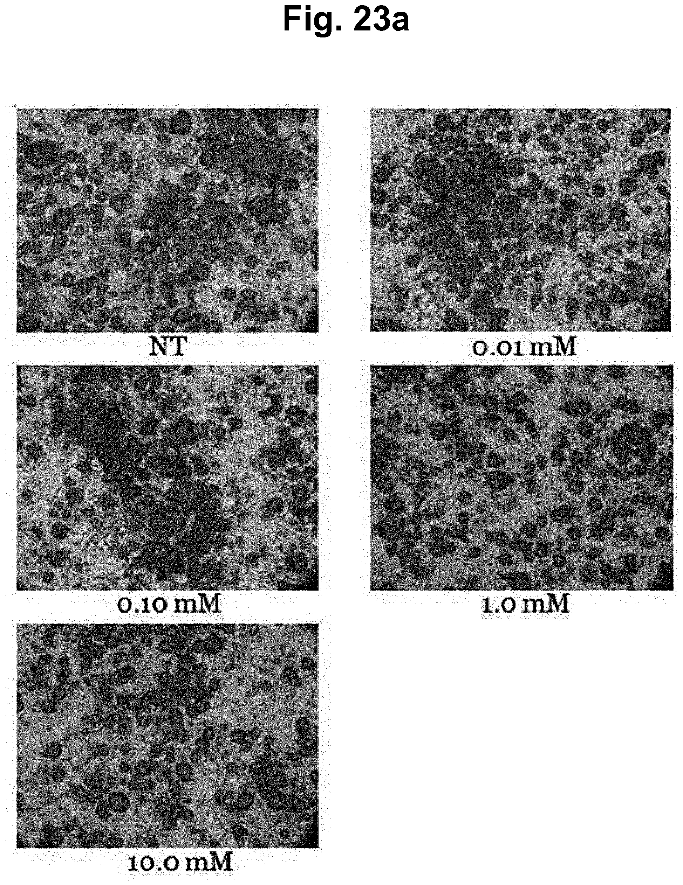

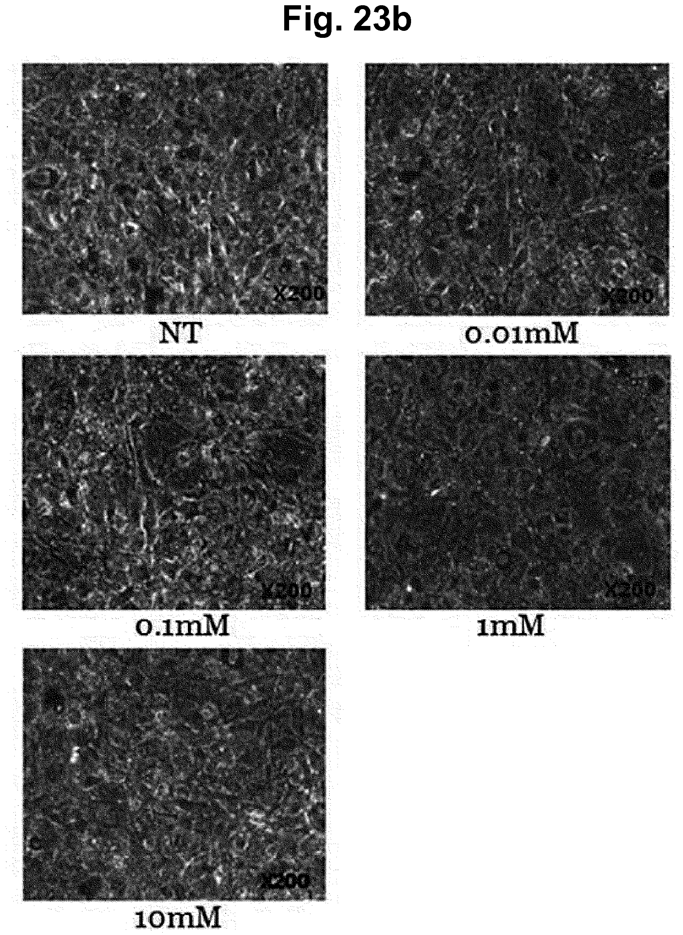

As can be confirmed from FIGS. 22 and 23, the intracellular fat was reduced by the addition of siallylactode in the differentiated adipocytes, and especially, the intracellular fat was greatly reduced in the test groups with 6'-siallylactose added.

FIGS. 22a and 22b show the intercellular fat changes when 6'-siallactose (FIG. 22a) and 3'-siallactose (FIG. 22b) were administered into differentiated adipocytes. NT represents a control group, and 0.01, 0.1, 1, and 10 mM represent test groups with 0.01, 0.1, 1, 10 mM 6'-siallylactose (FIG. 22a) and 3'-siallylactose (FIG. 22b), respectively.

FIGS. 23a and 23b are optical microscopic images of cells (Oil red O test) showing intercellular fat changes when 6'-siallactose (FIG. 23a) and 3'-siallactose siallactose (FIG. 23b) were administered into differentiated differentiated adipocytes. In the drawings, NT represents a control group, and 0.01, 0.1, 1, and 10 mM represent test groups treated with 0.01, 0.1, 1, 10 mM 6'-siallylactose (FIG. 23a) and 3'-siallylactose (FIG. 23b), respectively.

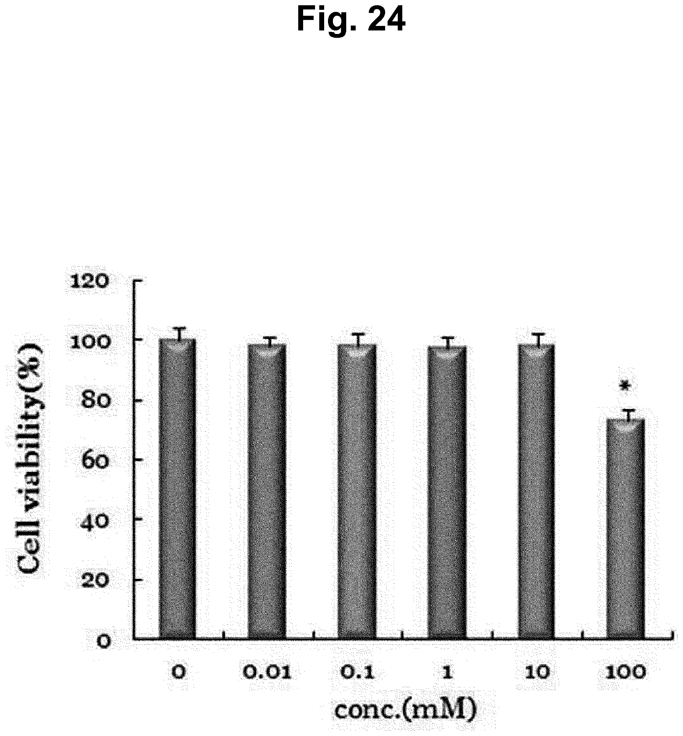

As can be confirmed from FIG. 24, siallylactoses did not affect cell viability up to 10 mM in differentiated adipocytes.

FIG. 24 shows the effect of 6'-sialyllactose on cell viability in differentiated adipocytes. NT represents a control group, and 0.01, 0.1, 1, 10, and 100 mM represent test groups treated with 0.01, 0.1, 1, 10, and 100 mM 6'-siallylactose, respectively.

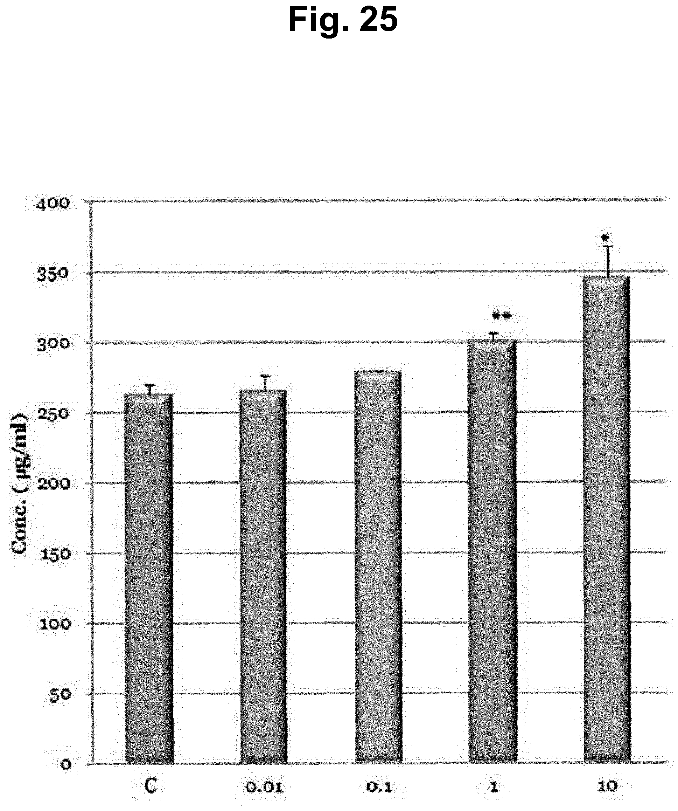

In additioin, as can be confirmed from FIG. 25, siallylactoses reduced intracellular fat by increasing the glycerol secretion of adipocytes in a dose-dependent manner.

Sialyllactoses stimulated the glycerol secretion regardless of cell viability in differentiated adipocytes, and especially, 6'-sialyllactose significantly reduced intracellular fat.

FIG. 25 shows glycerol secretion changes by sialyllactose in differentiated adipocytes In the drawings, NT represents a control group, and 0.01, 0.1, 1, and 10 mM represent test groups with 0.01, 0.1, 1, 10 mM 3'-siallylactose, respectively.

### Example 15: Subcutaneous fat changes of high-fat diet mice by subcutaneous injection of siallylactose (3'-SL & 6'-SL) compositions

### (1) High-fact diet mice

4-Week-old C56BL/6 mice were purchased from Dooyeul Biotech (Korea). Water was freely accessible, and a commercially available pellet feed (Dooyeul Biotech, Korea) was given for one week. A high-fact (60% fat) diet purchased from Research Diets (New Brunswick, U.S.A) was supplied for 28 days to construct fat-accumulated mice.

### (2) Siallylactose subcutaneous injection

0.5 ml of 100 mM 3'-siallylactose or 6'-siallylactose dissolved in a phosphate buffer was subcutaneously injected two times (day 0 and day 4) into four to five sites of the dorsal region of the mouse having fat accumulation induced by a high-fat diet. On day 4 and 7, the skin was observed by the naked eye and dermoscopy. A phosphate buffer was used as a control group (CTL).

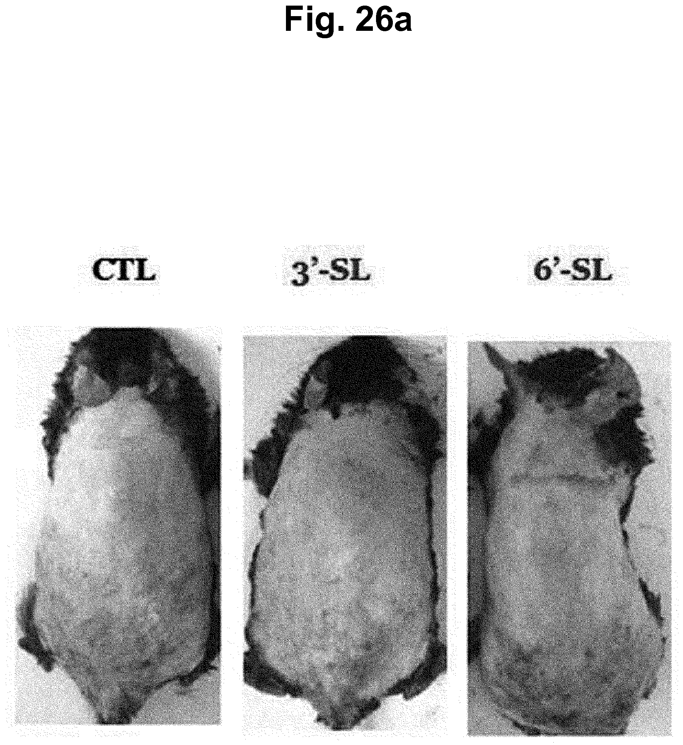

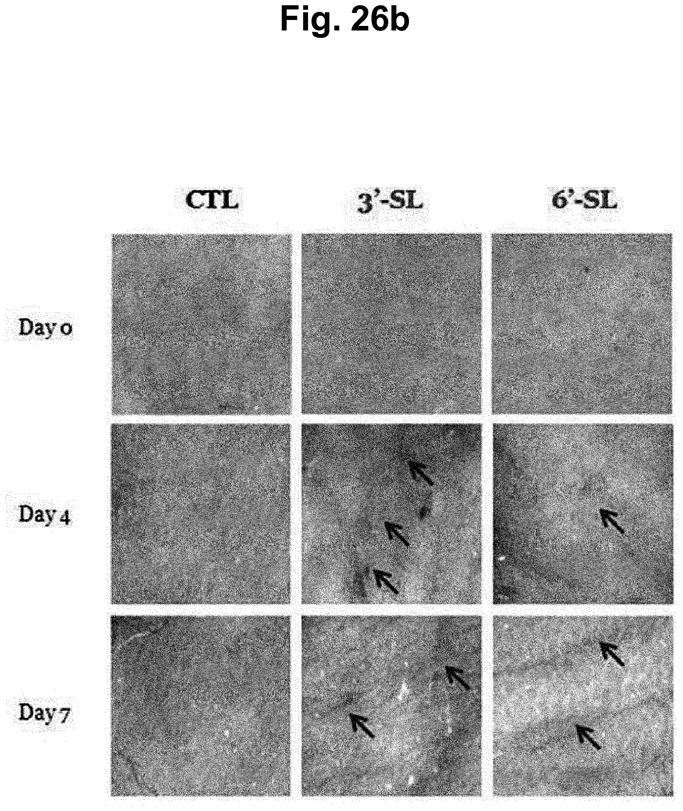

As can be confirmed from FIGS. 26a and 26b, the subcutaneous injection of siallylactoses reduced the subcutaneous fat in igh-fat diet mice, thereby inducing the wrinkles on the skin surface. Especially, 6'-siallylactose greatly reduced subcutaneous fat.

FIGS. 26a and 26b show skin changes of high-fat diet mice by subcontenous injection of sialyllactoses. The skin changes were confirmed by the naked eye and dermoscopy (FIGS. 26a and 26b). CTL represents a control group, and 3'-sillylactose (3'-SL) and 6'-sillylactose (6'-SL) represent test groups.

### REFERENCES

1. Galluzzi, L., Maiuri, M.C., Vitale, I., Zischka, H., Castedo, M., Zitvogel, L., Kroemer, G., 2007. Cell death modalities: classification and pathophysiological implications. Cell Death Differ. 14, 1237-1243.
2. Chipuk, J.E., Moldoveanu, T., Llambi, F., Parsons, M.J., Green, D.R., 2010. The BCL-2 family reunion. Mol. Cell 37, 299- 310.
3. Youle, R.J., Strasser, A., 2008. The BCL-2 protein family: opposing activities that mediate cell death. Nat. Rev. Mol. Cell Biol. 9, 47-59.
4. Fadeel, B., Orrenius, S., 2005. Apoptosis: a basic biological phenomenon with wide ranging implications in human disease. J. Intern. Med. 258, 479-517.
5. Aleck W.E. Jones, Zhi Yao, Jose Miguel Vicencio, Agnieszka Karkucinska-Wieckowska, Gyorgy Szabadkai, 2012. PGC-1 family coactivators and cell fate: Roles in cancer, neurodegeneration, cardiovascular disease and retrograde mitochondria-nucleus signaling. Mitochondrion. 12, 86-99.
6. Lin, J. et al. Spiegelman, B.M., 2004. Defects in adaptive energy metabolism with CNS-linked hyperactivity in PGC-1alpha null mice. Cell 119, 121-135.
7. Leone, T.C., Lehman, J.J., Finck, B.N., Schaeffer, P.J., Wende, A.R., Boudina, S., Courtois, M., Wozniak, D.F., Sambandam, N., Bernal-Mizrachi, C., Chen, Z., Holloszy, J.O., Medeiros, D.M., Schmidt, R.E., Saffitz, J.E., Abel, E.D., Semenkovich, C.F., Kelly, D.P., 2005. PGC-1alpha deficiency causes multi-system energy metabolic derangements: muscle dysfunction, abnormal weight control and hepatic steatosis. PLoS Biol. 3, e101.
8. Chaturvedi RK & Flint Beal M (2013) Mitochondrial diseases of the brain. Free Radic Biol Med 63, 1-29.
9. Katsouri L, Parr C, Bogdanovic N, Willem M & Sastre M (2011) PPARgamma co-activator-lalpha (PGC-1alpha) reduces amyloid-beta generation through a PPARgamma-dependent mechanism. J Alzheimers Dis 25, 151-162.
10. Qin W, Haroutunian V, Katsel P, Cardozo CP, Ho L, Buxbaum JD & Pasinetti GM (2009) PGC-1alpha expression decreases in the Alzheimer disease brain as a function of dementia. Arch Neurol 66, 352-361.
11. Wang R, Li JJ, Diao S, Kwak YD, Liu L, Zhi L, Bueler H, Bhat NR, Williams RW, Park EA et al. (2013) Metabolic stress modulates Alzheimer's beta secretase gene transcription via SIRT1- PPARgamma-PGC-1 in neurons. Cell Metab 17, 685-694.
12. Clark, J., Reddy, S., Zheng, K., Betensky, R., Simon, D., 2011. Association of PGC-1alpha polymorphisms with age of onset and risk of Parkinson's disease. BMC Med. Genet. 12, 69.
13. Weydt, P., Soyal, S., Gellera, C., DiDonato, S., Weidinger, C., Oberkofler, H., Landwehrmeyer, G.B., Patsch, W., 2009. The gene coding for PGC-1alpha modifies age at onset in Huntington's Disease. Mol. Neurodegener. 4, 3.
14. Cui, L., Jeong, H., Borovecki, F., Parkhurst, C.N., Tanese, N., Krainc, D., 2006. Transcriptional repression of PGC-1alpha by mutant huntingtin leads to mitochondrial dysfunction and neurodegeneration. Cell 127, 59-69.
15. Qin W, Haroutunian V, Katsel P, Cardozo CP, Ho L, Buxbaum JD & Pasinetti GM (2009) PGC-1alpha expression decreases in the Alzheimer disease brain as a function of dementia. Arch Neurol 66, 352-361.
16. Ranganathan, S., Harmison, G.G., Meyertholen, K., Pennuto, M., Burnett, B.G., Fischbeck, K.H., 2009. Mitochondrial abnormalities in spinal and bulbar muscular atrophy. Hum. Mol. Genet. 18, 27-42.
17. Weydt, P., Pineda, V.V., Torrence, A.E., Libby, R.T., Satterfield, T.F., Lazarowski, E.R., Gilbert, M.L., Morton, G.J., Bammler, T.K., Strand, A.D., Cui, L., Beyer, R.P., Easley, C.N., Smith, A.C., Krainc, D., Luquet, S., Sweet, I.R., Schwartz, M.W., La Spada, A.R., 2006. Thermoregulatory and metabolic defects in Huntington's disease transgenic mice implicate PGC-1alpha in Huntington's disease neurodegeneration. Cell Metab. 4, 349-362.
18. Xiang, Z., Valenza, M., Cui, L., Leoni, V., Jeong, H.-K., Brilli, E., Zhang, J., Peng, Q., Duan, W., Reeves, S.A., Cattaneo, E., Krainc, D., 2011. Peroxisome-proliferator- activated receptor gamma coactivator 1α contributes to dysmyelination in experimental experimental models of Huntington's disease. J. Neurosci. Neurosci. 31, 9544-9553.
19. Zheng, B., Liao, Z., Locascio, J. J., Lesniak, K. A., Roderick, S. S., Watt, M. L., Eklund, A. C., Zhang-James, Y., Kim, P. D., Hauser, M. A. et al. (2010). PGC-1α, a potential therapeutic target for early intervention in Parkinson's disease. Sci. Transl. Med. 2, 52ra73.
20. Chaturvedi, R.K., Adhihetty, P., Shukla, S., Hennessy, T., Calingasan, N., Yang, L., Starkov, A., Kiaei, M., Cannella, M., Sassone, J., Ciammola, A., Squitieri, F., Beal, M.F., 2009. Impaired PGC-1alpha function in muscle in Huntington's disease. Hum. Mol. Genet. 18, 3048-3065.
21. Zhao, W., Varghese, M., Yemul, S., Pan, Y., Cheng, A., Marano, P., Hassan, S., Vempati, P., Chen, F., Qian, X., Pasinetti, G., 2011. Peroxisome proliferator activator receptor gamma coactivator-1alpha (PGC-1alpha) improves motor performance and survival in a mouse model of amyotrophic lateral sclerosis. Mol. Neurodegener. 6, 51.
22. Chaturvedi RK & Flint Beal M (2013) Mitochondrial diseases of the brain. Free Radic Biol Med 63, 1-29.
23. St-Pierre, J., Lin, J., Krauss, S., Tarr, P.T., Yang, R., Newgard, C.B., Spiegelman, B.M., 2003. Bioenergetic analysis of peroxisome proliferator-activated receptor gamma coactivators 1alpha and 1beta (PGC-1alpha and PGC-1beta) in muscle cells. J. Biol.Chem. 278, 26597-26603.
24. Cowell, R.M., Talati, P., Blake, K.R., Meador-Woodruff, J.H., Russell, J.W., 2009. Identification of novel targets for PGC-1alpha and histone deacetylase inhibitors in neuroblastoma cells. Biochem. Biophys. Res. Commun. 379, 578-582.
25. St-Pierre, J., Drori, S., Uldry, M., Silvaggi, J.M., Rhee, J., Jager, S., Handschin, C., Zheng, K., Lin, J., Yang, W., Simon, D.K., Bachoo, R., Spiegelman, B.M., 2006. Suppression of reactive oxygen species and neurodegeneration by the PGC- 1 transcriptional coactivators. Cell 127, 397-408.
26. Valle, I., Alvarez-Barrientos, A., Arza, E., Lamas, S., Monsalve, M., 2005. PGC-1alpha regulates the mitochondrial antioxidant defense system in vascular endothelial cells. Cardiovasc. Res. 66, 562-573.
27. Xiong, S., Patrushev N., Forouuzandeh, F., Hilenski, L., Alexander, R.W., 2015. PGC-1a modulates telomere function and DNA damage inprotecting against age-related chronic diseases. Cell Report 12, 1391-1399.
28. Borniquel, S., Valle, I., Cadenas, S., Lamas, S., Monsalve, M., 2006. Nitric oxide regulates mitochondrial oxidative stress protection via the transcriptional coactivator PGC-1alpha. The FASEB journal: Official Publication of the Federation of American Societies for Experimental Biology, 20, pp. 1889-1891.
29. Lai, L., Leone, T.C., Zechner, C., Schaeffer, P.J., Kelly, S.M., Flanagan, D.P., Medeiros, D.M., Kovacs, A., Kelly, D.P., 2008. Transcriptional coactivators PGC-1alpha and PGC-lbeta control overlapping programs required for perinatal maturation of the heart. Genes Dev. 22, 1948-1961.
30. Garnier, A., Fortin, D., Delomenie, C., Momken, I., Veksler, V., Ventura-Clapier, R., 2003. Depressed mitochondrial transcription factors and oxidative capacity in rat failing cardiac and skeletal muscles. J. Physiol. 551, 491-501.
31. Ljubicic V, Joseph A, Saleem A, et al: Transcriptional and post-transcriptional regulation of mitochondrial biogenesis in skeletal muscle: effects of exercise and aging. Biochim Biophys Acta 2010;1800:223-234.
32. Gouspillou G, Picard M, Godin R, Burelle Y, Hepple R: Role of peroxisome proliferative activated receptor gamma coactivator 1- alpha (PGC-1α) in denervation-induced atrophy in aged muscle: facts and hypotheses. Longev Healthspan 2013;2:13.
33. Johnson ML, Robinson MM, Nair KS: Skeletal muscle aging and the mitochondrion. Trends Endocrin Met 2013;24:247-256.
34. Marzetti E, Calvani R, Cesari M, et al: Mitochondrial dysfunction and sarcopenia of aging: from signaling pathways to clinical trials. Int J Biochem Cell Biol 2013;45:2288-2301.
35. Calvani R, Joseph A, Adhihetty PJ, et al: Mitochondrial pathways in sarcopenia of aging and disuse muscle atrophy. Biol Chem 2013; 394:393-414.
36. Wallace DC: A mitochondrial paradigm of metabolic and degenerative diseases, aging, and cancer: a dawn for evolutionary medicine. Annu Rev Genet 2005;39:359.
37. Finck BN, Kelly DP: PGC-1 coactivators: inducible regulators of energy metabolism in health and disease. J Clin Invest 2006;116: 615-622
38. Tina Wenz, Susana G. Rossi, Richard L. Rotundo, Bruce M. Spiegelman, and Carlos T. Moraes. 2009, Increased muscle PGC-1a expression protects from sarcopenia and metabolic disease during aging, PNAS106, 20405-20410.
39. Rolfe, D. F. and Brown, G. C. (1997). Cellular energy utilization and molecular origin of standard metabolic rate in mammals. Physiol. Rev. 77, 731-758.
40. Jastroch, M., Divakaruni, A. S., Mookerjee, S., Treberg, J. R. and Brand, M. D. (2010). Mitochondrial proton and electron leaks. Essays Biochem. 47, 53-67.
41. Fukui Y, Masui S, Osada S, Umesono K, Motojima K. 2000. A new thiazolidinedione, NC-2100, which is a weak PPAR-g activator, exhibits potent antidiabetic effects and induces uncoupling protein 1 in white adipose tissue of KKAy obese mice. Diabetes 49: 759-767
42. Wilson-Fritch L, Nicoloro S, Chouinard M, Lazar MA, Chui PC, Leszyk J, Straubhaar J, Czech MP, Corvera S. 2004. Mitochondrial remodeling in adipose tissue associated with obesity and treatment with rosiglitazone. J Clin Invest 114: 1281-1289.
43. Quinlan, C. L., Treberg, J. R. and Brand, M. D. (2011). Mechanisms of mitochondrial free radical production and their relationship to the aging process. In Handbook of the Biology of Aging (Seventh Edition) (ed. J. M. Edward and N. A. Steven), pp. 47-61. San Diego, CA: Academic Press.
44. Sahin, E., Colla, S., Liesa, M., Moslehi, J., Mu¨ller, F. L., Guo, M., Cooper, M., Kotton, D., Fabian, A. J., Walkey, C. et al. (2011). Telomere dysfunction induces metabolic and mitochondrial compromise. Nature 470, 359-365.
45. Kujoth, G. C., Hiona, A., Pugh, T. D., Someya, S., Panzer, K., Wohlgemuth, S. E., Hofer, T., Seo, A. Y., Sullivan, R., Jobling, W. A. et al. (2005). Mitochondrial DNA mutations, oxidative stress, and apoptosis in mammalian aging. Science 309, 481-484.
46. Trifunovic, A., Wredenberg, A., Falkenberg, M., Spelbrink, J. N., Rovio, A. T., Bruder, C. E., Bohlooly-Y, M., Gidlo¨f, S., Oldfors, A., Wibom, R. et al. (2004). Premature ageing in mice expressing defective mitochondrial DNA polymerase. Nature 429, 417-423.
47. Lin, J., Wu, H., Tarr, P. T., Zhang, C. Y., Wu, Z., Boss, O., Michael, L. F., Puigserver, P., Isotani, E., Olson, E. N. et al. (2002b). Transcriptional co-activator PGC-1 alpha drives the formation of slow-twitch muscle fibres. Nature 418, 797-801.
48. Dillon, L. M., Williams, S. L., Hida, A., Peacock, J. D., Prolla, T. A., Lincoln, J. and Moraes, C. T. (2012). Increased mitochondrial biogenesis in muscle improves aging phenotypes in the mtDNA mutator mouse. Hum. Mol. Genet. 21, 2288-2297.
49. Wenz, T., Rossi, S. G., Rotundo, R. L., Spiegelman, B. M. and Moraes, C. T. (2009). Increased muscle muscle PGC-1alpha expression protects from sarcopenia and and metabolic disease during aging. Proc. Natl. Acad. Sci. USA 106, 20405-20410.

## Claims

1. A composition for preventing or treating a disease or symptom associated with a decrease in peroxisome proliferator-activated receptor coactivator 1-alpha (PGC-1α) expression, the composition comprising, as an active ingredient, a compound represented by general formula I or a salt, hydrate, or solvate thereof:
General formula I: S-(MS)p-(MS)q
wherein S is sialic acid; and (MS)p and (MS)q each are independently a monosaccharide residue,
wherein the composition is for use in reducing the volume of adipose tissue or in reducing the content of triglycerides in adipose tissue.

2. The composition for use according to claim 1, wherein the adipose tissue is subcutaneous adipose tissue.

3. The composition for use according to claim 2, wherein the subcutaneous adipose tissue is subcutaneous adipose tissue of the femoral region, chest, a lower part of the neck, neckline, buttocks, face, lips, cheeks, eyelids and/or hands.

4. The composition for use according to claim 1, wherein the adipose tissue is any adipose tissue that may be formed in the body, including adipose tissue formed by fat embolism.

## Patentansprüche

1. Zusammensetzung für die Vorbeugung oder Behandlung einer Krankheit oder eines Symptoms, die/das mit einer Abnahme einer Expression von Peroxisom-Proliferatoraktiviertem Rezeptor-Co-Aktivator 1-alpha (PGC-1α) assoziiert ist, wobei die Zusammensetzung als einen Wirkstoff eine Verbindung, dargestellt durch die allgemeine Formel I, oder ein Salz, Hydrat oder Solvat davon umfasst:
Allgemeine Formel I: S-(MS)p-(MS)q
wobei S Sialinsäure ist; und (MS)p und (MS)q jeweils unabhängig ein Monosaccharidrest sind,
wobei die Zusammensetzung für die Verwendung bei der Reduzierung des Volumens von Fettgewebe oder bei der Reduzierung des Gehalts von Triglyceriden in Fettgewebe dient.

2. Zusammensetzung für die Verwendung nach Anspruch 1, wobei das Fettgewebe subkutanes Fettgewebe ist.

3. Zusammensetzung für die Verwendung nach Anspruch 2, wobei das subkutane Fettgewebe subkutanes Fettgewebe der Oberschenkelregion, der Brust, des unteren Teils des Halses, des Dekolletés, des Gesäßes, des Gesichts, der Lippen, der Wangen, der Augenlider und/oder der Hände ist.

4. Zusammensetzung für die Verwendung nach Anspruch 1, wobei das Fettgewebe jedes Fettgewebe ist, das in dem Körper ausgebildet werden kann, einschließlich Fettgewebe, das durch Fettembolie ausgebildet wird.

## Revendications

1. Composition pour prévenir ou traiter une maladie ou un symptôme associé à une diminution de l'expression du coactivateur 1-alpha du récepteur activé par les proliférateurs de peroxysomes (PGC-1α), la composition comprenant, en tant qu'ingrédient actif, un composé représenté par la formule générale 1 ou un sel, un hydrate ou un solvate de celui-ci :
Formule générale I : S-(MS)p-(MS)q
dans laquelle S est l'acide sialique ; et (MS)p et (MS)q sont chacun indépendamment un résidu de monosaccharide,
dans laquelle la composition est destinée à être utilisée pour réduire le volume de tissu adipeux ou pour réduire la teneur en triglycérides dans le tissu adipeux.

2. Composition pour une utilisation selon la revendication 1, dans laquelle le tissu adipeux est du tissu adipeux sous-cutané.

3. Composition pour une utilisation selon la revendication 2, dans laquelle le tissu adipeux sous-cutané est le tissu adipeux sous-cutané de la région fémorale, de la poitrine, de la partie inférieure du cou, de la nuque, des fesses, du visage, des lèvres, des joues, des paupières et/ou des mains.

4. Composition pour une utilisation selon la revendication 1, dans laquelle le tissu adipeux est tout tissu adipeux susceptible de se former dans l'organisme, y compris le tissu adipeux formé par embolie graisseuse.
